(19) **Europäisches Patentamt / European Patent Office / Office européen des brevets**

(11) **EP 4 222 286 B1**

(12) **EUROPEAN PATENT SPECIFICATION**

(45) Date of publication and mention
of the grant of the patent:
**19.06.2024 Bulletin 2024/25**

(21) Application number: **21766506.6**

(22) Date of filing: **13.09.2021**

(51) International Patent Classification (IPC):
**C12Q 1/6886** $^{(2018.01)}$

(52) Cooperative Patent Classification (CPC):
**C12Q 1/6886;** C12Q 2600/118; C12Q 2600/158

(86) International application number:
**PCT/EP2021/075086**

(87) International publication number:
**WO 2022/069201 (07.04.2022 Gazette 2022/14)**

(54) **OUTCOME PREDICTION OF BLADDER OR KIDNEY CANCER**

ERGEBNISVORHERSAGE VON BLASEN- ODER NIERENKREBS

PRÉDICTION DE L'ISSUE DU CANCER DE LA VESSIE OU DU REIN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.10.2020 EP 20199659**

(43) Date of publication of application:
**09.08.2023 Bulletin 2023/32**

(73) Proprietor: **Koninklijke Philips N.V.**
**5656 AG Eindhoven (NL)**

(72) Inventors:
• **HOFFMANN, Ralf, Dieter**
**5656 AE Eindhoven (NL)**
• **STOFFELS, Monique**
**5656 AE Eindhoven (NL)**

(74) Representative: **Philips Intellectual Property & Standards**
**High Tech Campus 52**
**5656 AG Eindhoven (NL)**

(56) References cited:
WO-A1-2014/194078    WO-A1-2016/049276
WO-A1-2018/104147    WO-A2-2007/146668

• **QIANG ZHE ET AL: "Inhibition of TPL2 by interferon-[alpha] suppresses bladder cancer through activation of PDE4D", JOURNAL OF EXPERIMENTAL & CLINICAL CANCER RESEARCH , vol. 37, no. 1 27 November 2018 (2018-11-27), XP055783668, DOI: 10.1186/s13046-018-0971-4 Retrieved from the Internet: URL:http://link.springer.com/content/pdf/1 0.1186/s13046-018-0971-4.pdf**

Remarks:
The complete document including Reference Table(s) and the Sequence Listing(s) can be downloaded from the EPO website

**Description**

FIELD OF THE INVENTION

The invention relates to a method of predicting an outcome of a bladder or kidney cancer subject, and to an apparatus for predicting an outcome of a bladder or kidney cancer subject. Moreover, the invention relates to a diagnostic kit, to a use of the kit, to a use of the kit in a method of predicting an outcome of a bladder or kidney cancer subject, to a use of first, second, and/or third gene expression profile(s) in a method of predicting an outcome of a bladder or kidney cancer subject, and to a corresponding computer program product.

BACKGROUND OF THE INVENTION

Cancer is a class of diseases in which a group of cells displays uncontrolled growth, invasion and sometimes metastasis. These three malignant properties of cancers differentiate them from benign tumours, which are self-limited and do not invade or metastasize.

Bladder cancer is fairly common, ranking $6^{th}$ and representing 4.5% of new cancer cases per year in the US. For 2020, it is estimated that more than 81,000 new cases will present and nearly 18,000 people will die from this type of cancer. The median age of diagnosis is 74 with a 5-year relative survival of 76.9%. The earlier bladder cancer is diagnosed, the higher the chance of surviving more than 5 years after diagnosis; the 5-year survival for in situ bladder cancer is 95.8%, while only 5.5% of patients with metastatic bladder cancer survive for at least 5 years. About 1/3 of the patients represent with localized disease. More men than women are affected, and incidence increases with age. The main risk factors for bladder cancer are smoking, having a family history of bladder cancer, exposure to certain chemicals, and past treatment with radiation therapy in the pelvic area (see National Cancer Institute, Surveillance, Epidemology, and End Results Program, "Cancer Stat Facts: Bladder Cancer", Bethesda, MD).

The four standard treatments for bladder cancer are: surgery to remove the tumor, radiation therapy to kill cancer cells, chemotherapy to stop or slow down cell division, and immunotherapy to help the patient's own immune system to fight the cancer. In addition, new treatments are tested in clinical trials. Bladder cancer often recurs, even when it is non-invasive at time of diagnosis. Therefore, it is standard practice to perform surveillance of the urinary tract after initial diagnosis (see National Cancer Institute, PDQ® Adult Treatment Editorial Board, "PDQ Bladder Cancer Treatment" Bethesda, MD).

Bladder cancer is classified in muscle invasive (MIBC) and muscle non-invasive (NMIBC) BC. NMIBCs frequently recur and progress to MIBCs; approximately 25% of bladder cancer patients have muscle invasive bladder cancer, or MIBC, which is at high risk of spreading and therefore life threatening. In 20-25% of MIBC who have had radical cystectomy, still microscopic spread to the lymph nodes is found, which usually reduces the chance of cure considerably. Therefore, treatment options for MIBC are directed at both the localized tumor and at possible unsuspected spread to lymph nodes. The options considered are chemotherapy, radical cystectomy and radical radiotherapy. Due to uncertainty on the relative effectiveness and indications for each of these treatments, considerable variation in UK practice exists (see National Collaborating Centre for Cancer, "Bladder Cancer: Diagnosis and Management", National Institute for Health and Care Excellence, London, UK, 2015).

For bladder cancer patients who cannot undergo cystectomy, or who refuse operation, radiotherapy is an alternative treatment with comparatively good results. E.g. in patients with MIBC, no difference in survival was seen between those who underwent cystectomy and those who received RT. About 25% of patients receiving RT survived for 5 years while retaining the bladder. Improving quality of life with organ preservation is a significant consideration for patients with bladder cancer. Optimization of RT delivery in combination with newer systemic therapies, which requires close cooperation of the involved clinical disciplines, may allow for future improvements and adoption of an organ preserving strategy for a larger number of patients (see Zhang S. et al., "Radiotherapy in muscleinvasive bladder: the latest research progress and clinical application", Am J Cancer Res, Vol. 5, NO. 2, pages 854-868, 2015).

An improved prediction of effectiveness of RT for each patient be it in the radical or the salvage setting, would improve therapy selection and potentially survival. This can be achieved by 1) optimizing RT for those patients where RT is predicted to be effective (e.g. by dose escalation or a different starting time) and 2) guiding patients where RT is predicted not to be effective to an alternative, potentially more effective form of treatment. Further, this would reduce suffering for those patients who would be spared ineffective therapy and would reduce cost spent on ineffective therapies.

Instead of treating the tumor, the patient's own immune system can be stimulated to fight the tumor more actively. One immunotherapy that has already been used for over 40 years is intravesical Bacillus Calmette-Guérin (BCG) to prevent recurrence and progression of NMIBC. Although considered successful, its mechanism is still largely unknown, and still fails in up to 40% of patients. There is no gold standard intravesical treatment after BCG failure.

The newly emerging immunotherapies such as checkpoint inhibitors are hoped to fulfill this unmet need (see Alhunaidi O. and Zlotta A.R., "The use of intravesical BCG in urothelial carcinoma of the bladder", Ecancermedicalscience, Vol. 13, page 905, 2019). Recent clinical studies with immune checkpoint inhibitors have shown promising results, with many more trials ongoing or in development (see Fakhrejahani F. et al., "Immunotherapies for bladder cancer: a new hope", Curr Opin Urol, Vol. 25, No. 6, pages 586-596, 2015). Combining immune checkpoint inhibitors with other treatments such as chemotherapy, targeted therapy, or RT, may lead to development of new treatment strategies with synergistic antitumor activity in advanced bladder cancer. Nevertheless, there is a high need for predictive biomarkers that can identify patients likely to benefit from immunotherapies to help guide precision cancer treatment.

To detect bladder cancer, unpleasant and expensive cystoscopy and biopsies are needed, which are often accompanied by several adverse effects. Therefore, there is a need for new diagnostic methods for initial detection as well as for surveillance. Currently, several FDA-approved urine tests are available, but sensitivity, specificity and diagnostic accuracy are still suboptimal. DNA and RNA based markers, especially when derived from liquid biopsies, are promising potential markers in diagnostic, prognostic, predictive and monitoring urological malignancies. However, there is an urgent need to clinically validate the recently discovered biomarkers in well-designed multicenter studies (see Santoni G. et al., "Urinary Markers in Bladder Cancer: An Update", Front Oncol, Vol. 8, page 362, 2018).

Kidney cancer is among the 10 most common cancers in both men and women, representing 4.1% of new cancer cases per year in the US. For 2020, it is estimated that almost 74,000 new cases of kidney cancer will be diagnosed, and almost 15,000 people will die from this disease. The median age of diagnosis is 64 with a 5-year relative survival of 75.2%. The earlier kidney cancer is diagnosed, the higher the chance of surviving more than 5 years after diagnosis; the 5-year survival for localized kidney cancer is 92.6%, while only 13% of patients with distant kidney cancer survive for at least 5 years. About 65% of the patients present with localized disease. Men are affected twice as much as women, and incidence increases with age. The main risk factors for kidney cancer are smoking, obesity, high blood pressure, having a family history of kidney cancer, workplace exposures, gender, race, certain medicines, advanced kidney disease, and genetic and hereditary risk factors. Since the 1990's, incidence is rising, which can partly be explained by the use of newer imaging tests picking up cancers which previously would not have been detected (see National Cancer Institute, Surveillance, Epidemology, and End Results Program, "Cancer Stat Facts: Kidney Cancer", Bethesda, MD).

About 90% of kidney cancers are renal cell carcinoma (RCC). Usually they grow as one tumor in a single kidney, but multiple tumors in one or both kidneys at the same time are sometimes seen as well. 70% of RCC are clear cell RCC (ccRCC), called after the phenotype of the cells when seen under a microscope. The remaining non-ccRCC include papillary renal cell carcinoma (pRCC), chromophobe renal cell carcinoma (chRCC) and other rare types of renal cell carcinoma.

Identifying and differentiation the subtypes of kidney cancer is critical for management and treatment of patients. As described above, kidney cancers can be subtyped according to histology. This is however not providing insights in the mechanisms that underlie that particular subtype, and with the emerging plethora of new therapies becoming available, a better understanding of each subtype is needed.

Comprehensive genomic and phenotypic analysis of the RCC subtypes (ccRCC, pRCC, chRCC) revealed distinctive features of each histological subtype (see Ricketts C.J. et al., "The Cancer Genome Atlas Comprehensive Molecular Characterization of Renal Cell Carcinoma", Cell Rep, Vol. 23, No. 1, pages 313-326, 2018). Here, it was suggested that the combination of histology plus genomics provides unique insight into patient-centered management. Some features were shared by some RCC subtypes, but not all. Somatic alteration of BAP1, PBRM1, and PTEN and altered metabolic pathways correlated with subtype-specific decreased survival, while CDKN2A alteration, increased DNA hypermethylation, and increases in the immune-related Th2 gene expression signature correlated with decreased survival within all major histologic subtypes (see Ricketts et al., 2018, ibid). Main alterations in ccRCC can be grouped according to the mechanisms involved (see Linehan W.M. and Ricketts C.J., "The Cancer Genome Atlas of renal cell carcinoma: findings and clinical implications", Nat Rev Urol, Vol. 16, 9, pages 539-552, 2019). Most ccRCC had a loss of the VHL complex, leading to stabilization of HIF1-alpha and HIF-2alpha, creating a state of pseudohypoxia, which induces expression of angiogenesis and proliferation factors. mTOR pathway activating alterations are frequent in ccRCC, promoting protein synthesis and cell survival. Also common are mutations in chromatin-remodeling genes, which are proposed to alter gene transcription. Loss of CDKN2A results in increased activation of the cell cycle and was correlated with poorer survival (see Linehan W.M. and Ricketts C.J., 2019, ibid).

In contrast to most other cancer types, biopsies are sometimes not needed to diagnose kidney tumors and imaging can provide enough information to decide if an operation is needed. In these cases, the diagnosis will be confirmed on the part of the kidney that was removed. In case of insufficient imaging, small tumors, or when other treatments are considered, a biopsy may be taken.

As most kidney cancers are slow growing, for patients with small tumors active surveillance may be an option. When tumor removal is needed, surgery is the main treatment for most kidney cancers. Alternatives to surgery are cryoablation and radiofrequency ablation. When a patient is too fragile for surgery, other treatments may be tried first, such as external beam radiotherapy (EBRT). Usually however, RT is used in a palliative setting for kidney cancer.

Kidney cancer cells usually do not respond well to chemotherapy, which is therefore not a standard treatment for kidney cancers. Targeted therapies are usually applied to shrink or slow tumor growth, or as adjuvant therapy after surgery. For more advanced kidney cancers, immune checkpoint inhibitors are being used to help the immune system fight the tumor better, sometimes in combination with targeted therapies. In specific cases, the cytokines IL-2 and Interferon-alpha can be used to shrink the tumors.

Particularly for intermediate and poor risk patients the treatment paradigm for ccRCC has evolved, with recent addition of cabozantinib (tyrosine kinase inhibitor) and nivolumab/ipilimumab (anti-PD-1/anti-CTLA-4) (see Atkins M.B. and Tannir N.M., "Current and emerging therapies for first-line treatment of metastatic clear cell renal cell carcinoma", Cancer Treat Rev, Vol. 70, pages 127-137, 2018).

Treatment selection for ccRCC is still based on the available clinical evidence and expert opinions. Currently, no validated predictive biomarkers are available. There are suggestions that overexpression of PD-L1 is associated with poor outcome, but its role as biomarker is not clear. Other potential biomarkers for immunotherapy include PD-L2 expression, IDO-1 expression, and infiltration of CD8+ T cells. More hope was vested on distinct genetic features, such as loss-of-function mutations in the PBRM1 gene, which is involved in JAK/STAT transcription regulation, hypoxia, and immune signalling pathways. As for consensus on optimal treatment sequence in ccRCC (e.g. switching from targeted therapy to immunotherapy and sequential vs combination treatment), more data is needed. Prospectively validated biomarkers are needed to match patients to treatments, as well as for determining best strategies for treatment sequencing (see Atkins M.B. and TannirN.M., 2018, ibid).

As pRCC accounts for approximately 15-20% of RCC cases and is further divided into Type 1 and Type 2, and treatment for RCC patients is based on studies with minimal participation from patients with pRCC, conventional therapies tend to be less effective for non-ccRCC patients. As described above, MET is a known alteration in pRCC, making it a potential target for directed therapy (see Rhoades Smith K.E. and Bilen M.A., "A Review of Papillary Renal Cell Carcinoma and MET Inhibitors", Kidney Cancer, Vol. 3, No. 3, pages 151-161, 2019). Developing effective MET targeted therapies is needed since outcomes are typically worse for pRCC when treated with conventional therapies. MET targeting drugs in pRCC are still under active investigation. Additionally, there are indications that immune checkpoint inhibitors alone or combination with MET inhibitors could be beneficial in treatment of pRCC (see Rhoades Smith K.E. and Bilen M.A., 2019, ibid).

WO 2007/146668 A2 discloses a method for determining the prognosis of a subject having renal cell carcinoma (RCC) or bladder carcinoma, comprising determining the presence or level of IMP3 in a primary tumor of a subject, wherein the presence of IMP3 in the primary tumor of the subject indicates that the prognosis of the subject is poor, whereas an essentially undetectable level of IMP3 in the primary tumor of the subject indicates that the prognosis of the subject is good.

WO 2016/049276 A1 and WO 2018/104147 A1 disclose immune response related gene signatures that are associated to prognosis of bladder cancer, WO 2014/194078 A1 discloses immune response related gene signatures that are associated to prognosis of kidney cancer.

In conclusion, an improved understanding of the (molecular) mechanisms of disease, and the emerging availability of treatments underlines the strong need for better prediction of response to treatment remains, for primary bladder and kidney cancer as well as for the post-surgery setting.

SUMMARY OF THE INVENTION

It is an objective of the invention to provide a method of predicting an outcome of a bladder or kidney cancer subject, and to an apparatus for predicting an outcome of a bladder or kidney cancer subject, which allow to make better treatment decisions. It is a further aspect of the invention to provide a diagnostic kit, a use of the kit, a use of the kit in a method of predicting an outcome of a bladder or kidney cancer subject, a use of first, second, and/or third gene expression profile(s) in a method of predicting an outcome of a bladder or kidney cancer subject, and a corresponding computer program product.

In a first aspect of the present invention is provided a method of predicting an outcome of a bladder or kidney cancer subject, comprising:

- determining of a first gene expression profile for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, said first gene expression profile(s) being determined in a biological sample obtained from the subject, and/or
- determining of a second gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, said second gene expression profile(s) being determined in a biological sample obtained from the subject, and/or
- determining of a third gene expression profile for each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, said third gene expression profile(s) being determined in a biological sample obtained from the subject,
- determining the prediction of the outcome based on the first gene expression profile(s), or on the second gene expression profile(s), or on the third gene expression profile(s), or on the first, second, and third gene expression profile(s), and
- optionally, providing the prediction to a medical caregiver or the subject.

In an alternative embodiment the invention relates to a computer implemented method of predicting an outcome of a bladder or kidney cancer subject, comprising:

- receiving the result of a determination of a first gene expression profile for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, said first gene expression profile(s) being determined in a biological sample obtained from the subject, and/or
- receiving the result of a determination of a second gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, said second gene expression profile(s) being determined in a biological sample obtained from the subject, and/or
- receiving the result of a determination of a third gene expression profile for each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, said third gene expression profile(s) being determined in a biological sample obtained from the subject,
- determining the prediction of the outcome based on the first gene expression profile(s), or on the second gene expression profile(s), or on the third gene expression profile(s), or on the first, second, and third gene expression profile(s), and
- optionally, providing the prediction to a medical caregiver or the subject.

In one embodiment of the invention a method of predicting an outcome of a bladder cancer subject or a kidney cancer subject is presented, the method comprising:

- determining the gene expression profiles of three or more genes, wherein the three or more genes are selected from:

  - immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, or
  - T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, or
  - PDE4D7 correlated genes selected from the group consisting of: ABCC5, CUX2, KIAA1549, PDE4D, RAP1GAP2, SLC39A11, TDRD1, and VWA2, or
  - wherein said three or more genes comprise at least one or more gene(s) selected from each of the immune defense response genes, T-Cell receptor signaling genes and PDE4D7 correlated genes;

- wherein the gene expression profiles of the three or more genes are determined in a biological sample obtained from the subject;
- determining the prediction of the outcome based on the gene expression profiles of the three or more genes, and
- optionally, providing the prediction to a medical caregiver or the subject.

In an alternative embodiment the invention relates to a computer implemented method of predicting an outcome of a bladder cancer subject or a kidney cancer subject, the method comprising:

- receiving the result of a determination the gene expression profiles of three or more genes, wherein the three or more genes are selected from:

  - immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, or
  - T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, or

- PDE4D7 correlated genes selected from the group consisting of: ABCC5, CUX2, KIAA1549, PDE4D, RAP1GAP2, SLC39A11, TDRD1, and VWA2, or
- wherein said three or more genes comprise at least one or more gene(s) selected from each of the immune defense response genes, T-Cell receptor signaling genes and PDE4D7 correlated genes;

- wherein the gene expression profiles of the three or more genes are determined in a biological sample obtained from the subject;
- determining the prediction of the outcome based on the gene expression profiles of the three or more genes, and
- optionally, providing the prediction to a medical caregiver or the subject.

In recent years, the importance of the immune system in cancer inhibition as well as in cancer initiation, promotion and metastasis has become very evident (see Mantovani A. et al., "Cancerrelated inflammation", Nature, Vol. 454, No. 7203, pages 436-444, 2008, and Giraldo N.A. et al., "The clinical role of the TME in solid cancer", Br J Cancer, Vol. 120, No. 1, pages 45-53, 2019). The immune cells and the molecules they secrete form a crucial part of the tumour microenvironment and most immune cells can infiltrate the tumour tissue. The immune system and the tumour affect and shape one another. Thus, anti-tumour immunity can prevent tumour formation while an inflammatory tumour environment may promote cancer initiation and proliferation. At the same time, tumour cells that may have originated in an immune system-independent manner will shape the immune microenvironment by recruiting immune cells and can have a pro-inflammatory effect while also suppressing anti-cancer immunity.

Some of the immune cells in the tumour microenvironment will have either a general tumour-promoting or a general tumour-inhibiting effect, while other immune cells exhibit plasticity and show both tumour-promoting and tumour-inhibiting potential. Thus, the overall immune microenvironment of the tumour is a mixture of the various immune cells present, the cytokines they produce and their interactions with tumour cells and with other cells in the tumour microenvironment (see Giraldo N.A. et al., 2019, ibid).

The principles described above with regard to the role of the immune system in cancer in general also apply to prostate cancer. Chronic inflammation has been linked to the formation of benign as well as malignant prostate tissue (see Hall W.A. et al., 2016, ibid) and most prostate cancer tissue samples show immune cell infiltrates. The presence of specific immune cells with a pro-tumour effect has been correlated with worse prognosis, while tumours in which natural killer cells were more activated showed better response to therapy and longer recurrence-free periods (see Shiao S.L. et al., "Regulation of prostate cancer progression by tumor microenvironment", Cancer Lett, Vol. 380, No. 1, pages 340-348, 2016).

While a therapy will be influenced by the immune components of the tumour microenvironment, RT itself extensively affects the make-up of these components (see Barker H.E. et al., "The tumor microenvironment after radiotherapy: Mechanisms of resistance or recurrence", Nat Rev Cancer, Vol. 15, No. 7, pages 409-425, 2015). Because suppressive cell types are comparably radiationinsensitive, their relative numbers will increase. Counteractively, the inflicted radiation damage activates cell survival pathways and stimulates the immune system, triggering inflammatory responses and immune cell recruitment. Whether the net effect will be tumour-promoting or tumour-suppressing is as yet uncertain, but its potential for enhancement of cancer immunotherapies is being investigated. The present invention is based on the idea that, since the status of the immune system and of the immune microenvironment have an impact on therapy effectiveness, the ability to identify markers predictive for this effect might help to be better able to predict overall outcome.

**Immune response defense genes**

The integrity and stability of genomics DNA is permanently under stress induced by various cell internal and external factors like exposure to radiation, viral or bacterial infections, but also oxidation and replication stress (see Gasser S. et al., "Sensing of dangerous DNA", Mechanisms of Aging and Development, Vol. 165, pages 33-46, 2017). In order to maintain DNA structure and stability, a cell must be able to recognize all types of DNA damages like single or double strand breaks etc. induced by various factors. This process involves the participation of a multitude of specific proteins depending on the kind of damage as part of DNA recognition pathways.

Recent evidence suggests that mis-localized DNA (e.g., DNA unnaturally appearing in the cytosolic fraction of the cell in contrast to the nucleus) and damaged DNA (e.g., through mutations occurring in cancer development) is used by the immune system to identify infected or otherwise diseased cells while genomic and mitochondrial DNA present in healthy cells is ignored by DNA recognition pathways. In diseased cells, cytosolic DNA sensor proteins have been demonstrated to be involved in the detection of DNA occurring unnaturally in the cytosol of the cell. Detection of such DNA by different nucleic acid sensors translates into similar responses leading to nuclear factor kappa-B (NF-kB) and interferon type I (IFN type I) signalling followed by the activation of innate immune system components. While the recognition of viral DNA is known to induce an IFN type I response, evidence that sensing of DNA damage can initiate immune responses has only recently been accumulating.

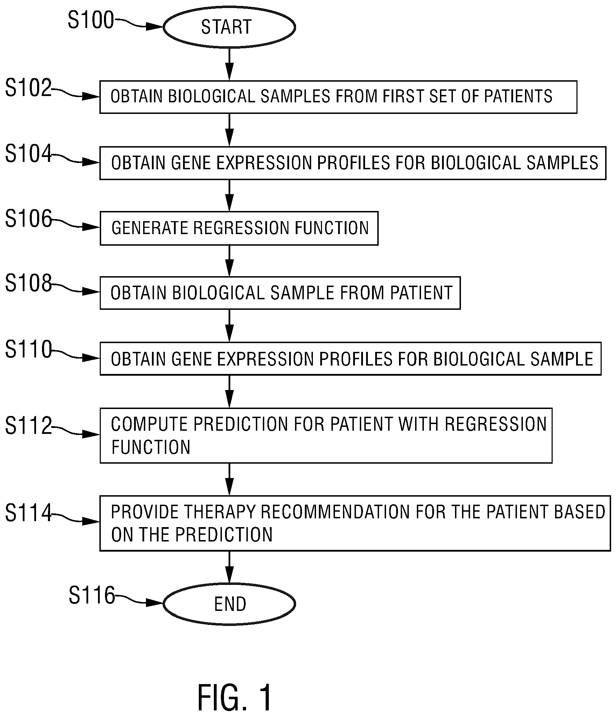

TLR9 (Toll-like receptor 9) located in the endosomes was one of the first DNA sensors molecules identified to be involved in the immune recognition of DNA by signalling downstream via the adaptor protein myeloid differentiation primary-response protein88 (MYD88). This interaction in turn activates mitogen-activated protein kinases (MAPKs) and NF-kB. TLR9 also induces the generation of type I interferons through the activation of IRF7 via IkB Kinase alpha (IKKalpha) in plasmacytoid dendritic cells (pDCs). Various other DNA immune receptors including IFI16 (IFN-gamma-inducible protein 16), cGAS (cyclic DMP-AMP synthase, DDX41 (DEAD-box helicase 41), as well as ZBP1 (Z-DNA-binding protein 1) interact with STING (stimulator of IFN genes), which activates the IKK complex and IRF3 through TBK1 (TANK binding kinase 1). ZBP1 also activates NF-kB via recruitment of RIP1 and RIP3 (receptor-interacting protein 1 and 3, respectively). While the helicase DHX36 (DEAH-box helicase 36) interacts in a complex with TRID to induce NF-kB and IRF-3/7 the DHX9 helicase stimulates MYD88-dependent signalling in plasmacytoid dendritic cells. The DNA sensor LRRFIP1 (leucine-rich repeat flightless-interacting protein) complexes with beta-catenin to activate the transcription of IRF3 whereas AIM2 (absent in melanoma 2) recruits the adaptor protein ASC (apoptosis speck-like protein) to induce a caspase-1-activating inflammasome complex leading to the secretion of interleukin-1beta (IL-1beta) and IL-18 (see Fig. 1 of Gasser S. et al., 2017, ibid, which provides a schematic overview of DNA damage and DNA sensor pathways leading to the production of inflammatory cytokines and the expression of ligands for activating innate immune receptors. Members of the non-homologous end joining pathway (orange), homologous recombination (red), inflammasome (dark green), NF- kB and interferon responses (light green) are shown).

The factors and mechanisms responsible for activating the DNA sensor pathways in cancer are currently not well elucidated. It will be important to identify the intratumoral DNA species, sensors and pathways implicated in the expression of IFNs in different cancer types at all stages of the disease. In addition to therapeutic targets in cancer, such factors may also have prognostic and predictive value. Novel DNA sensor pathway agonists and antagonists are currently being developed and tested in preclinical trials. Such compounds will be useful in characterizing the role of DNA sensor pathways in the pathogenesis of cancer, autoimmunity and potentially other diseases.

## T-Cell receptor signaling genes

An immune response against pathogens can be elicited at different levels: there are physical barriers, such as the skin, to keep invaders out. If breached, innate immunity comes into play; a first and fast non-specific response. If this is not sufficient, the adaptive immune response is elicited. This is much more specific and needs time to develop when encountering a pathogen for the first time. Lymphocytes are activated by interacting with activated antigen presenting cells from the innate immune system, and are also responsible for maintenance of memory for faster responses upon next encounters with the same pathogen.

As lymphocytes are highly specific and effective when activated, they are subject to negative selection for their ability to recognize self, a process known as central tolerance. As not all selfantigens are expressed at selection sites, peripheral tolerance mechanisms evolved as well, such as ligation of the TCR in absence of co-stimulation, expression of inhibitory co-receptors, and suppression by Tregs. A disturbed balance between activation and suppression may lead to autoimmune disorders, or immune deficiencies and cancer, respectively.

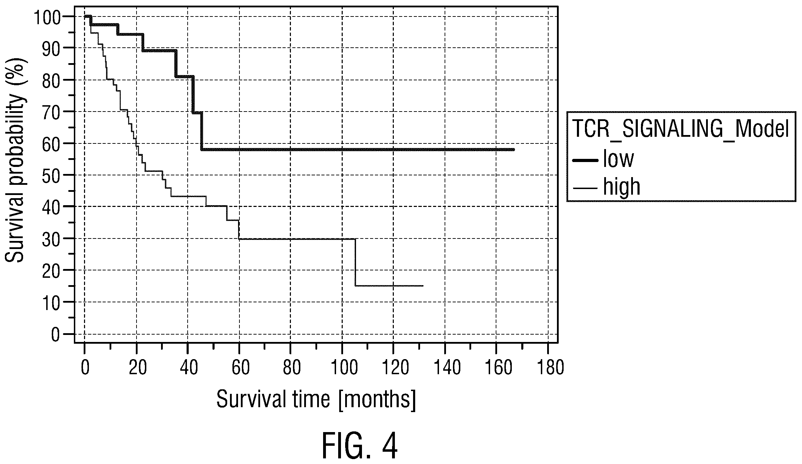

T-cell activation can have different functional consequences, depending on the location the type of T-cell involved. CD8+ T-cells differentiate into cytotoxic effector cells, whereas CD4+ T-cells can differentiate into Th1 (IFNγ secretion and promotion of cell mediated immunity) or Th2 (IL4/5/13 secretion and promotion of B cell and humoral immunity). Differentiation towards other, more recently identified T-cell subsets is also possible, for example the Tregs, which have a suppressive effect on immune activation (see Mosenden R. and Tasken K., "Cyclic AMP-mediated immune regulation - Overview of mechanisms of action in T-cells", Cell Signal, Vol. 23, No. 6, pages 1009-1016, 2011, in particular, Fig. 4, which T-cell activation and its modulation by PKA, and Tasken K. and Ruppelt A., "Negative regulation of T-cell receptor activation by the cAMP-PKA-Csk signaling pathway in T-cell lipid rafts", Front Biosci, Vol. 11, pages 2929-2939, 2006).

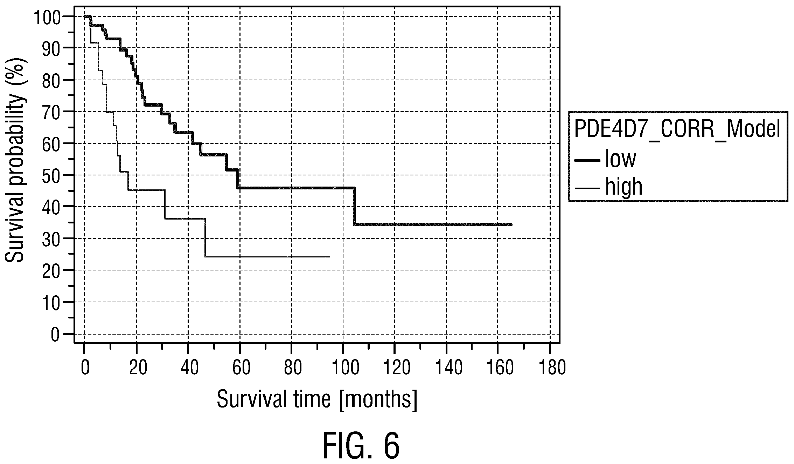

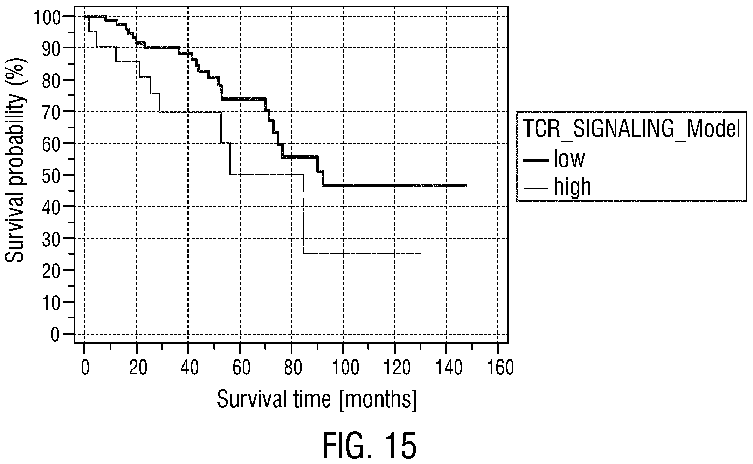

Both PKA and PDE4 regulated signaling intersect with TCR induced T-cell activation to fine-tune its regulation, with opposing effects (see Abrahamsen H. et al., "TCR- and CD28-mediated recruitment of phosphodiesterase 4 to lipid rafts potentiates TCR signaling", J Immunol, Vol. 173, pages 4847-4848, 2004, in particular, Fig. 6, which shows opposing effects of PKA and PDE4 on TCR activation). The molecule that connects these effectors is cyclic AMP (cAMP), an intracellular second messenger of extracellular ligand action. In T-cells, it mediates effects of prostaglandins, adenosine, histamine, betaadrenergic agonists, neuropeptide hormones and beta-endorphin. Binding of these extracellular molecules to GPCRs leads to their conformational change, release of stimulatory subunits and subsequent activation of adenylate cyclases (AC), which hydrolyze ATP to cAMP (see Fig. 6 of Abrahamsen H. et al., 2004, ibid). Although not the only one, PKA is the principal effector of cAMP signaling (see Mosenden R. and Tasken K., 2011, ibid, and Tasken K. and Ruppelt A., 2006, ibid). At a functional level, increased levels of cAMP lead to reduced IFNγ and IL-2 production in T-cells (see Abrahamsen H. et al., 2004, ibid). Aside from interfering with TCR activation, PKA has many more effector (see Fig. 15 of Torheim E.A., "Immunity Leashed - Mechanisms of Regulation in the Human Immune System", Thesis for the degree of Philosophiae Doctor (PhD), The Biotechnology Centre of Ola, University of Oslo, Norway, 2009).

In naive T-cells, hyperphosphorylated PAG targets Csk to lipid rafts. Via the Ezrin-EBP50-PAG scaffold complex PKA is targeted to Csk. Through specific phosphorylation by PKA, Csk can negatively regulate Lck and Fyn to dampen their activity and downregulate T-cell activation (see Fig. 6 of Abrahamsen H. et al., 2004, ibid). Upon TCR activation, PAG is dephosphorylated and Csk is released from the rafts. Dissociation of Csk is needed for T-cell activation to proceed. Within the same time course, a Csk-G3BP complex is formed and seems to sequester Csk outside lipid rafts (see Mosenden R. and Tasken K., 2011, ibid, and Tasken K. and Ruppelt A., 2006, ibid).

In contrast, combined TCR and CD28 stimulation mediates recruitment of the cyclic nucleotide phosphodiesterase PDE4 to lipid rafts, which enhances cAMP degradation (see Fig. 6 of Abrahamsen H. et al., 2004, ibid). As such, TCR induced production of cAMP is countered, and the T-cell immune response potentiated. Upon TCR stimulation alone, PDE4 recruitment may be too low to fully reduce the cAMP levels and therefore maximal T-cell activation cannot occur (see Abrahamsen H. et al., 2004, ibid).

Thus, by active suppression of proximal TCR signaling, signaling via cAMP-PKA-Csk is thought to set the threshold for T-cell activation. Recruitment of PDEs can counter this suppression. Tissue or cell-type specific regulation is accomplished through expression of multiple isoforms of AC, PKA, and PDEs. As mentioned above, the balance between activation and suppression needs to be tightly regulated to prevent development of autoimmune disorders, immune deficiencies and cancer.

**PDE4D7 correlated genes**

Phosphodiesterases (PDEs) provide the sole means for the degradation of the second messenger 3'-5'-cyclic AMP. As such they are poised to provide a key regulatory role. Thus, aberrant changes in their expression, activity and intracellular location may all contribute to the underlying molecular pathology of particular disease states. Indeed, it has recently been shown that mutations in PDE genes are enriched in prostate cancer patients leading to elevated cAMP signalling and a potential predisposition to prostate cancer. However, varied expression profiles in different cell types coupled with complex arrays of isoform variants within each PDE family makes understanding the links between aberrant changes in PDE expression and functionality during disease progression challenging. Several studies have endeavored to describe the complement of PDEs in prostate, all of which identified significant levels of PDE4 expression alongside other PDEs, leading to the development of a PDE4D7 biomarker (see Alves de Inda M. et al., "Validation of Cyclic Adenosine Monophosphate Phosphodiesterase-4D7 for its Independent Contribution to Risk Stratification in a Prostate Cancer Patient Cohort with Longitudinal Biological Outcomes", Eur Urol Focus, Vol. 4, No. 3, pages 376-384, 2018). Since the PDE4D7 biomarker has been proven to be a good predictor, we assumed that the ability to identify markers that are highly correlated with the PDE47 biomarker might also be helpful in prognosticating the outcome of certain cancer subjects.

**Selection of the genes**

The lists of genes have originally been selected by us for prognostication in prostate cancer subjects. In this document, it is shown that they are also of prognostic value with respect to an outcome of a bladder or kidney cancer subject.

The identified immune defense response genes ZBP1, and AIM2, APOBEC3A, CIAO1, DDX58, DHX9, IFI16, IFIH1, IFIT1, IFIT3, LRRFIP1, MYD88, OAS1, TLR8, and ZBP1, respectively, were identified as follows: A group of 538 prostate cancer patients were treated with RP and the prostate cancer tissue was stored together with clinical (e.g., pathological Gleason grade group (pGGG), pathology state (pT stage)) as well as relevant outcome parameters (e.g., biochemical recurrence (BCR), metastatic recurrence, prostate cancer specific death (PCa death), salvage radiation treatment (SRT), salvage androgen deprivation treatment (SADT), chemotherapy (CTX)). For each of these patients, a PDE4D7 score was calculated and categorized into four PDE4D7 score classes (see Alves de Inda M. et al., 2018, ibid). PDE4D7 score class 1 represents patient samples with lowest expression levels of PDE4D7, whereas PDE4D7 score class 4 represents patient samples with highest levels of PDE4D7 expression. RNASeq expression data (TPM - Transcripts Per Million) of the 538 prostate cancer subjects was then investigated for differential gene expression between the PDE4D7 score classes 1 and 4. In particular, it was determined for around 20,000 protein coding transcripts whether the mean expression level of the PDE4D7 score class 1 patients was more than twice as high as the mean expression level of the PDE4D7 score class 4 patients. This analysis resulted in 637 genes with a ratio PDE4D7 score class 1 / PDE4D7 score class 4 of > 2 with a minimum mean expression of 1 TPM in each of the four PDE4D7 score classes. These 637 genes were then further subjected to molecular pathway analysis (www.david.ncifcrf.gov), which resulted in a range of enriched annotation clusters. The annotation cluster #2 demonstrated enrichment (enrichment score: 10.8) in 30 genes with a function in defense response to viruses, negative regulation of viral genome replication as well as type I interferon signaling. A further heat map analysis confirmed that these immune defense response genes were generally higher expressed in samples from patients in PDE4D7 score class 1 than from patients in PDE4D7 score class 4. The class of genes with a function in defense response to viruses, negative regulation of viral genome replication as well as type I interferon signaling was further enriched to 61 genes by literature search to identify additional genes with the same molecular function. A further selection from the 61 genes was made based on the combinatorial power to separate patients who died from prostate cancer vs. those who did not, resulting of a preferred set of 14 genes. It was found that the number of events (metastases, prostate cancer specific death) was enriched in sub-cohorts with a low expression of these genes compared to the total patient cohort (#538) and a sub-cohort of 151 patients undergoing salvage RT (SRT) after post-surgical disease recurrence.

The identified T-Cell receptor signaling genes CD2, CD247, CD28, CD3E, CD3G, CD4, CSK, EZR, FYN, LAT, LCK, PAG1, PDE4D, PRKACA, PRKACB, PTPRC, and ZAP70 were identified as follows: A group of 538 prostate cancer patients were treated with RP and the prostate cancer tissue was stored together with clinical (e.g., pathological Gleason grade group (pGGG), pathology state (pT stage)) as well as relevant outcome parameters (e.g., biochemical recurrence (BCR), metastatic recurrence, prostate cancer specific death (PCa death), salvage radiation treatment (SRT), salvage androgen deprivation treatment (SADT), chemotherapy (CTX)). For each of these patients, a PDE4D7 score was calculated and categorized into four PDE4D7 score classes (see Alves de Inda M. et al., 2018, ibid). PDE4D7 score class 1 represents patient samples with lowest expression levels of PDE4D7, whereas PDE4D7 score class 4 represents patient samples with highest levels of PDE4D7 expression. RNASeq expression data (TPM - Transcripts Per Million) of the 538 prostate cancer subjects was then investigated for differential gene expression between the PDE4D7 score classes 1 and 4. In particular, it was determined for around 20,000 protein coding transcripts whether the mean expression level of the PDE4D7 score class 1 patients was more than twice as high as the mean expression level of the PDE4D7 score class 4 patients. This analysis resulted in 637 genes with a ratio PDE4D7 score class 1 / PDE4D7 score class 4 of > 2 with a minimum mean expression of 1 TPM in each of the four PDE4D7 score classes. These 637 genes were then further subjected to molecular pathway analysis (www.david.ncifcrf.gov), which resulted in a range of enriched annotation clusters. The annotation cluster #6 demonstrated enrichment (enrichment score: 5.9) in 17 genes with a function in primary immune deficiency and activation of T-Cell receptor signaling. A further heat map analysis confirmed that these T-Cell receptor signaling genes were generally higher expressed in samples from patients in PDE4D7 score class 1 than from patients in PDE4D7 score class 4.

The identified PDE4D7 correlated genes ABCC5, CUX2, KIAA1549, PDE4D, RAP1GAP2, SLC39A11, TDRD1, and VWA2 were identified as follows: We have identified in RNAseq data generated on 571 prostate cancer patients on close to 60,000 transcripts a range of genes that are correlated to the expression of the known biomarker PDE4D7 in this data. The correlation between the expression of any of these genes and PDE4D7 across the 571 samples was done by Pearson correlation and is expressed as a value between 0 to 1 in case of positive correlation or a value between -1 to 0 in case of negative correlation. As input data for the calculation of the correlation coefficient we used the PDE4D7 score (see Alves de Inda M. et al., 2018, ibid) and the RNAseq determined TPM gene expression value per gene of interest (see below).

The maximum negative correlation coefficient identified between the expression of any of the approximately 60,000 transcripts and the expression of PDE4D7 was -0.38 while the maximum positive correlation coefficient identified between the expression of any of the approximately 60,000 transcripts and the expression of PDE4D7 was +0.56. We selected genes in the range of correlation -0.31 to -0.38 as well as +0.41 to +0.56. We identified in total 77 transcripts matching these characteristics. From those 77 transcripts we selected the eight PDE4D7 correlated genes ABCC5, CUX2, KIAA1549, PDE4D, RAP1GAP2, SLC39A11, TDRD1, and VWA2 by testing Cox regression combination models iteratively in a sub-cohort of 186 patients who were undergoing salvage radiation treatment (SRT) due to post-surgical biochemical relapse. The clinical endpoint tested was prostate cancer specific death after start of SRT. The boundary condition for the selection of the eight genes was given by the restriction that the p-values in the multivariate Cox-regression were <0.1 for all genes retained in the model.

The term "ABCC5" refers to the human ATP binding cassette subfamily C member 5 gene (Ensembl: ENSG00000114770), for example, to the sequence as defined in NCBI Reference Sequence NM_001023587.2 or in NCBI Reference Sequence NM_005688.3, specifically, to the nucleotide sequence as set forth in SEQ ID NO:1 or in SEQ ID NO:2, which correspond to the sequences of the above indicated NCBI Reference Sequences of the ABCC5 transcript, and also relates to the corresponding amino acid sequence for example as set forth in SEQ ID NO:3 or in SEQ ID NO:4, which correspond to the protein sequences defined in NCBI Protein Accession Reference Sequence NP_001018881.1 and in NCBI Protein Accession Reference Sequence NP_005679 encoding the ABCC5 polypeptide.

The term "ABCC5" also comprises nucleotide sequences showing a high degree of homology to ABCC5, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 1 or in SEQ ID NO:2 or amino acid sequences being at least 75%, 80%, 85%, 90%, 91%, 92%, 93%, 94%, 95%, 96%, 97%, 98% or 99% identical to the sequence as set forth in SEQ ID NO:3 or in SEQ ID NO:4 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:3 or in SEQ ID NO:4 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 1 or in SEQ ID NO:2.

The term "AIM2" refers to the Absent in Melanoma 2 gene (Ensembl: ENSG00000163568), for example, to the sequence as defined in NCBI Reference Sequence NM_004833, specifically, to the nucleotide sequence as set forth in SEQ ID NO:5, which corresponds to the sequence of the above indicated NCBI Reference Sequence of the AIM2 transcript, and also relates to the corresponding amino acid sequence for example as set forth in SEQ ID NO:6, which corresponds to the protein sequence defined in NCBI Protein Accession Reference Sequence NP_004824 encoding the AIM2 polypeptide.

The term "AIM2" also comprises nucleotide sequences showing a high degree of homology to AIM2, e.g., nucleic acid sequences being at least 75%, 80%, 85%, 90%, 91%, 92%, 93%, 94%, 95%, 96%, 97%, 98% or 99% identical to the sequence as set forth in SEQ ID NO:5 or amino acid sequences being at least 75%, 80%, 85%, 90%, 91%, 92%, 93%, 94%, 95%, 96%, 97%, 98% or 99% identical to the sequence as set forth in SEQ ID NO:6 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:6 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:5.

The term "APOBEC3A" refers to the Apolipoprotein B mRNA Editing Enzyme Catalytic Subunit 3A gene (Ensembl: ENSG00000128383), for example, to the sequence as defined in NCBI Reference Sequence NM_145699, specifically, to the nucleotide sequence as set forth in SEQ ID NO:7, which corresponds to the sequence of the above indicated NCBI Reference Sequence of the APOBEC3A transcript, and also relates to the corresponding amino acid sequence for example as set forth in SEQ ID NO:8, which corresponds to the protein sequence defined in NCBI Protein Accession Reference Sequence NP_663745 encoding the APOBEC3A polypeptide.

The term "APOBEC3A" also comprises nucleotide sequences showing a high degree of homology to APOBEC3A, e.g., nucleic acid sequences being at least 75%, 80%, 85%, 90%, 91%, 92%, 93%, 94%, 95%, 96%, 97%, 98% or 99% identical to the sequence as set forth in SEQ ID NO:7 or amino acid sequences being at least 75%, 80%, 85%, 90%, 91%, 92%, 93%, 94%, 95%, 96%, 97%, 98% or 99% identical to the sequence as set forth in SEQ ID NO: 8 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 8 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:7.

The term "CD2" refers to the Cluster Of Differentiation 2 gene (Ensembl: ENSG00000116824), for example, to the sequence as defined in NCBI Reference Sequence NM_001767, specifically, to the nucleotide sequence as set forth in SEQ ID NO:9, which corresponds to the sequence of the above indicated NCBI Reference Sequence of the CD2 transcript, and also relates to the corresponding amino acid sequence for example as set forth in SEQ ID NO:10, which corresponds to the protein sequence defined in NCBI Protein Accession Reference Sequence NP_001758 encoding the CD2 polypeptide.

The term "CD2" also comprises nucleotide sequences showing a high degree of homology to CD2, e.g., nucleic acid sequences being at least 75%, 80%, 85%, 90%, 91%, 92%, 93%, 94%, 95%, 96%, 97%, 98% or 99% identical to the sequence as set forth in SEQ ID NO:9 or amino acid sequences being at least 75%, 80%, 85%, 90%, 91%, 92%, 93%, 94%, 95%, 96%, 97%, 98% or 99% identical to the sequence as set forth in SEQ ID NO:10 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:10 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:9.

The term "CD247" refers to the Cluster Of Differentiation 247 gene (Ensembl: ENSG00000198821), for example, to the sequence as defined in NCBI Reference Sequence NM_000734 or in NCBI Reference Sequence NM_198053, specifically, to the nucleotide sequence as set forth in SEQ ID NO:11 or in SEQ ID NO:12, which correspond to the sequences of the above indicated NCBI Reference Sequences of the CD247 transcript, and also relates to the corresponding amino acid sequence for example as set forth in SEQ ID NO:13 or in SEQ ID NO:14, which correspond to the protein sequences defined in NCBI Protein Accession Reference Sequence NP_000725 and in NCBI Protein Accession Reference Sequence NP_932170 encoding the CD247 polypeptide.

The term "CD247" also comprises nucleotide sequences showing a high degree of homology to CD247, e.g., nucleic acid sequences being at least 75%, 80%, 85%, 90%, 91%, 92%, 93%, 94%, 95%, 96%, 97%, 98% or 99% identical to the sequence as set forth in SEQ ID NO:11 or in SEQ ID NO:12 or amino acid sequences being at least 75%, 80%, 85%, 90%, 91%, 92%, 93%, 94%, 95%, 96%, 97%, 98% or 99% identical to the sequence as set forth in SEQ ID NO:13 or in SEQ ID NO:14 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:13 or in SEQ ID NO:14 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:11 or in SEQ ID NO:12.

The term "CD28" refers to the Cluster Of Differentiation 28 gene (Ensembl: ENSG00000178562), for example, to the sequence as defined in NCBI Reference Sequence NM_006139 or in NCBI Reference Sequence NM_001243078, specifically, to the nucleotide sequence as set forth in SEQ ID NO: 15 or in SEQ ID NO: 16, which correspond to the sequences of the above indicated NCBI Reference Sequences of the CD28 transcript, and also relates to the corresponding amino acid sequence for example as set forth in SEQ ID NO: 17 or in SEQ ID NO: 18, which correspond to the protein sequences defined in NCBI Protein Accession Reference Sequence NP_006130 and in NCBI Protein Accession Reference Sequence NP_001230007 encoding the CD28 polypeptide.

The term "CD28" also comprises nucleotide sequences showing a high degree of homology to CD28, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 15 or in SEQ ID NO: 16 or amino acid sequences being at least 75%, 80%, 85%, 90%, 91%, 92%, 93%, 94%, 95%, 96%, 97%, 98% or 99% identical to the sequence as set forth in SEQ ID NO: 17 or in SEQ ID NO: 18 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 17 or in SEQ ID NO: 18 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 15 or in SEQ ID NO: 16.

The term "CD3E" refers to the Cluster Of Differentiation 3E gene (Ensembl: ENSG00000198851), for example, to the sequence as defined in NCBI Reference Sequence NM_000733, specifically, to the nucleotide sequence as set forth in SEQ ID NO: 19, which corresponds to the sequence of the above indicated NCBI Reference Sequence of the CD3E transcript, and also relates to the corresponding amino acid sequence for example as set forth in SEQ ID NO:20, which corresponds to the protein sequence defined in NCBI Protein Accession Reference Sequence NP_000724 encoding the CD3E polypeptide.

The term "CD3E" also comprises nucleotide sequences showing a high degree of homology to CD3E, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 19 or amino acid sequences being at least 75%, 80%, 85%, 90%, 91%, 92%, 93%, 94%, 95%, 96%, 97%, 98% or 99% identical to the sequence as set forth in SEQ ID NO:20 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:20 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 19.

The term "CD3G" refers to the Cluster Of Differentiation 3G gene (Ensembl: ENSG00000160654), for example, to the sequence as defined in NCBI Reference Sequence NM_000073, specifically, to the nucleotide sequence as set forth in SEQ ID NO:21, which corresponds to the sequence of the above indicated NCBI Reference Sequence of the CD3G transcript, and also relates to the corresponding amino acid sequence for example as set forth in SEQ ID NO:22, which corresponds to the protein sequence defined in NCBI Protein Accession Reference Sequence NP_000064 encoding the CD3G polypeptide.

The term "CD3G" also comprises nucleotide sequences showing a high degree of homology to CD3G, e.g., nucleic acid sequences being at least 75%, 80%, 85%, 90%, 91%, 92%, 93%, 94%, 95%, 96%, 97%, 98% or 99% identical to the sequence as set forth in SEQ ID NO:21 or amino acid sequences being at least 75%, 80%, 85%, 90%, 91%, 92%, 93%, 94%, 95%, 96%, 97%, 98% or 99% identical to the sequence as set forth in SEQ ID NO:22 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:22 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:21.

The term "CD4" refers to the Cluster Of Differentiation 4 gene (Ensembl: ENSG00000010610), for example, to the sequence as defined in NCBI Reference Sequence NM_000616, specifically, to the nucleotide sequence as set forth in SEQ ID NO:23, which corresponds to the sequence of the above indicated NCBI Reference Sequence of the CD4 transcript, and also relates to the corresponding amino acid sequence for example as set forth in SEQ ID NO:24, which corresponds to the protein sequence defined in NCBI Protein Accession Reference Sequence NP_000607 encoding the CD4 polypeptide.

The term "CD4" also comprises nucleotide sequences showing a high degree of homology to CD4, e.g., nucleic acid sequences being at least 75%, 80%, 85%, 90%, 91%, 92%, 93%, 94%, 95%, 96%, 97%, 98% or 99% identical to the sequence as set forth in SEQ ID NO:23 or amino acid sequences being at least 75%, 80%, 85%, 90%, 91%, 92%, 93%, 94%, 95%, 96%, 97%, 98% or 99% identical to the sequence as set forth in SEQ ID NO:24 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:24 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:23.

The term "CIAO1" refers to the Cytosolic Iron-Sulfur Assembly Component 1 gene (Ensembl: ENSG00000144021), for example, to the sequence as defined in NCBI Reference Sequence NM_004804, specifically, to the nucleotide sequence as set forth in SEQ ID NO:25, which corresponds to the sequence of the above indicated NCBI Reference Sequence of the CIAO1 transcript, and also relates to the corresponding amino acid sequence for example as set forth in SEQ ID NO:26, which corresponds to the protein sequence defined in NCBI Protein Accession Reference Sequence NP_663745 encoding the CIAO1 polypeptide.

The term "CIAO1" also comprises nucleotide sequences showing a high degree of homology to CIAO1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:25 or amino acid sequences being at least 75%, 80%, 85%, 90%, 91%, 92%, 93%, 94%, 95%, 96%, 97%, 98% or 99% identical to the sequence as set forth in SEQ ID NO:26 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:26 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:25.

The term "CSK" refers to the C-Terminal Src Kinase gene (Ensembl: ENSG00000103653), for example, to the sequence as defined in NCBI Reference Sequence NM_004383, specifically, to the nucleotide sequence as set forth in SEQ ID NO:27, which corresponds to the sequence of the above indicated NCBI Reference Sequence of the CSK transcript, and also relates to the corresponding amino acid sequence for example as set forth in SEQ ID NO:28, which corresponds to the protein sequence defined in NCBI Protein Accession Reference Sequence NP_004374 encoding the CSK polypeptide.

The term "CSK" also comprises nucleotide sequences showing a high degree of homology to CSK, e.g., nucleic acid sequences being at least 75%, 80%, 85%, 90%, 91%, 92%, 93%, 94%, 95%, 96%, 97%, 98% or 99% identical to the sequence as set forth in SEQ ID NO:27 or amino acid sequences being at least 75%, 80%, 85%, 90%, 91%, 92%, 93%, 94%, 95%, 96%, 97%, 98% or 99% identical to the sequence as set forth in SEQ ID NO:28 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:28 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:27.

The term "CUX2" refers to the human Cut Like Homeobox 2 gene (Ensembl: ENSG00000111249), for example, to the sequence as defined in NCBI Reference Sequence NM_015267.3, specifically, to the nucleotide sequence as set forth in SEQ ID NO:29, which corresponds to the sequence of the above indicated NCBI Reference Sequence of the CUX2 transcript, and also relates to the corresponding amino acid sequence for example as set forth in SEQ ID NO:30, which corresponds to the protein sequence defined in NCBI Protein Accession Reference Sequence NP_056082.2 encoding the CUX2 polypeptide.

The term "CUX2" also comprises nucleotide sequences showing a high degree of homology to CUX2, e.g., nucleic acid sequences being at least 75%, 80%, 85%, 90%, 91%, 92%, 93%, 94%, 95%, 96%, 97%, 98% or 99% identical to the sequence as set forth in SEQ ID NO:29 or amino acid sequences being at least 75%, 80%, 85%, 90%, 91%, 92%, 93%, 94%, 95%, 96%, 97%, 98% or 99% identical to the sequence as set forth in SEQ ID NO:30 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:30 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:29.

The term "DDX58" refers to the DExD/H-box Helicase 58 gene (Ensembl: ENSG00000107201), for example, to the sequence as defined in NCBI Reference Sequence NM_014314, specifically, to the nucleotide sequence as set forth in SEQ ID NO:31, which corresponds to the sequence of the above indicated NCBI Reference Sequence of the DDX58 transcript, and also relates to the corresponding amino acid sequence for example as set forth in SEQ ID NO:32, which corresponds to the protein sequence defined in NCBI Protein Accession Reference Sequence NP_055129 encoding the DDX58 polypeptide.

The term "DDX58" also comprises nucleotide sequences showing a high degree of homology to DDX58, e.g., nucleic acid sequences being at least 75%, 80%, 85%, 90%, 91%, 92%, 93%, 94%, 95%, 96%, 97%, 98% or 99% identical to the sequence as set forth in SEQ ID NO:31 or amino acid sequences being at least 75%, 80%, 85%, 90%, 91%, 92%, 93%, 94%, 95%, 96%, 97%, 98% or 99% identical to the sequence as set forth in SEQ ID NO:32 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:32 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:31.

The term "DHX9" refers to the DExD/H-box Helicase 9 gene (Ensembl: ENSG00000135829), for example, to the sequence as defined in NCBI Reference Sequence NM_001357, specifically, to the nucleotide sequence as set forth in SEQ ID NO:33, which corresponds to the sequence of the above indicated NCBI Reference Sequence of the DHX9 transcript, and also relates to the corresponding amino acid sequence for example as set forth in SEQ ID NO:34, which corresponds to the protein sequence defined in NCBI Protein Accession Reference Sequence NP_001348 encoding the DHX9 polypeptide.

The term "DHX9" also comprises nucleotide sequences showing a high degree of homology to DHX9, e.g., nucleic acid sequences being at least 75%, 80%, 85%, 90%, 91%, 92%, 93%, 94%, 95%, 96%, 97%, 98% or 99% identical to the sequence as set forth in SEQ ID NO:33 or amino acid sequences being at least 75%, 80%, 85%, 90%, 91%, 92%, 93%, 94%, 95%, 96%, 97%, 98% or 99% identical to the sequence as set forth in SEQ ID NO:34 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:34 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:33.

The term "EZR" refers to the Ezrin gene (Ensembl: ENSG00000092820), for example, to the sequence as defined in NCBI Reference Sequence NM_003379, specifically, to the nucleotide sequence as set forth in SEQ ID NO:35, which corresponds to the sequence of the above indicated NCBI Reference Sequence of the EZR transcript, and also relates to the corresponding amino acid sequence for example as set forth in SEQ ID NO:36, which corresponds to the protein sequence defined in NCBI Protein Accession Reference Sequence NP_003370 encoding the EZR polypeptide.

The term "EZR" also comprises nucleotide sequences showing a high degree of homology to EZR, e.g., nucleic acid sequences being at least 75%, 80%, 85%, 90%, 91%, 92%, 93%, 94%, 95%, 96%, 97%, 98% or 99% identical to the sequence as set forth in SEQ ID NO:35 or amino acid sequences being at least 75%, 80%, 85%, 90%, 91%, 92%, 93%, 94%, 95%, 96%, 97%, 98% or 99% identical to the sequence as set forth in SEQ ID NO:36 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:36 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:35.

The term "FYN" refers to the FYN Proto-Oncogene gene (Ensembl: ENSG00000010810), for example, to the sequence as defined in NCBI Reference Sequence NM_002037 or in NCBI Reference Sequence NM_153047 or in NCBI Reference Sequence NM_153048, specifically, to the nucleotide sequence as set forth in SEQ ID NO:37 or in SEQ ID NO:38 or in SEQ ID NO:39, which correspond to the sequences of the above indicated NCBI Reference Sequences of the FYN transcript, and also relates to the corresponding amino acid sequence for example as set forth in SEQ ID NO:40 or in SEQ ID NO:41 or in SEQ ID NO:42, which correspond to the protein sequences defined in NCBI Protein Accession Reference Sequence NP_002028 and in NCBI Protein Accession Reference Sequence NP_694592 and in NCBI Protein Accession Reference Sequence XP_005266949 encoding the FYN polypeptide.

The term "FYN" also comprises nucleotide sequences showing a high degree of homology to FYN, e.g., nucleic acid sequences being at least 75%, 80%, 85%, 90%, 91%, 92%, 93%, 94%, 95%, 96%, 97%, 98% or 99% identical to the sequence as set forth in SEQ ID NO:37 or in SEQ ID NO:38 or in SEQ ID NO:39 or amino acid sequences being at least 75%, 80%, 85%, 90%, 91%, 92%, 93%, 94%, 95%, 96%, 97%, 98% or 99% identical to the sequence as set forth in SEQ ID NO:40 or in SEQ ID NO:41 or in SEQ ID NO:42 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:40 or in SEQ ID NO:41 or in SEQ ID NO:42 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:37 or in SEQ ID NO:38 or in SEQ ID NO:39.

The term "IFI16" refers to the Interferon Gamma Inducible Protein 16 gene (Ensembl: ENSG00000163565), for example, to the sequence as defined in NCBI Reference Sequence NM_005531, specifically, to the nucleotide sequence as set forth in SEQ ID NO:43, which corresponds to the sequence of the above indicated NCBI Reference Sequence of the IFI16 transcript, and also relates to the corresponding amino acid sequence for example as set forth in SEQ ID NO:44, which corresponds to the protein sequence defined in NCBI Protein Accession Reference Sequence NP_005522 encoding the IFI16 polypeptide.

The term "IFI16" also comprises nucleotide sequences showing a high degree of homology to IFI16, e.g., nucleic acid sequences being at least 75%, 80%, 85%, 90%, 91%, 92%, 93%, 94%, 95%, 96%, 97%, 98% or 99% identical to the sequence as set forth in SEQ ID NO:43 or amino acid sequences being at least 75%, 80%, 85%, 90%, 91%, 92%, 93%, 94%, 95%, 96%, 97%, 98% or 99% identical to the sequence as set forth in SEQ ID NO:44 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:44 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:43.

The term "IFIH1" refers to the Interferon Induced With Helicase C Domain 1 gene (Ensembl: ENSG00000115267), for example, to the sequence as defined in NCBI Reference Sequence NM_022168, specifically, to the nucleotide sequence as set forth in SEQ ID NO:45, which corresponds to the sequence of the above indicated NCBI Reference Sequence of the IFIH1 transcript, and also relates to the corresponding amino acid sequence for example as set forth in SEQ ID NO:46, which corresponds to the protein sequence defined in NCBI Protein Accession Reference Sequence NP_071451 encoding the IFIH1 polypeptide.

The term "IFIH1" also comprises nucleotide sequences showing a high degree of homology to IFIH1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:45 or amino acid sequences being at least 75%, 80%, 85%, 90%, 91%, 92%, 93%, 94%, 95%, 96%, 97%, 98% or 99% identical to the sequence as set forth in SEQ ID NO:46 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:46 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:45.

The term "IFIT1" refers to the Interferon Induced Protein With Tetratricopeptide Repeats 1 gene (Ensembl: ENSG00000185745), for example, to the sequence as defined in NCBI Reference Sequence NM_001270929 or in NCBI Reference Sequence NM_001548.5, specifically, to the nucleotide sequence as set forth in SEQ ID NO:47 or in SEQ ID NO:48, which correspond to the sequences of the above indicated NCBI Reference Sequences of the IFIT1 transcript, and also relates to the corresponding amino acid sequence for example as set forth in SEQ ID NO:49 or in SEQ ID NO:50, which correspond to the protein sequences defined in NCBI Protein Accession Reference Sequence NP_001257858 and in NCBI Protein Accession Reference Sequence NP_001539 encoding the IFIT1 polypeptide.

The term "IFIT1" also comprises nucleotide sequences showing a high degree of homology to IFIT1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:47 or in SEQ ID NO:48 or amino acid sequences being at least 75%, 80%, 85%, 90%, 91%, 92%, 93%, 94%, 95%, 96%, 97%, 98% or 99% identical to the sequence as set forth in SEQ ID NO:49 or in SEQ ID NO:50 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:49 or SEQ ID NO:50 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:47 or in SEQ ID NO:48.

The term "IFIT3" refers to the Interferon Induced Protein With Tetratricopeptide Repeats 3 gene (Ensembl: ENSG00000119917), for example, to the sequence as defined in NCBI Reference Sequence NM_001031683, specifically, to the nucleotide sequence as set forth in SEQ ID NO:51, which corresponds to the sequence of the above indicated NCBI Reference Sequence of the IFIT3 transcript, and also relates to the corresponding amino acid sequence for example as set forth in SEQ ID NO:52, which corresponds to the protein sequence defined in NCBI Protein Accession Reference Sequence NP_001026853 encoding the IFIT3 polypeptide.

The term "IFIT3" also comprises nucleotide sequences showing a high degree of homology to IFIT3, e.g., nucleic acid sequences being at least 75%, 80%, 85%, 90%, 91%, 92%, 93%, 94%, 95%, 96%, 97%, 98% or 99% identical to the sequence as set forth in SEQ ID NO:51 or amino acid sequences being at least 75%, 80%, 85%, 90%, 91%, 92%, 93%, 94%, 95%, 96%, 97%, 98% or 99% identical to the sequence as set forth in SEQ ID NO:52 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:52 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:51.

The term "KIAA1549" refers to the human KIAA1549 gene (Ensembl: ENSG00000122778), for example, to the sequence as defined in NCBI Reference Sequence NM_020910 or in NCBI Reference Sequence NM_001164665, specifically, to the nucleotide sequence as set forth in SEQ ID NO:53 or in SEQ ID NO:54, which correspond to the sequences of the above indicated NCBI Reference Sequence of the KIAA1549 transcript, and also relates to the corresponding amino acid sequence for example as set forth in SEQ ID NO:55 or in SEQ ID NO:56, which correspond to the protein sequences defined in NCBI Protein Accession Reference Sequence NP_065961 and in NCBI Protein Accession Reference Sequence NP_001158137 encoding the KIAA1549 polypeptide.

The term "KIAA1549" also comprises nucleotide sequences showing a high degree of homology to KIAA1549, e.g., nucleic acid sequences being at least 75%, 80%, 85%, 90%, 91%, 92%, 93%, 94%, 95%, 96%, 97%, 98% or 99% identical to the sequence as set forth in SEQ ID NO:53 or in SEQ ID NO:54 or amino acid sequences being at least 75%, 80%, 85%, 90%, 91%, 92%, 93%, 94%, 95%, 96%, 97%, 98% or 99% identical to the sequence as set forth in SEQ ID NO:55 or in SEQ ID NO:56 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:55 or in SEQ ID NO:56 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:53 or in SEQ ID NO:54.

The term "LAT" refers to the Linker For Activation Of T-Cells gene (Ensembl: ENSG00000213658), for example, to the sequence as defined in NCBI Reference Sequence NM_001014987 or in NCBI Reference Sequence NM_014387, specifically, to the nucleotide sequence as set forth in SEQ ID NO:57 or in SEQ ID NO:58, which correspond to the sequences of the above indicated NCBI Reference Sequences of the LAT transcript, and also relates to the corresponding amino acid sequence for example as set forth in SEQ ID NO:59 or in SEQ ID NO:60, which corresponds to the protein sequences defined in NCBI Protein Accession Reference Sequence NP_001014987 and in NCBI Protein Accession Reference Sequence NP_055202 encoding the LAT polypeptide.

The term "LAT" also comprises nucleotide sequences showing a high degree of homology to LAT, e.g., nucleic acid sequences being at least 75%, 80%, 85%, 90%, 91%, 92%, 93%, 94%, 95%, 96%, 97%, 98% or 99% identical to the sequence as set forth in SEQ ID NO:57 or in SEQ ID NO:58 or amino acid sequences being at least 75%, 80%, 85%, 90%, 91%, 92%, 93%, 94%, 95%, 96%, 97%, 98% or 99% identical to the sequence as set forth in SEQ ID NO:59 or in SEQ ID NO:60 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:59 or in SEQ ID NO:60 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:57 or in SEQ ID NO:58.

The term "LCK" refers to the LCK Proto-Oncogene gene (Ensembl: ENSG00000182866), for example, to the sequence as defined in NCBI Reference Sequence NM_005356, specifically, to the nucleotide sequence as set forth in SEQ ID NO:61, which corresponds to the sequence of the above indicated NCBI Reference Sequence of the LCK transcript, and also relates to the corresponding amino acid sequence for example as set forth in SEQ ID NO:62, which corresponds to the protein sequence defined in NCBI Protein Accession Reference Sequence NP_005347 encoding the LCK polypeptide.

The term "LCK" also comprises nucleotide sequences showing a high degree of homology to LCK, e.g., nucleic acid sequences being at least 75%, 80%, 85%, 90%, 91%, 92%, 93%, 94%, 95%, 96%, 97%, 98% or 99% identical to the sequence as set forth in SEQ ID NO:61 or amino acid sequences being at least 75%, 80%, 85%, 90%, 91%, 92%, 93%, 94%, 95%, 96%, 97%, 98% or 99% identical to the sequence as set forth in SEQ ID NO:62 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:62 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:61.

The term "LRRFIP1" refers to the LRR Binding FLII Interacting Protein 1 gene (Ensembl: ENSG00000124831), for example, to the sequence as defined in NCBI Reference Sequence NM_004735 or in NCBI Reference Sequence NM_001137550 or in NCBI Reference Sequence NM_001137553 or in NCBI Reference Sequence NM_001137552, specifically, to the nucleotide sequence as set forth in SEQ ID NO:63 or in SEQ ID NO:64 or in SEQ ID NO:65 or in SEQ ID NO:66, which correspond to the sequences of the above indicated NCBI Reference Sequences of the LRRFIP1 transcript, and also relates to the corresponding amino acid sequence for example as set forth in SEQ ID NO:67 or in SEQ ID NO:68 or in SEQ ID NO:69 or in SEQ ID NO:70, which correspond to the protein sequences defined in NCBI Protein Accession Reference Sequence NP_004726 and in NCBI Protein Accession Reference Sequence NP_001131022 and in NCBI Protein Accession Reference Sequence NP_001131025 and in NCBI Protein Accession Reference Sequence NP_001131024 encoding the LRRFIP1 polypeptide.

The term "LRRFIP1" also comprises nucleotide sequences showing a high degree of homology to LRRFIP1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:63 or in SEQ ID NO:64 or in SEQ ID NO:65 or in SEQ ID NO:66 or amino acid sequences being at least 75%, 80%, 85%, 90%, 91%, 92%, 93%, 94%, 95%, 96%, 97%, 98% or 99% identical to the sequence as set forth in SEQ ID NO:67 or in SEQ ID NO:68 or in SEQ ID NO:69 or in SEQ ID NO:70 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:67 or in SEQ ID NO:68 or in SEQ ID NO:69 or in SEQ ID NO:70 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:63 or in SEQ ID NO:64 or in SEQ ID NO:65 or in SEQ ID NO:66.

The term "MYD88" refers to the MYD88 Innate Immune Signal Transduction Adaptor gene (Ensembl: ENSG00000172936), for example, to the sequence as defined in NCBI Reference Sequence NM_001172567 or in NCBI Reference Sequence NM_001172568 or in NCBI Reference Sequence NM_001172569 or in NCBI Reference Sequence NM_001172566 or in NCBI Reference Sequence NM_002468, specifically, to the nucleotide sequences as set forth in SEQ ID NO:71 or in SEQ ID NO:72 or in SEQ ID NO:73 or in SEQ ID NO:74 or in SEQ ID NO:75, which correspond to the sequences of the above indicated NCBI Reference Sequences of the MYD88 transcript, and also relates to the corresponding amino acid sequence for example as set forth in SEQ ID NO:76 or in SEQ ID NO:77 or in SEQ ID NO:78 or in SEQ ID NO:79 or in SEQ ID NO:80, which correspond to the protein sequences defined in NCBI Protein Accession Reference Sequence NP_001166038 and in NCBI Protein Accession Reference Sequence NP_001166039 and in NCBI Protein Accession Reference Sequence NP_001166040 and in NCBI Protein Accession Reference Sequence NP_001166037 and in NCBI Protein Accession Reference Sequence NP_002459 encoding the MYD88 polypeptide.

The term "MYD88" also comprises nucleotide sequences showing a high degree of homology to MYD88, e.g., nucleic acid sequences being at least 75%, 80%, 85%, 90%, 91%, 92%, 93%, 94%, 95%, 96%, 97%, 98% or 99% identical to the sequence as set forth in SEQ ID NO:71 or in SEQ ID NO:72 or in SEQ ID NO:73 or in SEQ ID NO:74 or in SEQ ID NO:75 or amino acid sequences being at least 75%, 80%, 85%, 90%, 91%, 92%, 93%, 94%, 95%, 96%, 97%, 98% or 99% identical to the sequence as set forth in SEQ ID NO:76 or in SEQ ID NO:77 or in SEQ ID NO:78 or in SEQ ID NO:79 or in SEQ ID NO:80 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:76 or in SEQ ID NO:77 or in SEQ ID NO:78 or in SEQ ID NO:79 or in SEQ ID NO:80 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:71 or in SEQ ID NO:72 or in SEQ ID NO:73 or in SEQ ID NO:74 or in SEQ ID NO:75.

The term "OAS1" refers to the 2'-5'-Oligoadenylate Synthetase 1 gene (Ensembl: ENSG00000089127), for example, to the sequence as defined in NCBI Reference Sequence NM_001320151 or in NCBI Reference Sequence NM_002534 or in NCBI Reference Sequence NM_001032409 or in NCBI Reference Sequence NM_016816, specifically, to the nucleotide sequences as set forth in SEQ ID NO:81 or in SEQ ID NO:82 or in SEQ ID NO:83 or in SEQ ID NO:84, which correspond to the sequences of the above indicated NCBI Reference Sequences of the OAS1 transcript, and also relates to the corresponding amino acid sequences for example as set forth in SEQ ID NO:85 or in SEQ ID NO:86 or in SEQ ID NO:87 or in SEQ ID NO:88, which correspond to the protein sequences defined in NCBI Protein Accession Reference Sequence NP_001307080 and in NCBI Protein Accession Reference Sequence NP_002525 and in NCBI Protein Accession Reference Sequence NP _001027581 and in NCBI Protein Accession Reference Sequence NP_058132 encoding the OAS1 polypeptide.

The term "OAS1" also comprises nucleotide sequences showing a high degree of homology to OAS1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:81 or in SEQ ID NO:82 or in SEQ ID NO:83 or in SEQ ID NO:84 or amino acid sequences being at least 75%, 80%, 85%, 90%, 91%, 92%, 93%, 94%, 95%, 96%, 97%, 98% or 99% identical to the sequence as set forth in SEQ ID NO:85 or in SEQ ID NO:86 or in SEQ ID NO:87 or in SEQ ID NO:88 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:85 or in SEQ ID NO:86 or in SEQ ID NO:87 or in SEQ ID NO:88 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:81 or in SEQ ID NO:82 or in SEQ ID NO:83 or in SEQ ID NO:84.

The term "PAG1" refers to the Phosphoprotein Membrane Anchor With Glycosphingolipid Microdomains 1 gene (Ensembl: ENSG00000076641), for example, to the sequence as defined in NCBI Reference Sequence NM_018440, specifically, to the nucleotide sequence as set forth in SEQ ID NO:89, which corresponds to the sequence of the above indicated NCBI Reference Sequence of the PAG1 transcript, and also relates to the corresponding amino acid sequence for example as set forth in SEQ ID NO:90, which corresponds to the protein sequence defined in NCBI Protein Accession Reference Sequence NP_060910 encoding the PAG1 polypeptide.

The term "PAG1" also comprises nucleotide sequences showing a high degree of homology to PAG1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:89 or amino acid sequences being at least 75%, 80%, 85%, 90%, 91%, 92%, 93%, 94%, 95%, 96%, 97%, 98% or 99% identical to the sequence as set forth in SEQ ID NO:90 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:90 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:89.

The term "PDE4D" refers to the human Phosphodiesterase 4D gene (Ensembl: ENSG00000113448), for example, to the sequence as defined in NCBI Reference Sequence NM_001104631 or in NCBI Reference Sequence NM_001349242 or in NCBI Reference Sequence NM_001197218 or in NCBI Reference Sequence NM_006203 or in NCBI Reference Sequence NM_001197221 or in NCBI Reference Sequence NM_001197220 or in NCBI Reference Sequence NM_001197223 or in NCBI Reference Sequence NM_001165899 or in NCBI Reference Sequence NM_001165899, specifically, to the nucleotide sequence as set forth in SEQ ID NO:91 or in SEQ ID NO:92 or in SEQ ID NO:93 or in SEQ ID NO:94 or in SEQ ID NO:95 or in SEQ ID NO:96 or in SEQ ID NO:97 or in SEQ ID NO:98 or in SEQ ID NO:99, which correspond to the sequences of the above indicated NCBI Reference Sequence of the PDE4D transcript, and also relates to the corresponding amino acid sequence for example as set forth in SEQ ID NO:100 or in SEQ ID NO:101 or in SEQ ID NO:102 or in SEQ ID NO:103 or in SEQ ID NO:104 or in SEQ ID NO:105 or in SEQ ID NO:106 or in SEQ ID NO:107 or in SEQ ID NO:108, which correspond to the protein sequences defined in NCBI Protein Accession Reference Sequence NP_001098101 and in NCBI Protein Accession Reference Sequence NP_001336171 and in NCBI Protein Accession Reference Sequence NP_001184147 and in NCBI Protein Accession Reference Sequence NP_006194 and in NCBI Protein Accession Reference Sequence NP_001184150 and in NCBI Protein Accession Reference Sequence NP_001184149 and in NCBI Protein Accession Reference Sequence NP_001184152 and in NCBI Protein Accession Reference Sequence NP_001159371 and in NCBI Protein Accession Reference Sequence NP_001184148 encoding the PDE4D polypeptide.

The term "PDE4D" also comprises nucleotide sequences showing a high degree of homology to PDE4D, e.g., nucleic acid sequences being at least 75%, 80%, 85%, 90%, 91%, 92%, 93%, 94%, 95%, 96%, 97%, 98% or 99% identical to the sequence as set forth in SEQ ID NO:91 or in SEQ ID NO:92 or in SEQ ID NO:93 or in SEQ ID NO:94 or in SEQ ID NO:95 or in SEQ ID NO:96 or in SEQ ID NO:97 or in SEQ ID NO:98 or in SEQ ID NO:99 or amino acid sequences being at least 75%, 80%, 85%, 90%, 91%, 92%, 93%, 94%, 95%, 96%, 97%, 98% or 99% identical to the sequence as set forth in SEQ ID NO:100 or in SEQ ID NO:101 or in SEQ ID NO:102 or in SEQ ID NO:103 or in SEQ ID NO:104 or in SEQ ID NO:105 or in SEQ ID NO:106 or in SEQ ID NO:107 or in SEQ ID NO:108 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:100 or in SEQ ID NO:101 or in SEQ ID NO:102 or in SEQ ID NO:103 or in SEQ ID NO:104 or in SEQ ID NO:105 or in SEQ ID NO:106 or in SEQ ID NO:107 or in SEQ ID NO:108 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:91 or in SEQ ID NO:92 or in SEQ ID NO:93 or in SEQ ID NO:94 or in SEQ ID NO:95 or in SEQ ID NO:96 or in SEQ ID NO:97 or in SEQ ID NO:98 or in SEQ ID NO:99.

The term "PRKACA" refers to the Protein Kinase cAMP-Activated Catalytic Subunit Alpha gene (Ensembl: ENSG00000072062), for example, to the sequence as defined in NCBI Reference Sequence NM_002730 or in NCBI Reference Sequence NM_207518, specifically, to the nucleotide sequences as set forth in SEQ ID NO:109 or in SEQ ID NO:110, which correspond to the sequences of the above indicated NCBI Reference Sequences of the PRKACA transcript, and also relates to the corresponding amino acid sequence for example as set forth in SEQ ID NO:111 or in SEQ ID NO:112, which correspond to the protein sequences defined in NCBI Protein Accession Reference Sequence NP_002721 and in NCBI Protein Accession Reference Sequence NP_997401 encoding the PRKACA polypeptide.

The term "PRKACA" also comprises nucleotide sequences showing a high degree of homology to PRKACA, e.g., nucleic acid sequences being at least 75%, 80%, 85%, 90%, 91%, 92%, 93%, 94%, 95%, 96%, 97%, 98% or 99% identical to the sequence as set forth in SEQ ID NO:109 or in SEQ ID NO:110 or amino acid sequences being at least 75%, 80%, 85%, 90%, 91%, 92%, 93%, 94%, 95%, 96%, 97%, 98% or 99% identical to the sequence as set forth in SEQ ID NO:111 or in SEQ ID NO:112 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:111 or in SEQ ID NO:112 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:109 or in SEQ ID NO:110.

The term "PRKACB" refers to the Protein Kinase cAMP-Activated Catalytic Subunit Beta gene (Ensembl: ENSG00000142875), for example, to the sequence as defined in NCBI Reference Sequence NM_002731 or in NCBI Reference Sequence NM_182948 or in NCBI Reference Sequence NM_001242860 or in NCBI Reference Sequence NM_001242859 or in NCBI Reference Sequence NM_001242858 or in NCBI Reference Sequence NM_001242862 or in NCBI Reference Sequence NM_001242861 or in NCBI Reference Sequence NM_001300915 or in NCBI Reference Sequence NM_207578 or in NCBI Reference Sequence NM_001242857 or in NCBI Reference Sequence NM_001300917, specifically, to the nucleotide sequence as set forth in SEQ ID NO:113 or in SEQ ID NO:114 or in SEQ ID NO:115 or in SEQ ID NO:116 or in SEQ ID NO:117 or in SEQ ID NO:118 or in SEQ ID NO:119 or in SEQ ID NO:120 or in SEQ ID NO:121 or in SEQ ID NO:122 or in SEQ ID NO:123, which correspond to the sequences of the above indicated NCBI Reference Sequences of the PRKACB transcript, and also relates to the corresponding amino acid sequence for example as set forth in SEQ ID NO:124 or in SEQ ID NO:125 or in SEQ ID NO:126 or in SEQ ID NO:127 or in SEQ ID NO:128 or in SEQ ID NO:129 or in SEQ ID NO:130 or in SEQ ID NO:131 or in SEQ ID NO:132 or in SEQ ID NO:133 or in SEQ ID NO:134, which correspond to the protein sequences defined in NCBI Protein Accession Reference Sequence NP_002722 and in NCBI Protein Accession Reference Sequence NP_891993 and in NCBI Protein Accession Reference Sequence NP_001229789 and in NCBI Protein Accession Reference Sequence NP_001229788 and in NCBI Protein Accession Reference Sequence NP_001229787 and in NCBI Protein Accession Reference Sequence NP_001229791 and in NCBI Protein Accession Reference Sequence NP_001229790 and in NCBI Protein Accession Reference Sequence NP_001287844 and in NCBI Protein Accession Reference Sequence NP_997461 and in NCBI Protein Accession Reference Sequence NP_001229786 and in NCBI Protein Accession Reference Sequence NP_001287846 encoding the PRKACB polypeptide.

The term "PRKACB" also comprises nucleotide sequences showing a high degree of homology to PRKACB, e.g., nucleic acid sequences being at least 75%, 80%, 85%, 90%, 91%, 92%, 93%, 94%, 95%, 96%, 97%, 98% or 99% identical to the sequence as set forth in SEQ ID NO:113 or in SEQ ID NO:114 or in SEQ ID NO:115 or in SEQ ID NO:116 or in SEQ ID NO:117 or in SEQ ID NO:118 or in SEQ ID NO:119 or in SEQ ID NO:120 or in SEQ ID NO:121 or in SEQ ID NO:122 or in SEQ ID NO:123 or amino acid sequences being at least 75%, 80%, 85%, 90%, 91%, 92%, 93%, 94%, 95%, 96%, 97%, 98% or 99% identical to the sequence as set forth in SEQ ID NO:124 or in SEQ ID NO:125 or in SEQ ID NO:126 or in SEQ ID NO:127 or in SEQ ID NO:128 or in SEQ ID NO:129 or in SEQ ID NO:130 or in SEQ ID NO:131 or in SEQ ID NO:132 or in SEQ ID NO:133 or in SEQ ID NO:134 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:124 or in SEQ ID NO:125 or in SEQ ID NO:126 or in SEQ ID NO:127 or in SEQ ID NO:128 or in SEQ ID NO:129 or in SEQ ID NO:130 or in SEQ ID NO:131 or in SEQ ID NO:132 or in SEQ ID NO:133 or in SEQ ID NO:134 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:113 or in SEQ ID NO:114 or in SEQ ID NO:115 or in SEQ ID NO:116 or in SEQ ID NO:117 or in SEQ ID NO:118 or in SEQ ID NO:119 or in SEQ ID NO:120 or in SEQ ID NO:121 or in SEQ ID NO:122 or in SEQ ID NO:123.

The term "PTPRC" refers to the Protein Tyrosine Phosphatase Receptor Type C gene (Ensembl: ENSG00000081237), for example, to the sequence as defined in NCBI Reference Sequence NM_002838 or in NCBI Reference Sequence NM_080921, specifically, to the nucleotide sequence as set forth in SEQ ID NO:135 or in SEQ ID NO:136, which correspond to the sequences of the above indicated NCBI Reference Sequences of the PTPRC transcript, and also relates to the corresponding amino acid sequence for example as set forth in SEQ ID NO:137 or in SEQ ID NO:138, which correspond to the protein sequences defined in NCBI Protein Accession Reference Sequence NP_002829 encoding the PTPRC polypeptide and in NCBI Protein Accession Reference Sequence NP_563578.

The term "PTPRC" also comprises nucleotide sequences showing a high degree of homology to PTPRC, e.g., nucleic acid sequences being at least 75%, 80%, 85%, 90%, 91%, 92%, 93%, 94%, 95%, 96%, 97%, 98% or 99% identical to the sequence as set forth in SEQ ID NO:135 or in SEQ ID NO:136 or amino acid sequences being at least 75%, 80%, 85%, 90%, 91%, 92%, 93%, 94%, 95%, 96%, 97%, 98% or 99% identical to the sequence as set forth in SEQ ID NO:137 or in SEQ ID NO:138 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:137 or in SEQ ID NO:138 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:135 or in SEQ ID NO:136.

The term "RAP1GAP2" refers to the human RAP1 GTPase Activating Protein 2 gene (ENSG00000132359), for example, to the sequence as defined in NCBI Reference Sequence NM_015085 or in NCBI Reference Sequence NM_001100398 or in NCBI Reference Sequence NM_001330058, specifically, to the nucleotide sequence as set forth in SEQ ID NO:139 or in SEQ ID NO:140 or in SEQ ID NO:141, which correspond to the sequences of the above indicated NCBI Reference Sequences of the RAP1GAP2 transcript, and also relates to the corresponding amino acid sequence for example as set forth in SEQ ID NO:142 or in SEQ ID NO:143 or in SEQ ID NO:144, which correspond to the protein sequences defined in NCBI Protein Accession Reference Sequence NP_055900 and in NCBI Protein Accession Reference Sequence NP_001093868 and in NCBI Protein Accession Reference Sequence NP_001316987 encoding the RAP1GAP2 polypeptide.

The term "RAP1GAP2" also comprises nucleotide sequences showing a high degree of homology to RAP1GAP2, e.g., nucleic acid sequences being at least 75%, 80%, 85%, 90%, 91%, 92%, 93%, 94%, 95%, 96%, 97%, 98% or 99% identical to the sequence as set forth in SEQ ID NO:139 or in SEQ ID NO:140 or in SEQ ID NO:141 or amino acid sequences being at least 75%, 80%, 85%, 90%, 91%, 92%, 93%, 94%, 95%, 96%, 97%, 98% or 99% identical to the sequence as set forth in SEQ ID NO:142 or in SEQ ID NO:143 or in SEQ ID NO:144 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:142 or in SEQ ID NO:143 or in SEQ ID NO:144 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:139 or in SEQ ID NO:140 or in SEQ ID NO:141.

The term "SLC39A11" refers to the human Solute Carrier Family 39 Member 11 gene (Ensembl: ENSG00000133195), for example, to the sequence as defined in NCBI Reference Sequence NM_139177 or in NCBI Reference Sequence NM_001352692, specifically, to the nucleotide sequence as set forth in SEQ ID NO:145 or in SEQ ID NO:146, which correspond to the sequences of the above indicated NCBI Reference Sequences of the SLC39A11 transcript, and also relates to the corresponding amino acid sequence for example as set forth in SEQ ID NO:147 or in SEQ ID NO:148, which correspond to the protein sequences defined in NCBI Protein Accession Reference Sequence NP_631916 and in NCBI Protein Accession Reference Sequence NP_001339621 encoding the SLC39A11 polypeptide.

The term "SLC39A11" also comprises nucleotide sequences showing a high degree of homology to SLC39A11, e.g., nucleic acid sequences being at least 75%, 80%, 85%, 90%, 91%, 92%, 93%, 94%, 95%, 96%, 97%, 98% or 99% identical to the sequence as set forth in SEQ ID NO:145 or in SEQ ID NO:146 or amino acid sequences being at least 75%, 80%, 85%, 90%, 91%, 92%, 93%, 94%, 95%, 96%, 97%, 98% or 99% identical to the sequence as set forth in SEQ ID NO:147 or in SEQ ID NO:148 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 147 or in SEQ ID NO:148 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:145 or in SEQ ID NO: 146.

The term "TDRD1" refers to the human Tudor Domain Containing 1 gene (Ensembl: ENSG00000095627), for example, to the sequence as defined in NCBI Reference Sequence NM_198795, specifically, to the nucleotide sequence as set forth in SEQ ID NO: 149, which corresponds to the sequence of the above indicated NCBI Reference Sequence of the TDRD1 transcript, and also relates to the corresponding amino acid sequence for example as set forth in SEQ ID NO: 150, which corresponds to the protein sequence defined in NCBI Protein Accession Reference Sequence NP_942090 encoding the TDRD1 polypeptide.

The term "TDRD1" also comprises nucleotide sequences showing a high degree of homology to TDRD1, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 149 or amino acid sequences being at least 75%, 80%, 85%, 90%, 91%, 92%, 93%, 94%, 95%, 96%, 97%, 98% or 99% identical to the sequence as set forth in SEQ ID NO: 150 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 150 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 149.

The term "TLR8" refers to the Toll Like Receptor 8 gene (Ensembl: ENSG00000101916), for example, to the sequence as defined in NCBI Reference Sequence NM_138636 or in NCBI Reference Sequence NM_016610, specifically, to the nucleotide sequences as set forth in SEQ ID NO:151 or in SEQ ID NO:152, which correspond to the sequences of the above indicated NCBI Reference Sequences of the TLR8 transcript, and also relates to the corresponding amino acid sequences for example as set forth in SEQ ID NO:153 or in SEQ ID NO:154, which corresponds to the protein sequences defined in NCBI Protein Accession Reference Sequence NP_619542 and in NCBI Protein Accession Reference Sequence NP_057694 encoding the TLR8 polypeptide.

The term "TLR8" also comprises nucleotide sequences showing a high degree of homology to TLR8, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 151 or in SEQ ID NO: 152 or amino acid sequences being at least 75%, 80%, 85%, 90%, 91%, 92%, 93%, 94%, 95%, 96%, 97%, 98% or 99% identical to the sequence as set forth in SEQ ID NO:153 or in SEQ ID NO:154 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 153 or in SEQ ID NO: 154 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 151 or in SEQ ID NO: 152.

The term "VWA2" refers to the human Von Willebrand Factor A Domain Containing 2 gene (Ensembl: ENSG00000165816), for example, to the sequence as defined in NCBI Reference Sequence NM_001320804, specifically, to the nucleotide sequence as set forth in SEQ ID NO:155, which corresponds to the sequence of the above indicated NCBI Reference Sequence of the VWA2 transcript, and also relates to the corresponding amino acid sequence for example as set forth in SEQ ID NO: 156, which corresponds to the protein sequence defined in NCBI Protein Accession Reference Sequence NP_001307733 encoding the VWA2 polypeptide.

The term "VWA2" also comprises nucleotide sequences showing a high degree of homology to VWA2, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 155 or amino acid sequences being at least 75%, 80%, 85%, 90%, 91%, 92%, 93%, 94%, 95%, 96%, 97%, 98% or 99% identical to the sequence as set forth in SEQ ID NO: 156 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 156 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 155.

The term "ZAP70" refers to the Zeta Chain Of T-Cell Receptor Associated Protein Kinase 70 gene (Ensembl: ENSG00000115085), for example, to the sequence as defined in NCBI Reference Sequence NM_001079 or in NCBI Reference Sequence NM_207519, specifically, to the nucleotide sequences as set forth in SEQ ID NO: 157 or in SEQ ID NO: 158, which correspond to the sequences of the above indicated NCBI Reference Sequences of the ZAP70 transcript, and also relates to the corresponding amino acid sequence for example as set forth in SEQ ID NO: 159 or in SEQ ID NO: 160, which corresponds to the protein sequences defined in NCBI Protein Accession Reference Sequence NP_001070 and in NCBI Protein Accession Reference Sequence NP_997402 encoding the ZAP70 polypeptide.

The term "ZAP70" also comprises nucleotide sequences showing a high degree of homology to ZAP70, e.g., nucleic acid sequences being at least 75%, 80%, 85%, 90%, 91%, 92%, 93%, 94%, 95%, 96%, 97%, 98% or 99% identical to the sequence as set forth in SEQ ID NO:157 or in SEQ ID NO:158or amino acid sequences being at least 75%, 80%, 85%, 90%, 91%, 92%, 93%, 94%, 95%, 96%, 97%, 98% or 99% identical to the sequence as set forth in SEQ ID NO:159 or in SEQ ID NO:160 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 159 or in SEQ ID NO: 160 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:157 or in SEQ ID NO:158.

The term "ZBP1" refers to the Z-DNA Binding Protein 1 gene (Ensembl: ENSG00000124256), for example, to the sequence as defined in NCBI Reference Sequence NM_030776 or in NCBI Reference Sequence NM_001160418 or in NCBI Reference Sequence NM_001160419, specifically, to the nucleotide sequence as set forth in SEQ ID NO:161 or in SEQ ID NO:162 or in SEQ ID NO:163, which corresponds to the sequence of the above indicated NCBI Reference Sequence of the ZBP1 transcript, and also relates to the corresponding amino acid sequence for example as set forth in SEQ ID NO:164 or in SEQ ID NO:165 or in SEQ ID NO:166, which corresponds to the protein sequences defined in NCBI Protein Accession Reference Sequence NP_110403 and in NCBI Protein Accession Reference Sequence NP_001153890 and in NCBI Protein Accession Reference Sequence NP_001153891 encoding the ZBP1 polypeptide.

The term "ZBP1" also comprises nucleotide sequences showing a high degree of homology to ZBP1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:161 or in SEQ ID NO:162 or in SEQ ID NO:163 or amino acid sequences being at least 75%, 80%, 85%, 90%, 91%, 92%, 93%, 94%, 95%, 96%, 97%, 98% or 99% identical to the sequence as set forth in SEQ ID NO:164 or in SEQ ID NO:165 or in SEQ ID NO:166or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:164 or in SEQ ID NO:165 or in SEQ ID NO:166 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:161 or in SEQ ID NO:162 or in SEQ ID NO:163.

The term "biological sample" or "sample obtained from a subject" refers to any biological material obtained via suitable methods known to the person skilled in the art from a subject, e.g., a bladder or kidney cancer patient. The biological sample used may be collected in a clinically acceptable manner, e.g., in a way that nucleic acids (in particular RNA) or proteins are preserved.

The biological sample(s) may include body tissue and/or a fluid, such as, but not limited to, blood (or blood derived such as serum, plasma, or PBMC's (peripheral blood mononuclear cells)), sweat, saliva, urine, and a needle biopsy or resection biopsy. Furthermore, the biological sample may contain a cell extract derived from or a cell population including an epithelial cell, such as a cancerous epithelial cell or an epithelial cell derived from tissue suspected to be cancerous. The biological sample may contain a cell population derived from a glandular tissue, e.g., the sample may be derived from the bladder or kidney of a subject. Additionally, cells may be purified from obtained body tissues and fluids if necessary, and then used as the biological sample. In some realizations, the sample may be a tissue sample, a urine sample, a urine sediment sample, a blood sample, a saliva sample, a semen sample, a sample including circulating tumour cells, extracellular vesicles, a sample containing prostate secreted exosomes, or cell lines or cancer cell line.

In one particular realization, biopsy or resections samples may be obtained and/or used. Such samples may include cells or cell lysates.

It is also conceivable that the content of a biological sample is submitted to an enrichment step. For instance, a sample may be contacted with ligands specific for the cell membrane or organelles of certain cell types, e.g., bladder or kidney cells, functionalized for example with magnetic particles. The material concentrated by the magnetic particles may subsequently be used for detection and analysis steps as described herein above or below.

Furthermore, cells, e.g., tumour cells, may be enriched via filtration processes of fluid or liquid samples, e.g., blood, urine, etc. Such filtration processes may also be combined with enrichment steps based on ligand specific interactions as described herein above.

The term "bladder cancer" refers to a cancer of the tissues of the urinary bladder. Bladder cancer most often begins in the cells (urothelial cells) that line the inside of the bladder.

The term "kidney cancer" refers to a group of renal cancers that starts in the kidney. The main types of kidney cancer is renal cell cancer (RCC), responsible for approximately 90-95% of all cases. Renal cell carcinoma is a kidney cancer that originates in the lining of the proximal convoluted tubule, a part of the very small tubes in the kidney that transport primary urine. Renal cell carcinoma (RCC) comprises a heterogeneous group of tumors and can be further sub-classified into papillary RCC (pRCC) vs. clear cell RCC (ccRCC) with differences in survival outcomes.

The term "TNM" refers to a classification system, which is used to describe the characteristics of malignant tumors.

The term "T stage" refers to the extent and size of the tumor according to the TNM classification system. T stage can have the attributes T1 (small local tumor; typically < 2 cm in size); T2 (larger local tumor; typically 2-5 cm in size); T3 (larger locally advanced tumor; typically >5 cm in size; T4 (advanced/metastatic tumor).

The term "N stage" refers to the presence and extent of tumor positive lymph nodes according to the TNM classification system. N stage can have the attributes NO (no evidence of tumor positive lymph nodes); N1 (evidence of tumor positive lymph nodes; N2/N3 (more extended number of tumor positive lymph nodes); NX (no assessment of lymph node status possible).

The term "M stage" refers to the presence and extent of tumor metastases according to the TNM classification system. M stage can have the attributes M0 (no evidence of the presence of distant metastases); M1 (evidence of the presence of distant metastases).

The term "survival" refers to survival of a patient from his or her bladder or kidney cancer.

It is preferred that:

- the one or more immune defense response genes comprise three or more, preferably, six or more, more preferably, nine or more, most preferably, all of the immune defense genes, and/or
- the one or more T-Cell receptor signaling genes comprise three or more, preferably, six or more, more preferably, nine or more, most preferably, all of the T-Cell receptor signaling genes, and/or
- the one or more PDE4D7 correlated genes comprise three or more, preferably, six or more, most preferably, all of the PDE4D7 correlated genes.

Moreover is it preferred that in the method of the invention:

- the three or more immune defense response genes comprise six or more, preferably, nine or more, more preferably, all of the immune defense genes, or
- the three or more T-Cell receptor signaling genes comprise six or more, preferably, nine or more, more preferably, all of the T-Cell receptor signaling genes, or
- the three or more PDE4D7 correlated genes comprise six or more, preferably, nine or more, most preferably, all of the PDE4D7 correlated genes, or
- the three or more genes comprise at least two or more genes selected from each of the immune defense response genes, T-Cell receptor signaling genes and PDE4D7 correlated genes, preferably three or more genes selected from each of the immune defense response genes, T-Cell receptor signaling genes and PDE4D7 correlated genes, most preferably all of the genes selected from each of the immune defense response genes, T-Cell receptor signaling genes and PDE4D7 correlated genes.

It is preferred that the determining of the outcome comprises:

- combining the first gene expression profiles for two or more, for example, 2, 3, 4, 5, 6, 7, 8, 9, 10, 11, 12, 13 or all, of the immune defense response genes with a regression function that had been derived from a population of bladder or kidney cancer subjects, and/or
- combining the second gene expression profiles for two or more, for example, 2, 3, 4, 5, 6, 7, 8, 9, 10, 11, 12, 13, 14, 15, 16 or all, of the T-Cell receptor signaling genes with a regression function that had been derived from a population of bladder or kidney cancer subjects, and/or
- combining the third gene expression profiles for two or more, for example, 2, 3, 4, 5, 6, 7 or all, of the PDE4D7 correlated genes with a regression function that had been derived from a population of bladder or kidney cancer subjects.

Moreover is it preferred that the determining of the prediction of the outcome comprises:

- combining the gene expression profiles for two or more, for example, 2, 3, 4, 5, 6, 7, 8, 9, 10, 11, 12, 13 or all, of the immune defense response genes with a regression function that had been derived from a population of bladder or kidney cancer subjects, and/or
- combining the gene expression profiles for two or more, for example, 2, 3, 4, 5, 6, 7, 8, 9, 10, 11, 12, 13, 14, 15, 16 or all, of the T-Cell receptor signaling genes with a regression function that had been derived from a population of bladder or kidney cancer subjects, and/or
- combining the gene expression profiles for two or more, for example, 2, 3, 4, 5, 6, 7 or all, of the PDE4D7 correlated genes with a regression function that had been derived from a population of bladder or kidney cancer subjects.

Cox proportional-hazards regression allows analyzing the effect of several risk factors on time to a tested event like survival. Thereby, the risk factors maybe dichotomous or discrete variables like a risk score or a clinical stage but may also be a continuous variable like a biomarker measurement or gene expression values. The probability of the endpoint (e.g., death or disease recurrence) is called the hazard. Next to the information on whether or not the tested endpoint was reached by e.g. subject in a patient cohort (e.g., patient did die or not) also the time to the endpoint is considered in the regression analysis. The hazard is modeled as: $H(t) = H_0(t) \cdot \exp(w_1 \cdot V_1 + w_2 \cdot V_2 + w_3 \cdot V_3 + ...)$, where $V_i$, $V_2$, $V_3$ ... are predictor variables and $H_0(t)$ is the baseline hazard while $H(t)$ is the hazard at any time t. The hazard ratio (or the risk to reach the event) is represented by $Ln[H(t)/ H_0(t)] = w_1 \cdot V_1 + w_2 \cdot V_2 + w_3 \cdot V_3 + ...$, where the coefficients or weights $w_i$, $w_2$, $w_3$ ... are estimated by the Cox regression analysis and can be interpreted in a similar manner as for logistic regression analysis.

In one particular realization, the combination of the first gene expression profiles for the two or more, for example, 2, 3, 4, 5, 6, 7, 8, 9, 10, 11, 12, 13 or all, of the immune defense response genes with a regression function is determined as follows:

IDR_model:

$$(w_1 \cdot AIM2) + (w_2 \cdot APOBEC3A) + (w_3 \cdot CIAO1) + (w_4 \cdot DDX58) +$$

$$(w_5 \cdot DHX9) + (w_6 \cdot IFI16) + (w_7 \cdot IFIH1) + (w_8 \cdot IFIT1) + (w_9 \cdot IFIT3) \quad (1)$$

$$+ (w_{10} \cdot LRRFIP1) + (w_{11} \cdot MYD88) + (w_{12} \cdot OAS1) + (w_{13} \cdot TLR8) +$$

$$(w_{14} \cdot ZBP1)$$

where $w_i$ to $w_{14}$ are weights and AIM2, APOBEC3A, CIAO1, DDX58, DHX9, IFI16, IFIH1, IFIT1, IFIT3, LRRFIP1, MYD88, OAS1, TLR8, and ZBP1 are the expression levels of the immune defense response genes.

In one example for bladder cancer, $w_1$ may be about -0.1 to 0.9, such as -0.4645, $w_2$ may be about -0.4 to 0.6, such as 0.06517, $w_3$ may be about -0.7 to 0.3, such as -0.1792, $w_4$ may be about -1.5 to -0.3, such as -0.7515, $w_5$ may be about 0.1 to 1.1, such as 0.5795, $w_6$ may be about 0.1 to 1.1, such as 0.6215, $w_7$ may be about -0.4 to 0.6, such as 0.06787, $w_s$ may be about -0.4 to 0.6, such as 0.08374, $w_9$ may be about -0.3 to 0.7, such as 0.2078, $w_{10}$ may be about -0.1 to 0.9, such as 0.3887, $w_{11}$ may be about -0.8 to 0.2, such as -0.3025, $w_{12}$ may be about -1.0 to 0.0, such as -0.487, $w_{13}$ may be about -0.3 to 0.7, such as 0.1532, and $w_{14}$ may be about -0.5 to 0.5, such as -0.00899.

In one example for ccRCC kidney cancer, $w_1$ may be about -0.2 to 0.8, such as 0.2961, $w_2$ may be about -0.4 to 0.6, such as 0.0749, $w_3$ may be about -0.4 to 0.6, such as 0.05333, $w_4$ may be about -0.8 to 0.2, such as -0.2559, $w_5$ may be about -0.5 to 0.5, such as 0.005466, $w_6$ may be about -0.5 to 0.5, such as -0.01445, $w_7$ may be about -0.5 to 0.5, such as -0.02134, $w_8$ may be about -0.5 to 0.5, such as -0.00236, $w_9$ may be about -0.5 to 0.5, such as -0.06985, $w_{10}$ may be about -0.5 to 0.5, such as -0.04794, $w_{11}$ may be about -0.3 to 0.8, such as 0.2408, $w_{12}$ may be about -0.6 to 0.4, such as -0.1432, $w_{13}$ may be about -0.7 to 0.3, such as -0.2362, and $w_{14}$ may be about -0.3 to 0.8, such as 0.1874.

In one example for pRCC kidney cancer, $w_1$ may be about -0.3 to 0.7, such as 0.1518, $w_2$ may be about 0.9 to 1.9, such as 1.4194, $w_3$ may be about -0.3 to 0.7, such as 0.1616, $w_4$ may be about 0.1 to 1.1, such as 0.5588, $w_5$ may be about -1.5 to -0.5, such as -1.008, $w_6$ may be about 0.1 to 1.1, such as 0.594, $w_7$ may be about 0.8 to 1.8, such as 1.3409, $w_8$ may be about -0.8 to 0.2, such as -0.2564, $w_9$ may be about -1.0 to 0.0, such as -0.5356, $w_{10}$ may be about 0.4 to 1.4, such as 0.9293, $w_{11}$ may be about -0.4 to 0.6, such as 0.07879, $w_{12}$ may be about -1.0 to 0.0, such as -0.4654, $w_{13}$ may be about -1.6 to -0.6, such as -1.0803, and $w_{14}$ may be about -0.5 to 0.5, such as 0.00752.

In one particular realization, the combination of the second gene expression profiles for the two or more, for example, 2, 3, 4, 5, 6, 7, 8, 9, 10, 11, 12, 13, 14, 15, 16 or all, of the T-Cell receptor signaling genes with a regression function is determined as follows:

TCR_SIGNALING_model:

$$(w_{15} \cdot C2) + (w_{16} \cdot CD247) + (w_{17} \cdot CD28) + (w_{18} \cdot CD3E) + (w_{19} \cdot$$

$$CD3G) + (w_{20} \cdot CD4) + (w_{21} \cdot CSK) + (w_{22} \cdot EZR) + (w_{23} \cdot FYN) + \quad (2)$$

$$(w_{24} \cdot LAT) + (w_{25} \cdot LCK) + (w_{26} \cdot PAG1) + (w_{27} \cdot PDE4D) + (w_{28} \cdot$$

$$PRKACA) + (w_{29} \cdot PRKACB) + (w_{30} \cdot PTPRC) + (w_{31} \cdot ZAP70)$$

where $w_{15}$ to $w_{31}$ are weights and CD2, CD247, CD28, CD3E, CD3G, CD4, CSK, EZR, FYN, LAT, LCK, PAG1, PDE4D, PRKACA, PRKACB, PTPRC, and ZAP70 are the expression levels of the T-Cell receptor signaling genes.

In one example, for bladder cancer, $w_{15}$ may be about -0.2 to 0.8, such as 0.3242, $w_{16}$ may be about -0.5 to 0.5, such as -0.03061, $w_{17}$ may be about 0.0 to 1.0, such as 0.4636, $w_{18}$ may be about -0.8 to 0.2, such as -0.3372, $w_{19}$ may be about -0.6 to 0.4, such as -0.09887, $w_{20}$ may be about -0.3 to 0.7, such as 0.1825, $w_{21}$ may be about -0.7 to 0.3, such as -0.1961, $w_{22}$ may be about -0.5 to 0.5, such as -0.03954, $w_{23}$ may be about -0.4 to 0.6, such as 0.1259, $w_{24}$ may be about -0.6 to 0.4, such as -0.06249, $w_{25}$ may be about -0.6 to 0.4, such as -0.07393, $w_{26}$ may be about -1.2 to -0.2, such a7s -0.705, $w_{27}$ may be about -0.2 to 0.8, such as 0.3454, $w_{28}$ may be about 0.0 to 1.0, such as 0.4765, $w_{29}$ may be about -0.2 to 0.8, such as 0.2676, $w_{30}$ may be about 0.2 to 12, such as 0.6749, and $w_{31}$ may be about -1.4 to -0.4, such as -0.8557.

In one example, for ccRCC kidney cancer, $w_{15}$ may be about -0.6 to 0.4, such as -0.1186, $w_{16}$ may be about -0.8 to 0.2, such as -0.3247, $w_{17}$ may be about -0.6 to 0.4, such as -0.09282, $w_{18}$ may be about -0.6 to 0.4, such as -0.06851, $w_{19}$ may be about -1.3 to -0.3, such as -0.8094, $w_{20}$ may be about -0.8 to 0.2, such as -0.2527, $w_{21}$ may be about -0.4 to 0.6, such as 0.09508, $w_{22}$ may be about -0.7 to 0.3, such as -0.1908, $w_{23}$ may be about -0.5 to 0.5, such as -0.0408, $w_{24}$ may be about -0.1 to 0.9, such as 0.3905, $w_{25}$ may be about 0.3 to 1.3, such as 0.79, $w_{26}$ may be about -0.3 to 0.7, such as 0.169, $w_{27}$ may be about -0.5 to 0.5, such as -0.05041, $w_{28}$ may be about -0.3 to 0.7, such as 0.1539, $w_{29}$ may be about -0.6 to 0.3, such as -0.07244, $w_{30}$ may be about -0.1 to 0.9, such as 0.3935, and $w_{31}$ may be about -0.4 to 0.9, such as 0.0895.

In one example, for pRCC kidney cancer, $w_{15}$ may be about 0.9 to 1.9, such as 1.3546, $w_{16}$ may be about 3.2 to 4.2, such as 3.7462, $w_{17}$ may be about -0.6 to 0.4, such as -0.134, $w_{18}$ may be about -3.0 to -2.0, such as -2.5246, $w_{19}$ may be about -0.1 to 0.9, such as 0.3545, $w_{20}$ may be about -0.9 to 0.1, such as -0.4082, $w_{21}$ may be about -2.6 to -1.6, such as -2.1136, $w_{22}$ may be about -0.4 to 0.6, such as 0.06057, $w_{23}$ may be about -0.8 to 0.2, such as -0.2527, $w_{24}$ may be about -1.2 to -0.2, such as -0.7183, $w_{25}$ may be about -1.4 to -0.4, such as -0.9357, $w_{26}$ may be about -1.1 to -0.1, such as -0.6145, $w_{27}$ may be about -1.4 to -0.4, such as -0.9477, $w_{28}$ may be -1.5 to -0.5, such as -1.0039, $w_{29}$ may be about -1.0 to 0.0, such as -0.538, $w_{30}$ may be about 0.3 to 1.3, such as 0.7621, and $w_{31}$ may be about 0.0 to 1.0, such as 0.5318.

In one particular realization, the combination of the third gene expression profiles for the two or more, for example, 2, 3, 4, 5, 6, 7 or all, of the PDE4D7 correlated genes with a regression function is determined as follows:

$$\text{PDE4D7\_CORR\_model:}$$
$$(w_{32} \cdot \text{ABCC5}) + (w_{33} \cdot \text{CUX2}) + (w_{34} \cdot \text{KIAA1549}) + (w_{35} \cdot \text{PDE4D}) + (w_{36} \cdot \text{RAP1GAP2}) + (w_{37} \cdot \text{SLC39A11}) + (w_{38} \cdot \text{TDRD1}) + (w_{39} \cdot \text{VWA2}) \quad (3)$$

where $w_{32}$ to $w_{39}$ are weights and ABCC5, CUX2, KIAA1549, PDE4D, RAP1GAP2, SLC39A11, TDRD1, and VWA2 are the expression levels of the PDE4D7 correlated genes.

In one example, for bladder cancer, $w_{32}$ may be about -0.6 to 0.4, such as -0.112, $w_{33}$ may be about -0.8 to 0.2, such as -0.3117, $w_{34}$ may be about 0.0 to 1.0, such as -0.00355, $w_{35}$ may be about -0.2 to 0.8, such as 0.2887, $w_{36}$ may be about -0.6 to 0.4, such as -0.08836, $w_{37}$ may be about -0.7 to 0.3, such as -0.1632, $w_{38}$ may be about -0.3 to 0.7, such as 0.2415, and $w_{39}$ may be about -0.3 to 0.7, such as 0.209.

In one example, for ccRCC kidney cancer, $w_{32}$ may be about -0.3 to 0.8, such as 0.2267, $w_{33}$ may be about -0.7 to 0.3, such as 0.2039, $w_{34}$ may be about -0.6 to 0.4, such as -0.1044, $w_{35}$ may be about -0.8 to 0.2, such as -0.2691, $w_{36}$ may be about -0.8 to 0.2, such as -0.2746, $w_{37}$ may be about -0.4 to 0.6, such as 0.07656, $w_{38}$ may be about -0.5 to 0.5, such as -0.02863, and $w_{39}$ may be about -0.4 to 0.6, such as 0.111.

In one example, for pRCC kidney cancer, $w_{32}$ may be about -0.9 to 0.1, such as -0.3819, $w_{33}$ may be about -2.2 to -1.2, such as -1.7267, $w_{34}$ may be about -0.5 to 0.5, such as -0.03515, $w_{35}$ may be about -1.1 to -0.1, such as -0.5589, $w_{36}$ may be about -1.4 to -0.4, such as -0.8972, $w_{37}$ may be about -0.5 to 0.5, such as -0.04555, $w_{38}$ may be about -0.1 to 0.9, such as 0.4498, and $w_{39}$ may be about -0.8 to 0.2, such as -0.2879.

It is further preferred that the determining of the prediction of the outcome further comprises combining the combination of the first gene expression profiles, the combination of the second gene expression profiles, and the combination of the third gene expression profiles with a regression function that had been derived from a population of bladder or kidney cancer subjects.

Moreover is it preferred that the determining of the prediction of the outcome further comprises combining the combination of the gene expression profiles with a regression function that had been derived from a population of bladder or kidney cancer subjects.

In one particular realization, the prediction of the outcome is determined as follows:

$$\text{BCAI\_model (bladder cancer) or KCAI\_model (ccRCC kidney}$$

$$\text{cancer) or PKCAI\_model (pRCC kidney cancer):}$$

$$(w_{40} \cdot \text{IDR\_model}) + (w_{41} \cdot \text{TCR\_SIGNALING\_model}) + (w_{42} \cdot$$

$$\text{PDE4D7\_CORR\_model)} \tag{4}$$

where $w_{40}$ to $w_{42}$ are weights, IDR_model is the above-described regression model based on the expression profiles for the two or more, for example, 2, 3, 4, 6, 7, 8, 9, 10, 11, 12, 13 or all, of the immune defense response genes, TCR_SIGNALING_model is the above-described regression model based on the expression profiles for the two or more, for example, 2, 3, 4, 6, 7, 8, 9, 10, 11, 12, 13, 14, 15, 16 or all, of the T-Cell receptor signaling genes, and PDE4D7_CORR_model is the above-described regression model based on the expression profiles for the two or more, for example, 2, 3, 4, 6, 7 or all, of the PDE4D7 correlated genes.

In one example for bladder cancer, $w_{40}$ may be about 0.2 to 12, such as 0.7474, $w_{41}$ may be about 0.1 to 1.1, such as 0.6412, and $w_{42}$ may be about -0.2 to 0.8, such as 0.2693.

In one example for ccRCC kidney cancer, $w_{40}$ may be about -0.2 to 0.8, such as 0.3436, $w_{41}$ may be about 0.2 to 12, such as 0.6589, and $w_{42}$ may be about -0.2 to 0.8, such as 0.2746.

In one example for pRCC kidney cancer, $w_{40}$ may be about 0.3 to 1.2, such as 0.7855, $w_{41}$ may be about 0.1 to 1.1, such as 0.5792, and $w_{42}$ may be about 0.0 to 1.0, such as 0.4559.

The prediction of the outcome may also be classified or categorized into one of at least two risk groups, based on the value of the prediction of the outcome. For example, there may be two risk groups, or three risk groups, or four risk groups, or more than four predefined risk groups. Each risk group covers a respective range of (non-overlapping) values of the prediction of the outcome. For example, a risk group may indicate a probability of occurrence of a specific clinical event from 0 to <0.1 or from 0.1 to <0.25 or from 0.25 to <0.5 or from 0.5 to 1.0 or the like.

It is further preferred that the determining of the prediction of the outcome is further based on one or more clinical parameters obtained from the subject.

As mentioned above, various measures based on clinical parameters have been investigated. By further basing the prediction of the outcome on such clinical parameter(s), it can be possible to further improve the prediction.

It is preferred that the clinical parameters comprise one or more of: (i) T stage attribute (T1, T2, T3, or T4); (ii) N stage attribute (N0, N1, N2, or N3), and; (iii) M stage attribute (M0, M1). Additionally or alternatively, the clinical parameters comprise one or more other clinical parameters that is/are relevant for the diagnosis and/or prognosis of bladder or kidney cancer.

It is further preferred that the determining of the prediction of the outcome comprises combining one or more of: (i) the first gene expression profile(s) for the one or more immune defense response genes; (ii) the second gene expression profile(s) for the one or more T-Cell receptor signaling genes; (iii) the third gene expression profile(s) for the one or more PDE4D7 correlated genes, and; (iv) the combination of the first gene expression profiles, the combination of the second gene expression profiles, and the combination of the third gene expression profiles, and the one or more clinical parameters obtained from the subject with a regression function that had been derived from a population of bladder or kidney cancer subjects.

Moreover is it preferred that the determining of the prediction of the outcome comprises combining one or more of the gene expression profile(s) of: (i) the three or more immune defense response genes; (ii) the three or more T-Cell receptor signaling genes; (iii) the three or more PDE4D7 correlated genes, and; (iv) the combination of one or more immune defense response genes, one or more T-Cell receptor signaling genes and one or more PDE4D7 correlated genes, and the one or more clinical parameters obtained from the subject with a regression function that had been derived from a population of bladder or kidney cancer subjects.

In one particular realization, the prediction of the outcome is determined as follows:

$$\text{BCAI\&Clinical\_model (bladder cancer):}$$

$$(w_{40} \cdot \text{IDR\_model}) + (w_{41} \cdot \text{TCR\_SIGNALING\_model}) + (w_{42} \cdot$$

$$\text{PDE4D7\_CORR\_model)} + (w_{43} \cdot \text{N\_stage\_N1}) + (w_{44} \cdot \text{N\_stage\_N2}) +$$

$$(w_{45} \cdot \text{N\_stage\_N3}) \tag{5}$$

where $w_{40}$ to $w_{45}$ are weights, IDR_model is the above-described regression model based on the expression profiles for the two or more, for example, 2, 3, 4, 6, 7, 8, 9, 10, 11, 12, 13 or all, of the immune defense response genes, TCR_SIGNALING_model is the above-described regression model based on the expression profiles for the two or more, for example, 2, 3, 4, 6, 7, 8, 9, 10, 11, 12, 13, 14, 15, 16 or all, of the T-Cell receptor signaling genes, and PDE4D7_CORR_model is the above-described regression model based on the expression profiles for the two or more, for example, 2, 3, 4, 6, 7 or all, of the PDE4D7 correlated genes, and N_stage_N1 to N_stage_N3 are clinical N stage attributes according to the TNM classification of malignant tumors.

In one example, $w_{40}$ may be about 0.3 to 1.1, such as 0.809, $w_{41}$ may be about 0.1 to 1.1, such as 0.6018, $w_{42}$ may be about -0.1 to 0.9, such as 0.3501, $w_{43}$ may be about 0.3 to 1.3, such as 0.7607, $w_{44}$ may be about -0.2 to 0.8, such as 0.3335, and $w_{45}$ may be about 1.9 to 2.9, such as 2.4497.

In one particular realization, the prediction of the outcome is determined as follows:

$$\text{KCAI\&Clinical\_model (ccRCC kidney cancer):}$$
$$(w_{40} \cdot \text{IDR\_model}) + (w_{41} \cdot \text{TCR\_SIGNALING\_model}) + (w_{42} \cdot \text{PDE4D7\_CORR\_model}) + (w_{43} \cdot \text{T\_stage\_T2}) + (w_{44} \cdot \text{T\_stage\_T3}) + (w_{45} \cdot \text{T\_stage\_T4}) \tag{6}$$

where $w_{40}$ to $w_{45}$ are weights, IDR_model is the above-described regression model based on the expression profiles for the two or more, for example, 2, 3, 4, 6, 7, 8, 9, 10, 11, 12, 13 or all, of the immune defense response genes, TCR_SIGNALING_model is the above-described regression model based on the expression profiles for the two or more, for example, 2, 3, 4, 6, 7, 8, 9, 10, 11, 12, 13, 14, 15, 16 or all, of the T-Cell receptor signaling genes, and PDE4D7_CORR_model is the above-described regression model based on the expression profiles for the two or more, for example, 2, 3, 4, 6, 7 or all, of the PDE4D7 correlated genes, and T_stage_T2 to T_stage_T4 are clinical T stage attributes according to the TNM classification of malignant tumors.

In one example, $w_{40}$ may be about -0.2 to 0.8, such as 0.3117, $w_{41}$ may be about 0.1 to 1.1, such as 0.6033, $w_{42}$ may be about -0.3 to 0.7, such as 0.1932, $w_{43}$ may be about -0.3 to 0.8, such as 0.1509, $w_{44}$ may be about 0.2 to 12, such as 0.6923, and $w_{45}$ may be about 1.2 to 2.2, such as 1.6854.

It is preferred that the biological sample is obtained from the subject before the start of the therapy. The gene expression profile(s) may be determined in the form of mRNA or protein in tissue of prostate cancer. Alternatively, if the genes are present in a soluble form, the gene expression profile(s) may be determined in blood.

It is further preferred that the therapy is surgery, radiotherapy, cytotoxic chemotherapy (CTX), or immunotherapy.

It is preferred that the prediction of the therapy response is negative or positive for the effectiveness of the therapy, wherein a therapy is recommended based on the prediction and, if the prediction is negative, the recommended therapy comprises one or more of: (i) therapy provided earlier than is the standard; (ii) therapy with an increased effective dose; (iii) an adjuvant therapy, such as chemotherapy; and (iv) an alternative therapy, such as immunotherapy.

In a further aspect of the present invention, an apparatus for predicting an outcome of a bladder or kidney cancer subject is presented, comprising:

- an input adapted to receive data indicative of a first gene expression profile for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, said first gene expression profile(s) being determined in a biological sample obtained from the subject, and/or of a second gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, said second gene expression profile(s) being determined in a biological sample obtained from the subject, and/or of a third gene expression profile for each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, said third gene expression profile(s) being determined in a biological sample obtained from the subject,
- a processor adapted to determine the prediction of the outcome based on the first gene expression profile(s), or on the second gene expression profile(s), or on the third gene expression profile(s), or on the first, second, and third gene expression profile(s), and
- optionally, a providing unit adapted to provide the prediction to a medical caregiver or the subject.

In a further aspect of the present invention an apparatus for predicting an outcome of a bladder or kidney cancer subject is presented, comprising:

- an input adapted to receive data indicative of the gene expression profiles of three or more genes, wherein the three or more genes are selected from:

  - immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, or
  - T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, or
  - PDE4D7 correlated genes selected from the group consisting of: ABCC5, CUX2, KIAA1549, PDE4D, RAP1GAP2, SLC39A11, TDRD1, and VWA2, or
  - wherein said three or more genes comprise at least one or more gene(s) selected from each of the immune defense response genes, T-Cell receptor signaling genes and PDE4D7 correlated genes;

- wherein the gene expression profile(s) are being determined in a biological sample obtained from a bladder or kidney cancer subject
- a processor adapted to determine the prediction of outcome based on the gene expression profiles of the three or more genes,
- a computer program product according to claim 11, and
- optionally, a providing unit adapted to provide the prediction to a medical caregiver or the subject.

In a further aspect of the present invention, a computer program product is presented comprising instructions which, when the program is executed by a computer, cause the computer to carry out a method comprising:

- receiving data indicative of a first gene expression profile for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, said first gene expression profile(s) being determined in a biological sample obtained from a bladder or kidney cancer subject, and/or of a second gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, said second gene expression profile(s) being determined in a biological sample obtained from a bladder or kidney cancer subject, and/or of a third gene expression profile for each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, said third gene expression profile(s) being determined in a biological sample obtained from a bladder or kidney cancer subject,
- determining a prediction of an outcome of the subject based on the first gene expression profile(s), or on the second gene expression profile(s), or on the third gene expression profile(s), or on the first, second, and third gene expression profile(s), and
- optionally, providing the prediction to a medical caregiver or the subject.

In a further aspect of the present invention, a computer program product is presented comprising instructions which, when the program is executed by a computer, cause the computer to carry out a method comprising:

- receiving data indicative of the gene expression profiles of three or more genes, wherein the three or more genes are selected from:

  - immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, or
  - T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, or
  - PDE4D7 correlated genes selected from the group consisting of: ABCC5, CUX2, KIAA1549, PDE4D, RAP1GAP2, SLC39A11, TDRD1, and VWA2, or
  - wherein said three or more genes comprise at least one or more gene(s) selected from each of the immune defense response genes, T-Cell receptor signaling genes and PDE4D7 correlated genes, and

- wherein the gene expression profile(s) are being determined in a biological sample obtained from a bladder or kidney cancer subject

- determining the prediction of the outcome based on the gene expression profiles of the three or more genes, and
- optionally, providing the prediction to a medical caregiver or the subject.

In a further aspect of the present invention, a diagnostic kit is presented, comprising:

- at least one primer and/or probe for determining a first gene expression profile for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, in a biological sample obtained from a subject, and/or
- at least one primer and/or probe for determining a second gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, in a biological sample obtained from a subject, and/or
- at least one primer and/or probe for determining a gene expression profile for each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, in a biological sample obtained from a subject, and
- optionally, an apparatus as defined in claim 12 or a computer program product as defined in claim 11.

In a further aspect of the present invention a diagnostic kit is presented comprising:

- at least three primers and probes for determining the gene expression profiles of three or more genes, wherein the three or more genes are selected from:

  - immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, or
  - T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, or
  - PDE4D7 correlated genes selected from the group consisting of: ABCC5, CUX2, KIAA1549, PDE4D, RAP1GAP2, SLC39A11, TDRD1, and VWA2, or
  - wherein said three or more genes comprise at least one or more gene(s) selected from each of the immune defense response genes, T-Cell receptor signaling genes and PDE4D7 correlated genes, and

- wherein the gene expression profile(s) are being determined in a biological sample obtained from a bladder or kidney cancer subject
- optionally, an apparatus as defined in claim 12 or a computer program product as defined in claim 11.

In a further aspect of the present invention, a use of the kit as defined in claim 13 is presented.
It is preferred that the use as defined in claim 14 is in a method of predicting an outcome of a bladder or kidney cancer subject.
In a further aspect of the present invention, a method is presented, comprising:

- receiving one or more biological sample(s) obtained from a bladder or kidney cancer subject,
- using the kit as defined in claim 13 to determine a first gene expression profile for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, in a biological sample obtained from the subject, and/or
- using the kit as defined in claim 13 to determine a second gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, in a biological sample obtained from the subject, and/or
- using the kit as defined in claim 13 to determine a third gene expression profile for each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, in a biological sample obtained from the subject.

In a further aspect of the invention, a method is presented, comprising:

- receiving one or more biological sample(s) obtained from a bladder cancer subject or a kidney cancer subject,

- using the kit as defined in claim 13 to determine the gene expression profiles of three or more genes, wherein the three or more genes are selected from:

  - immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, or
  - T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, or
  - PDE4D7 correlated genes selected from the group consisting of: ABCC5, CUX2, KIAA1549, PDE4D, RAP1GAP2, SLC39A11, TDRD1, and VWA2 or
  - wherein said three or more genes comprise at least one or more gene(s) selected from each of the immune defense response genes, T-Cell receptor signaling genes and PDE4D7 correlated genes.

In a further aspect of the present invention, a use of a first gene expression profile for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or of a second gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, and/or of a third gene expression profile for each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, in a method of predicting an outcome of a bladder or kidney cancer subject is presented, comprising:

- determining the prediction of the outcome based on the first gene expression profile(s), or on the second gene expression profile(s), or on the third gene expression profile(s), or on the first, second, and third gene expression profile(s), and
- optionally, providing the prediction or the personalization or a therapy recommendation based on the prediction or the personalization to a medical caregiver or the subject.

In a further aspect of the present invention a use of a computer program product according to claim 11 or an apparatus according to claim 12 for using the gene expression profiles of three or more genes, wherein the three or more genes are selected from:

- immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, or
- T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, or
- PDE4D7 correlated genes selected from the group consisting of: ABCC5, CUX2, KIAA1549, PDE4D, RAP1GAP2, SLC39A11, TDRD1, and VWA2, or
- wherein said three or more genes comprise at least one or more gene(s) selected from each of the immune defense response genes, T-Cell receptor signaling genes and PDE4D7 correlated genes,

in a method of predicting an outcome of a bladder or kidney cancer subject is presented, comprising:

- determining the prediction of the outcome based on the gene expression profiles of the three or more genes, and
- optionally, providing the prediction to a medical caregiver or the subject. It shall be understood that the method of claim 1, the apparatus of claim 12, the computer program product of claim 11, the diagnostic kit of claim 13, the use of the diagnostic kit of claim 14, the method of claim 16, and the use of a computer program product according to claim 11 or an apparatus according to claim 12 for using the gene expression profile(s) of claim 17 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

In the following drawings:

Fig. 1 shows schematically and exemplarily a flowchart of an embodiment of a method of predicting an outcome of a bladder or kidney cancer subject,

Fig. 2 shows a Kaplan-Meier curve of the IDR_model in a 95 patient TCGA bladder cancer cohort (training set used to develop the IDR_model),

Fig. 3 shows a Kaplan-Meier curve of the IDR_model in a 94 patient TCGA bladder cancer cohort (testing set used to validate the IDR_model as developed on the 95 patient training set),

Fig. 4 shows a Kaplan-Meier curve of the TCR_SIGNALING_model in a 95 patient TCGA bladder cancer cohort (training set used to develop the TCR_SIGNALING_model),

Fig. 5 shows a Kaplan-Meier curve of the TCR_SIGNALING_model in a 94 patient TCGA bladder cancer cohort (testing set used to validate the TCR_SIGNALING_model as developed on the 95 patient training set),

Fig. 6 shows a Kaplan-Meier curve of the PDE4D7_CORR_model in a 95 patient TCGA bladder cancer cohort (training set used to develop the PDE4D7_CORR_model),

Fig. 7 shows a Kaplan-Meier curve of the PDE4D7_CORR_model in a 94 patient TCGA bladder cancer cohort (testing set used to validate the PDE4D7_CORR_model as developed on the 95 patient training set),

Fig. 8 shows a Kaplan-Meier curve of the BCAI_model in a 95 patient TCGA bladder cancer cohort (training set used to develop the BCAI_model),

Fig. 9 shows a Kaplan-Meier curve of the BCAI_model in a 94 patient TCGA bladder cancer cohort (testing set used to validate the BCAI_model as developed on the 95 patient training set),

Fig. 10 shows a Kaplan-Meier curve of the BCAI&Clinical_model in a 95 patient TCGA bladder cancer cohort (training set used to develop the BCAI&Clinical model),

Fig. 11 shows a Kaplan-Meier curve of the BCAI&Clinical_model in a 94 patient TCGA bladder cancer cohort (testing set used to validate the BCAI&Clinical_model as developed on the 95 patient training set),

Fig. 12 shows a Kaplan-Meier curve of the IDR_model in a 311 patient TCGA ccRCC kidney cancer cohort (training set used to develop the IDR model),

Fig. 13 shows a Kaplan-Meier curve of the IDR_model in a 100 patient TCGA ccRCC kidney cancer cohort (testing set used to validate the IDR_model as developed on the 311 patient training set),

Fig. 14 shows a Kaplan-Meier curve of the TCR_SIGNALING_model in a 311 patient TCGA ccRCC kidney cancer cohort (training set used to develop the TCR_SIGNALING model),

Fig. 15 shows a Kaplan-Meier curve of the TCR_SIGNALING_model in a 100 patient TCGA ccRCC kidney cancer cohort (testing set used to validate the TCR_SIGNALING_model as developed on the 311 patient training set),

Fig. 16 shows a Kaplan-Meier curve of the PDE4D7_CORR_model in a 311 patient TCGA ccRCC kidney cancer cohort (training set used to develop the PDE4D7_CORR_model),

Fig. 17 shows a Kaplan-Meier curve of the PDE4D7_CORR_model in a 100 patient TCGA ccRCC kidney cancer cohort (testing set used to validate the PDE4D7_CORR_model as developed on the 311 patient training set),

Fig. 18 shows a Kaplan-Meier curve of the KCAI_model in a 311 patient TCGA ccRCC kidney cancer cohort (training set used to develop the KCAI_model),

Fig. 19 shows a Kaplan-Meier curve of the KCAI_model in a 100 patient TCGA ccRCC kidney cancer cohort (testing set used to validate the KCAI_model as developed on the 311 patient training set),

Fig. 20 shows a Kaplan-Meier curve of the KCAI&Clinical_model in a 311 patient TCGA ccRCC kidney cancer cohort (training set used to develop the KCAI&Clinical model),

Fig. 21 shows a Kaplan-Meier curve of the KCAI&Clinical_model in a 100 patient TCGA ccRCC kidney cancer cohort (testing set used to validate the KCAI&Clinical_model as developed on the 311 patient training set),

Fig. 22 shows a Kaplan-Meier curve of the IDR_model in a 91 patient TCGA pRCC kidney cancer cohort (training set used to develop the IDR_model),

Fig. 23 shows a Kaplan-Meier curve of the TCR_SIGNALING_model in a 91 patient TCGA pRCC kidney cancer cohort (training set used to develop the TCR_SIGNALING model),

Fig. 24 shows a Kaplan-Meier curve of the PDE4D7_CORR_model in a 91 patient TCGA pRCC kidney cancer cohort (training set used to develop the PDE4D7_CORR_model), and

Fig. 25 shows a Kaplan-Meier curve of the PKCAI_model in a 91 patient TCGA pRCC kidney cancer cohort (training set used to develop the PKCAI_model).

Fig. 26 shows a Kaplan-Meier curve of the BCAI_3.1 model in a 189 patient TCGA bladder cancer cohort (total number of patients with non-metastasized disease (m0), comprising both the training set and validation set).

Fig. 27 shows a Kaplan-Meier curve of the BCAI_3.2 model in a 189 patient TCGA bladder cancer cohort (total number of patients with non-metastasized disease (m0), comprising both the training set and validation set).

Fig. 28 shows a Kaplan-Meier curve of the BCAI_3.3 model in a 189 patient TCGA bladder cancer cohort (total number of patients with non-metastasized disease (m0), comprising both the training set and validation set).

Fig. 29 shows a Kaplan-Meier curve of the BCAI_3.4 model in a 189 patient TCGA bladder cancer cohort (total number of patients with non-metastasized disease (m0), comprising both the training set and validation set).

Fig. 30 shows a Kaplan-Meier curve of the BCAI_3.5 model in a 189 patient TCGA bladder cancer cohort (total number of patients with non-metastasized disease (m0), comprising both the training set and validation set).

Fig. 31 shows a Kaplan-Meier curve of the BCAI_3.6 model in a 189 patient TCGA bladder cancer cohort (total number of patients with non-metastasized disease (m0), comprising both the training set and validation set).

Fig. 32 shows a Kaplan-Meier curve of the BCAI_4.1 model in a 189 patient TCGA bladder cancer cohort (total number of patients with non-metastasized disease (m0), comprising both the training set and validation set).

Fig. 33 shows a Kaplan-Meier curve of the BCAI_4.2 model in a 189 patient TCGA bladder cancer cohort (total number of patients with non-metastasized disease (m0), comprising both the training set and validation set).

Fig. 34 shows a Kaplan-Meier curve of the BCAI_4.3 model in a 189 patient TCGA bladder cancer cohort (total number of patients with non-metastasized disease (m0), comprising both the training set and validation set).

Fig. 35 shows a Kaplan-Meier curve of the BCAI_4.4 model in a 189 patient TCGA bladder cancer cohort (total number of patients with non-metastasized disease (m0), comprising both the training set and validation set).

Fig. 36 shows a Kaplan-Meier curve of the BCAI_4.5 model in a 189 patient TCGA bladder cancer cohort (total number of patients with non-metastasized disease (m0), comprising both the training set and validation set).

Fig. 37 shows a Kaplan-Meier curve of the BCAI_4.6 model in a 189 patient TCGA bladder cancer cohort (total number of patients with non-metastasized disease (m0), comprising both the training set and validation set).

Fig. 38 shows a Kaplan-Meier curve of the KCAI_3.1 model in a 411 patient TCGA ccRCC kidney cancer cohort (total number of patients with non-metastasized disease (m0), comprising both the training set and validation set).

Fig. 39 shows a Kaplan-Meier curve of the KCAI_3.2 model in a 411 patient TCGA ccRCC kidney cancer cohort (total number of patients with non-metastasized disease (m0), comprising both the training set and validation set).

Fig. 40 shows a Kaplan-Meier curve of the KCAI_3.3 model in a 411 patient TCGA ccRCC kidney cancer cohort (total number of patients with non-metastasized disease (m0), comprising both the training set and validation set).

Fig. 41 shows a Kaplan-Meier curve of the KCAI_3.4 model in a 411 patient TCGA ccRCC kidney cancer cohort (total number of patients with non-metastasized disease (m0), comprising both the training set and validation set).

Fig. 42 shows a Kaplan-Meier curve of the KCAI_3.5 model in a 411 patient TCGA ccRCC kidney cancer cohort (total number of patients with non-metastasized disease (m0), comprising both the training set and validation set).

Fig. 43 shows a Kaplan-Meier curve of the KCAI_3.6 model in a 411 patient TCGA ccRCC kidney cancer cohort (total number of patients with non-metastasized disease (m0), comprising both the training set and validation set).

Fig. 44 shows a Kaplan-Meier curve of the KCAI_4.1 model in a 411 patient TCGA ccRCC kidney cancer cohort (total number of patients with non-metastasized disease (m0), comprising both the training set and validation set).

Fig. 45 shows a Kaplan-Meier curve of the KCAI_4.2 model in a 411 patient TCGA ccRCC kidney cancer cohort (total number of patients with non-metastasized disease (m0), comprising both the training set and validation set).

Fig. 46 shows a Kaplan-Meier curve of the KCAI_4.3 model in a 411 patient TCGA ccRCC kidney cancer cohort (total number of patients with non-metastasized disease (m0), comprising both the training set and validation set).

Fig. 47 shows a Kaplan-Meier curve of the KCAI_4.4 model in a 411 patient TCGA ccRCC kidney cancer cohort (total number of patients with non-metastasized disease (m0), comprising both the training set and validation set).

Fig. 48 shows a Kaplan-Meier curve of the KCAI_4.5 model in a 411 patient TCGA ccRCC kidney cancer cohort (total number of patients with non-metastasized disease (m0), comprising both the training set and validation set).

Fig. 49 shows a Kaplan-Meier curve of the KCAI_4.6 model in a 411 patient TCGA ccRCC kidney cancer cohort (total number of patients with non-metastasized disease (m0), comprising both the training set and validation set).

Figure 50 shows a Kaplan-Meier curve of the IDR_3.1 model in a 189 patient TCGA bladder cancer cohort (total number of patients with non-metastasized disease (m0), comprising both the training set and validation set).

Figure 51 shows a Kaplan-Meier curve of the IDR_3.2 model in a 189 patient TCGA bladder cancer cohort (total number of patients with non-metastasized disease (m0), comprising both the training set and validation set).

Figure 52 shows a Kaplan-Meier curve of the IDR_3.3 model in a 189 patient TCGA bladder cancer cohort (total number of patients with non-metastasized disease (m0), comprising both the training set and validation set).

Figure 53 shows a Kaplan-Meier curve of the TCR_3.1 model in a 189 patient TCGA bladder cancer cohort (total number of patients with non-metastasized disease (m0), comprising both the training set and validation set).

Figure 54 shows a Kaplan-Meier curve of the TCR_3.2 model in a 189 patient TCGA bladder cancer cohort (total number of patients with non-metastasized disease (m0), comprising both the training set and validation set).

Figure 55 shows a Kaplan-Meier curve of the TCR_3.3 model in a 189 patient TCGA bladder cancer cohort (total number of patients with non-metastasized disease (m0), comprising both the training set and validation set).

Figure 56 shows a Kaplan-Meier curve of the PDE4D7_3.1 model in a 189 patient TCGA bladder cancer cohort (total number of patients with non-metastasized disease (m0), comprising both the training set and validation set).

Figure 57 shows a Kaplan-Meier curve of the PDE4D7_3.2 model in a 189 patient TCGA bladder cancer cohort (total number of patients with non-metastasized disease (m0), comprising both the training set and validation set).

Figure 58 shows a Kaplan-Meier curve of the PDE4D7_3.3 model in a 189 patient TCGA bladder cancer cohort (total number of patients with non-metastasized disease (m0), comprising both the training set and validation set).

Figure 59 shows a Kaplan-Meier curve of the IDR_3.1 model in a 411 patient TCGA ccRCC kidney cancer cohort (total number of patients with non-metastasized disease (m0), comprising both the training set and validation set).

Figure 60 shows a Kaplan-Meier curve of the IDR_3.2 model in a 411 patient TCGA ccRCC kidney cancer cohort (total number of patients with non-metastasized disease (m0), comprising both the training set and validation set).

Figure 61 shows a Kaplan-Meier curve of the IDR_3.3 model in a 411 patient TCGA ccRCC kidney cancer cohort (total number of patients with non-metastasized disease (m0), comprising both the training set and validation set).

Figure 62 shows a Kaplan-Meier curve of the TCR_3.1 model in a 411 patient TCGA ccRCC kidney cancer cohort (total number of patients with non-metastasized disease (m0), comprising both the training set and validation set).

Figure 63 shows a Kaplan-Meier curve of the TCR_3.2 model in a 411 patient TCGA ccRCC kidney cancer cohort (total number of patients with non-metastasized disease (m0), comprising both the training set and validation set).

Figure 64 shows a Kaplan-Meier curve of the TCR_3.3 model in a 411 patient TCGA ccRCC kidney cancer cohort (total number of patients with non-metastasized disease (m0), comprising both the training set and validation set).

Figure 65 shows a Kaplan-Meier curve of the PDE4D7_3.1 model in a 411 patient TCGA ccRCC kidney cancer cohort (total number of patients with non-metastasized disease (m0), comprising both the training set and validation set).

Figure 66 shows a Kaplan-Meier curve of the PDE4D7_3.2 model in a 411 patient TCGA ccRCC kidney cancer cohort (total number of patients with non-metastasized disease (m0), comprising both the training set and validation set).

Figure 67 shows a Kaplan-Meier curve of the PDE4D7_3.3 model in a 411 patient TCGA ccRCC kidney cancer cohort (total number of patients with non-metastasized disease (m0), comprising both the training set and validation set).


DETAILED DESCRIPTION OF EMBODIMENTS

**Overview Of Outcome Prediction**

Fig. 1 shows schematically and exemplarily a flowchart of an embodiment of a method of predicting an outcome of a bladder or kidney cancer subject.

The method begins at step S100.

At step S102, a biological sample is obtained from each of a first set of patients (subjects) diagnosed with bladder or kidney cancer. Preferably, monitoring bladder or kidney cancer has been performed for these bladder or kidney cancer patients over a period of time, such as at least one year, or at least two years, or about five years, after obtaining the biological sample.

At step S104, a first gene expression profile for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or a second gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, and/or a third gene expression profile for each of two or more, for example, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, is obtained for each of the biological samples obtained from the first set of patients, e.g., by performing RT-qPCR (real-time quantitative PCR) on RNA extracted from each biological sample. The exemplary gene expression profiles include an expression level (e.g., value) for each of the two or more genes which can be normalized using value(s) for each of a set of reference genes, such as B2M, HPRT1, POLR2A, and/or PUM1. In one realization, the gene expression level for each of the two or more genes of the first gene expression profiles, and/or the second gene expression profiles, and/or the third gene expression profiles is normalized with respect to one or more reference genes selected from the group consisting of ACTB, ALAS1, B2M, HPRT1, POLR2A, PUM1, RPLP0, TBP, TUBA1B, and/or YWHAZ, e.g., at least one, or at least two, or at least three, or, preferably, all of these reference genes.

At step S106, a regression function for assigning a prediction of the outcome is determined based on the first gene expression profiles for the two or more immune defense response genes, AIM2, APOBEC3A, CIAO1, DDX58, DHX9, IFI16, IFIH1, IFIT1, IFIT3, LRRFIP1, MYD88, OAS1, TLR8, and/or ZBP1, and/or the second gene expression profiles for the two or more T-Cell receptor signaling genes, CD2, CD247, CD28, CD3E, CD3G, CD4, CSK, EZR, FYN, LAT, LCK, PAG1, PDE4D, PRKACA, PRKACB, PTPRC, and/or ZAP70, and/or the third gene expression profiles for the two or more PDE4D7 correlated genes, ABCC5, CUX2, KIAA1549, PDE4D, RAP1GAP2, SLC39A11, TDRD1, and/or VWA2, obtained for at least some of the biological samples obtained for the first set of patients and respective results obtained from the monitoring. In one particular realization, the regression function is determined as specified in Eq. (4) above.

At step S108, a biological sample is obtained from a patient (subject or individual). The patient can be a new patient or one of the first set.

At step S110, a first gene expression profile for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or a second gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, and/or a third gene expression profile is obtained for each of the two or more, for example, 2, 3, 4, 5, 6, 7 or all, PDE4D7 correlated genes, e.g., by performing PCR on the biological sample. In one realization, the gene expression level for each of the two or more genes of the first gene expression profiles, and/or the second gene expression profiles, and/or the third gene expression profiles is normalized with respect to one or more reference genes selected from the group consisting of ACTB, ALAS1, B2M, HPRT1, POLR2A, PUM1, RPLP0, TBP, TUBA1B, and/or YWHAZ, e.g., at least one, or at least two, or at least three, or, preferably, all of these reference genes. This is substantially the same as in step S104.

At step S112, a prediction of the outcome based on the first, second, and third gene expression profiles is determined for the patient using the regression function. This will be described in more detail later in the description.

At S114, a therapy recommendation may be provided, e.g., to the patient or his or her guardian, to a doctor, or to another healthcare worker, based on the prediction or the personalization. To this end, the prediction or personalization may be categorized into one of a predefined set of risk groups, based on the value of the prediction or personalization. In one particular realization, the therapy may be radiotherapy and the prediction of the therapy response may be negative or positive for the effectiveness of the therapy. If the prediction is negative, the recommended therapy may comprise one or more of: (i) therapy provided earlier than is the standard; (ii) therapy with an increased effective dose; (iii) an adjuvant therapy, such as chemotherapy; and (iv) an alternative therapy, such as immunotherapy.

The method ends at S116.

In one embodiment, the gene expression profiles at steps S104 and S 110 are determined by detecting mRNA expression using two or more primers and/or probes and/or two or more sets thereof.

In one embodiment, steps S104 and S110 further comprise obtaining clinical parameters from the first set of patients and the patient, respectively. The clinical parameters may comprise one or more of: (i) T stage attribute (T1, T2, T3, or T4); (ii) N stage attribute (N0, N1, N2, or N3), and; (iii) M stage attribute (M0, M1). Additionally or alternatively, the clinical parameters comprise one or more other clinical parameters that is/are relevant for the diagnosis and/or prognosis of bladder or kidney cancer. The regression function for assigning the prediction of the outcome that is determined in step S106 is then further based on the one or more clinical parameters obtained from at least some of the first set of patients. In step S112, the prediction of the outcome is then further based on the one or more clinical parameters, e.g., the T stage attribute or the N stage attribute, obtained from the patient and is determined for the patient using the regression function. In one particular realization, the regression function is determined as specified in Eq. (5) or in Eq. (6) above.

Based on the significant correlation with survival outcome after therapy, we expect that the identified molecules will provide predictive value with regard to the effectiveness of the treatment of primary bladder or kidney cancers.

RESULTS

For each gene, the log2 expression value as provided in the download from the respective TCGA database (TCGA Bladder Urothelial Carcinoma, Firehose legacy, http://www.linkedomics.org, accessed March 6, 2020; Kidney Renal Clear Cell Carcinoma, TCGA, Firehose legacy, http://www.cbioportal.org/, accessed February 2, 2020; Kidney Renal Papillary Cell Carcinoma, TCGA, Firehose legacy, http://www.linkedomics.org, accessed February 17, 2020) was obtained.

The log2 expression values for each gene were transformed into z-scores by calculating:

$$\text{log2\_gene transformed z-score} = ((\text{log2\_gene}) - (\text{mean\_samples}))/(\text{stdev\_samples}) \tag{7}$$

where log2_gene is the log2 gene expression value per gene, mean_samples is the mathematical mean of the log2_gene values across all samples, and stdev_samples is the standard deviation of the log2_gene values across all samples.

This process distributes the transformed log2_gene values around the mean 0 with a standard deviation of 1.

For the multivariate analysis of the genes of interest we used the log2_gene transformed z-score value of each gene as input.

**Cox Regression Analysis**

We then set out to test whether the combination of the 14 immune defense response genes, the combination of the 17 T-Cell receptor signalling genes, the combination of the eight PDE4D7 correlated genes, and a combination thereof will exhibit a prognostic value for bladder and kidney cancer. With Cox regression we modelled the expression levels of the 14 immune defense response genes, of the 17 T-Cell receptor signalling genes, and of the eight PDE4D7 correlated genes, respectively, to overall survival in a TCGA cohort of 412 bladder cancer patients, a TCGA cohort of 533 ccRCC kidney cancer patients, and a TCGA cohort of 290 pRCC kidney cancer patients, respectively.

From the TCGA bladder cancer cohort, only samples with combined presence of clinical parameters, gene expression values, and survival information were included. From this subset, we only included samples from patients with non-metastasized disease (m0), which resulted in a total number of 189 patients. These 189 patients were randomly split into 4 groups. Cohort 1 (n=95) was used as a train cohort and consisted of groups 1+2. Cohort 2 (n=94) consisted of groups 3+4 and was used to validate the risk models as derived from the train cohort.

From the TCGA ccRCC kidney cancer cohort, only samples with combined presence of clinical parameters, gene expression values, and survival information were included. From this subset, we only included samples from patients with non-metastasized disease (m0), which resulted in a total number of 411 patients. These 411 patients were randomly split into 4 groups. Cohort 1 (n=311) was used as a train cohort and consisted of groups 1+2+3. Cohort 2 (n=100) consisted of group 4 and was used to validate the risk models as derived from the train cohort.

From the TCGA ccRCC kidney cancer cohort, only samples with combined presence of clinical parameters, gene expression values, and survival information were included. From this subset, we only included samples from patients with non-metastasized disease (m0), which resulted in a total number of 91 patients. This cohort was not further split into a train and test cohort, because both would be underpowered. Therefore we kept the full n=91 patients in the train cohort.

The Cox regression functions were derived as follows:

IDR model:

$$(w_1 \cdot AIM2) + (w_2 \cdot APOBEC3A) + (w_3 \cdot CIAO1) + (w_4 \cdot DDX58) +$$
$$(w_5 \cdot DHX9) + (w_6 \cdot IFI16) + (w_7 \cdot IFIH1) + (w_8 \cdot IFIT1) + (w_9 \cdot IFIT3)$$
$$+ (w_{10} \cdot LRRFIP1) + (w_{11} \cdot MYD88) + (w_{12} \cdot OAS1) + (w_{13} \cdot TLR8) +$$
$$(w_{14} \cdot ZBP1)$$

TCR SIGNALING model:

$$(w_{15} \cdot C2) + (w_{16} \cdot CD247) + (w_{17} \cdot CD28) + (w_{18} \cdot CD3E) + (w_{19} \cdot$$
$$CD3G) + (w_{20} \cdot CD4) + (w_{21} \cdot CSK) + (w_{22} \cdot EZR) + (w_{23} \cdot FYN) + (w_{24}$$
$$\cdot LAT) + (w_{25} \cdot LCK) + (w_{26} \cdot PAG1) + (w_{27} \cdot PDE4D) + (w_{28} \cdot$$
$$PRKACA) + (w_{29} \cdot PRKACB) + (w_{30} \cdot PTPRC) + (w_{31} \cdot ZAP70)$$

PDE4D7_CORR_model:

$$(w_{32} \cdot ABCC5) + (w_{33} \cdot CUX2) + (w_{34} \cdot KIAA1549) + (w_{35} \cdot PDE4D) +$$
$$(w_{36} \cdot RAP1GAP2) + (w_{37} \cdot SLC39A11) + (w_{38} \cdot TDRD1) + (w_{39} \cdot$$
$$VWA2)$$

The details for the weights $w_1$ to $w_{39}$ are shown in the following TABLES 1 to 3.

TABLE 1: Variables and weights for the three individual Cox regression models, i.e., the immune defense response model (IDR_model), the T-Cell receptor signaling model (TCR SIGNALING model), and the PDE4D7 correlation model (PDE4D7_CORR_model) for bladder cancer; NA - not available.

| Variable | | Weights | | |
|---|---|---|---|---|
| Model | | IDR_model | TCR_ SIGNALING_ model | PDE4D7_ CORR_ model |
| AIM2 | $w_1$ | -0.4645 | NA | NA |
| APOBEC3A | $w_2$ | 0.06517 | NA | NA |
| CIAO1 | $w_3$ | -0.1792 | NA | NA |
| DDX58 | $w_4$ | -0.7515 | NA | NA |
| DHX9 | $w_5$ | 0.5795 | NA | NA |
| IFI16 | $w_6$ | 0.6215 | NA | NA |
| IFIH1 | $w_7$ | 0.06787 | NA | NA |
| IFIT1 | $w_8$ | 0.08374 | NA | NA |
| IFIT3 | $w_9$ | 0.2078 | NA | NA |
| LRRFIP1 | $w_{10}$ | 0.3887 | NA | NA |
| MYD88 | $w_{11}$ | -0.3025 | NA | NA |
| OAS1 | $w_{12}$ | -0.487 | NA | NA |
| TLR8 | $w_{13}$ | 0.1532 | NA | NA |
| ZBP1 | $w_{14}$ | -0.00899 | NA | NA |
| CD2 | $w_{15}$ | NA | 0.3242 | NA |
| CD247 | $w_{16}$ | NA | 0.03061 | NA |
| CD28 | $w_{17}$ | NA | 0.4636 | NA |
| CD3E | $w_{18}$ | NA | -0.3372 | NA |
| CD3G | $w_{19}$ | NA | -0.09887 | NA |
| CD4 | $w_{20}$ | NA | 0.1825 | NA |
| CSK | $w_{21}$ | NA | -0.1961 | NA |
| EZR | $w_{22}$ | NA | -0.03954 | NA |
| FYN | $w_{23}$ | NA | 0.1259 | NA |
| LAT | $w_{24}$ | NA | -0.06429 | NA |
| LCK | $w_{25}$ | NA | -0.07393 | NA |
| PAG1 | $w_{26}$ | NA | -0.705 | NA |
| PDE4D | $w_{27}$ | NA | 0.3454 | NA |
| PRKACA | $w_{28}$ | NA | 0.4765 | NA |
| PRKACB | $w_{29}$ | NA | 0.2676 | NA |
| PTPRC | $w_{30}$ | NA | 0.6749 | NA |
| ZAP70 | $w_{31}$ | NA | -0.8557 | NA |

(continued)

| Variable | | Weights | | |
|---|---|---|---|---|
| Model | | IDR_model | TCR_ SIGNALING_ model | PDE4D7_ CORR_ model |
| ABCC5 | $w_{32}$ | NA | NA | -0.112 |
| CUX2 | $w_{33}$ | NA | NA | -0.3117 |
| KIAA1549 | $w_{34}$ | NA | NA | -0.00355 |
| PDE4D | $w_{35}$ | NA | NA | 0.2887 |
| RAP1GAP2 | $w_{36}$ | NA | NA | -0.08836 |
| SLC39A11 | $w_{37}$ | NA | NA | -0.1632 |
| TDRD1 | $w_{38}$ | NA | NA | 0.2415 |
| VWA2 | $W_{39}$ | NA | NA | 0.209 |

TABLE 2: Variables and weights for the three individual Cox regression models, i.e., the immune defense response model (IDR_model), the T-Cell receptor signaling model (TCR SIGNALING model), and the PDE4D7 correlation model (PDE4D7_CORR_model) for ccRCC kidney cancer; NA - not available.

| Variable | | Weights | | |
|---|---|---|---|---|
| Model | | IDR_model | TCR_ SIGNALING_ model | PDE4D7_ CORR_ model |
| AIM2 | $w_1$ | 0.2961 | NA | NA |
| APOBEC3A | $w_2$ | 0.0749 | NA | NA |
| CIAO1 | $w_3$ | 0.05333 | NA | NA |
| DDX58 | $w_4$ | -0.2559 | NA | NA |
| DHX9 | $w_5$ | 0.005466 | NA | NA |
| IFI16 | $w_6$ | -0.01445 | NA | NA |
| IFIH1 | $w_7$ | -0.02134 | NA | NA |
| IFIT1 | $w_8$ | -0.00236 | NA | NA |
| IFIT3 | $w_9$ | -0.06985 | NA | NA |
| LRRFIP1 | $w_{10}$ | -0.04794 | NA | NA |
| MYD88 | $w_{11}$ | 0.2408 | NA | NA |
| OAS1 | $w_{12}$ | -0.1432 | NA | NA |
| TLR8 | $w_{13}$ | -0.2362 | NA | NA |
| ZBP1 | $w_{14}$ | 0.1874 | NA | NA |
| CD2 | $w_{15}$ | NA | -0.1186 | NA |
| CD247 | $w_{16}$ | NA | -0.3247 | NA |
| CD28 | $w_{17}$ | NA | -0.09282 | NA |
| CD3E | $w_{18}$ | NA | -0.06851 | NA |
| CD3G | $w_{19}$ | NA | -0.8094 | NA |

(continued)

| Variable | | Weights | | |
|---|---|---|---|---|
| CD4 | $w_{20}$ | NA | -0.2527 | NA |
| CSK | $w_{21}$ | NA | 0.09508 | NA |
| EZR | $w_{22}$ | NA | -0.1908 | NA |
| FYN | $w_{23}$ | NA | -0.0408 | NA |
| LAT | $w_{24}$ | NA | 0.3905 | NA |
| LCK | $w_{25}$ | NA | 0.79 | NA |
| PAG1 | $w_{26}$ | NA | 0.169 | NA |
| PDE4D | $w_{27}$ | NA | -0.05041 | NA |
| PRKACA | $w_{28}$ | NA | 0.1539 | NA |
| PRKACB | $w_{29}$ | NA | -0.07244 | NA |
| PTPRC | $w_{30}$ | NA | 0.3935 | NA |
| ZAP70 | $w_{31}$ | NA | 0.0895 | NA |
| ABCC5 | $w_{32}$ | NA | NA | 0.2267 |
| CUX2 | $w_{33}$ | NA | NA | -0.2039 |
| KIAA1549 | $w_{34}$ | NA | NA | -0.1044 |
| PDE4D | $w_{35}$ | NA | NA | -0.2691 |
| RAP1GAP2 | $w_{36}$ | NA | NA | -0.2746 |
| SLC39A11 | $w_{37}$ | NA | NA | 0.07656 |
| TDRD1 | $w_{38}$ | NA | NA | -0.02863 |
| VWA2 | $W_{39}$ | NA | NA | 0.111 |

TABLE 3: Variables and weights for the three individual Cox regression models, i.e., the immune defense response model (IDR_model), the T-Cell receptor signaling model (TCR SIGNALING model), and the PDE4D7 correlation model (PDE4D7_CORR_model) for pRCC kidney cancer; NA - not available.

| Variable | | Weights | | |
|---|---|---|---|---|
| Model | | IDR_model | TCR_ SIGNALING_ model | PDE4D7_ CORR_ model |
| AIM2 | $w_1$ | 0.1518 | NA | NA |
| APOBEC3A | $w_2$ | 1.4194 | NA | NA |
| CIAO1 | $w_3$ | 0.1616 | NA | NA |
| DDX58 | $w_4$ | 0.5588 | NA | NA |
| DHX9 | $w_5$ | -1.008 | NA | NA |
| IFI16 | $w_6$ | 0.594 | NA | NA |
| IFIH1 | $w_7$ | 1.3409 | NA | NA |
| IFIT1 | $w_8$ | -0.2564 | NA | NA |

(continued)

| Variable | | Weights | | |
|---|---|---|---|---|
| Model | | IDR_model | TCR_ SIGNALING_ model | PDE4D7_ CORR_ model |
| IFIT3 | $w_9$ | -0.5356 | NA | NA |
| LRRFIP1 | $w_{10}$ | 0.9293 | NA | NA |
| MYD88 | $w_{11}$ | 0.07879 | NA | NA |
| OAS1 | $w_{12}$ | -0.4654 | NA | NA |
| TLR8 | $w_{13}$ | -1.0803 | NA | NA |
| ZBP1 | $w_{14}$ | 0.00752 | NA | NA |
| CD2 | $w_{15}$ | NA | 1.3546 | NA |
| CD247 | $w_{16}$ | NA | 3.7462 | NA |
| CD28 | $w_{17}$ | NA | -0.134 | NA |
| CD3E | $w_{18}$ | NA | -2.5246 | NA |
| CD3G | $w_{19}$ | NA | 0.3545 | NA |
| CD4 | $w_{20}$ | NA | -0.4082 | NA |
| CSK | $w_{21}$ | NA | -2.1136 | NA |
| EZR | $w_{22}$ | NA | 0.06057 | NA |
| FYN | $w_{23}$ | NA | -0.2527 | NA |
| LAT | $w_{24}$ | NA | -0.7183 | NA |
| LCK | $w_{25}$ | NA | -0.9357 | NA |
| PAG1 | $w_{26}$ | NA | -0.6145 | NA |
| PDE4D | $w_{27}$ | NA | -0.9477 | NA |
| PRKACA | $w_{28}$ | NA | 1.0039 | NA |
| PRKACB | $w_{29}$ | NA | -0.538 | NA |
| PTPRC | $w_{30}$ | NA | 0.7621 | NA |
| ZAP70 | $w_{31}$ | NA | 0.5318 | NA |
| ABCC5 | $w_{32}$ | NA | NA | -0.3819 |
| CUX2 | $w_{33}$ | NA | NA | -1.7267 |
| KIAA1549 | $w_{34}$ | NA | NA | -0.03515 |
| PDE4D | $w_{35}$ | NA | NA | -0.5589 |
| RAP1GAP2 | $w_{36}$ | NA | NA | -0.8972 |
| SLC39A11 | $w_{37}$ | NA | NA | -0.04555 |
| TDRD1 | $w_{38}$ | NA | NA | 0.4498 |
| VWA2 | $W_{39}$ | NA | NA | -0.2879 |

Based on the three individual Cox regression models (IDR model, TCR SIGNALING model, PDE4D7_CORR_model) we then again used Cox regression to model the combination thereof to overall survival with (BCAI&Clinical_model and KCAI&Clinical_model) or without (BCAI_model, KCAI_model, and PKCAI_model) the presence of the clinical variables (N stage attributes, T stage attributes) in the respective cohorts of bladder and kidney cancer patients. We tested the two models in Kaplan-Meier survival analysis.

The Cox regression functions were derived as follows:
BCAI_model (bladder cancer) or KCAI_model (ccRCC kidney cancer) or PKCAI_model (pRCC kidney cancer):

$$(w_{40} \cdot \text{IDR\_model}) + (w_{41} \cdot \text{TCR\_SIGNALING\_model}) + (w_{42} \cdot \text{PDE4D7\_CORR\_model})$$

BCAI&Clinical_model (bladder cancer):

$$(w_{40} \cdot \text{IDR\_model}) + (w_{41} \cdot \text{TCR\_SIGNALING\_model}) + (w_{42} \cdot \text{PDE4D7\_CORR\_model}) + (w_{43} \cdot \text{N\_stage\_N1}) + (w_{44} \cdot \text{N\_stage\_N2}) + (w_{45} \cdot \text{N\_stage\_N3})$$

KCAI&Clinical_model (ccRCC kidney cancer):

$$(w_{40} \cdot \text{IDR\_model}) + (w_{41} \cdot \text{TCR\_SIGNALING\_model}) + (w_{42} \cdot \text{PDE4D7\_CORR\_model}) + (w_{43} \cdot \text{T\_stage\_T2}) + (w_{44} \cdot \text{T\_stage\_T3}) + (w_{45} \cdot \text{T\_stage\_T4})$$

The details for the weights $w_{40}$ to $w_{48}$ are shown in the following TABLES 4 to 6.

TABLE 4: Variables and weights for two combination Cox regression models, i.e., bladder cancer AI model (BCAI_ model) and the bladder cancer & clinical model (BCAI&Clinical_model); NA - not available.

| Variable | Weights | | |
|---|---|---|---|
| Model | | BCAI_model | BCAI&Clinical_model |
| IDR_model | $w_{40}$ | 0.7484 | 0.809 |
| TCR_SIGNALING_model | $w_{41}$ | 0.6412 | 0.6018 |
| PDE47_CORR_model | $w_{42}$ | 0.2693 | 0.3501 |
| N_stage_N1 | $w_{43}$ | NA | 0.7607 |
| N_stage_N2 | $w_{44}$ | NA | 0.3335 |
| N_stage_N3 | $w_{45}$ | NA | 2.4497 |

TABLE 5: Variables and weights for two combination Cox regression models, i.e., ccRCC kidney cancer AI model (KCAI_model) and the ccRCC kidney cancer & clinical model (KCAI&Clinical_model); NA - not available.

| Variable | Weights | | |
|---|---|---|---|
| Model | | KCAI_model | KCAI&Clinical_model |
| IDR_model | $w_{40}$ | 0.3436 | 0.3117 |
| TCR_SIGNALING_model | $w_{41}$ | 0.6589 | 0.6033 |

(continued)

| Variable | Weights | | |
|---|---|---|---|
| Model | | KCAI_model | KCAI&Clinical_model |
| PDE47_CORR_model | $w_{42}$ | 0.2746 | 0.1932 |
| T_stage_T2 | $w_{43}$ | NA | 0.1509 |
| T_stage_T3 | $w_{44}$ | NA | 0.6923 |
| T_stage_T4 | $w_{45}$ | NA | 1.6854 |

TABLE 6: Variables and weights for combination Cox regression model, i.e., pRCC kidney cancer AI model (PKCAI_ model); NA - not available.

| Variable | Weights | |
|---|---|---|
| Model | | PKCAI_model |
| IDR_model | $w_{40}$ | 0.7855 |
| TCR_SIGNALING_model | $w_{41}$ | 0.5792 |
| PDE47_CORR_model | $w_{42}$ | 0.4559 |

## Kaplan-Meier Survival Analysis

For Kaplan-Meier survival curve analysis, the Cox functions of the risk models (IDR model, TCR SIGNALING model, PDE4D7_CORR_model, BCAI model, BCAI&Clinical_model, KCAI_model, KCAI&Clinical_model, and PKCAI_model) were categorized into two sub-cohorts based on a cut-off. The threshold for group separation into low and high risk was based on the risk to experience the clinical endpoint (outcome) as predicted by the respective Cox regression model

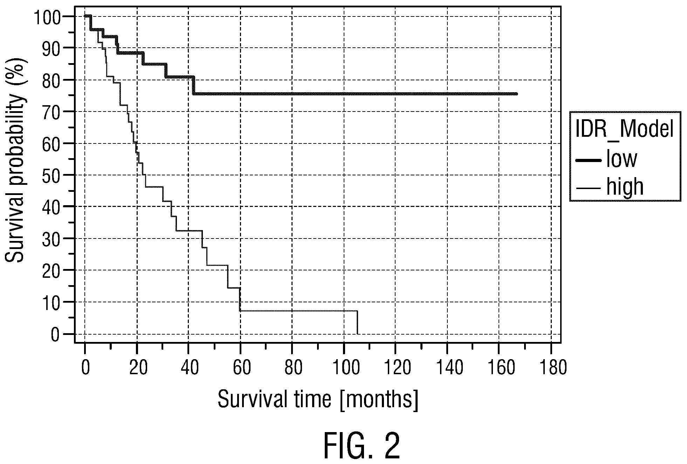

Fig. 2 shows a Kaplan-Meier curve of the IDR_model in a 95 patient TCGA bladder cancer cohort (training set used to develop the IDR_model). The clinical endpoint that was tested was the overall death (logrank $p<0.0001$; HR=4.7; 95% CI=2.4-9.2). The following supplementary lists indicate the number of patients at risk for the IDR_model classes analyzed (threshold=0), i.e., the patients at risk at any time interval +20 months are shown: Low risk: 46, 26, 15, 8, 4, 3, 2, 1, 1, 0; High risk: 49, 17, 7, 1, 1, 1, 0, 0, 0, 0.

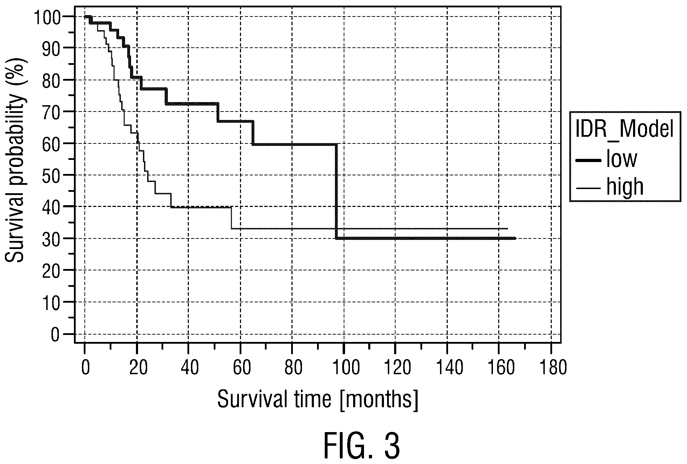

Fig. 3 shows a Kaplan-Meier curve of the IDR_model in a 94 patient TCGA bladder cancer cohort (testing set used to validate the IDR_model as developed on the 95 patient training set). The clinical endpoint that was tested was the overall death (logrank $p=0.01$; HR=2.3; 95% CI=1.2-4.5). The following supplementary lists indicate the number of patients at risk for the IDR_model classes analyzed (threshold=0), i.e., the patients at risk at any time interval +20 months are shown: Low risk: 48, 23, 14, 10, 3, 1, 1, 1, 1, 0; High risk: 46, 22, 9, 5, 3, 3, 2, 2, 1, 0.

Fig. 4 shows a Kaplan-Meier curve of the TCR_SIGNALING_model in a 95 patient TCGA bladder cancer cohort (training set used to develop the TCR_SIGNALING_model). The clinical endpoint that was tested was the overall death (logrank $p=0.002$; HR=2.8; 95% CI=1.5-5.5). The following supplementary lists indicate the number of patients at risk for the TCR_SIGNALING_model classes analyzed (threshold=0), i.e., the patients at risk at any time interval +20 months are shown: Low risk: 38, 19, 8, 4, 2, 2, 1, 1, 1, 0; High risk: 57, 24, 14, 5, 3, 2, 1, 0, 0, 0.

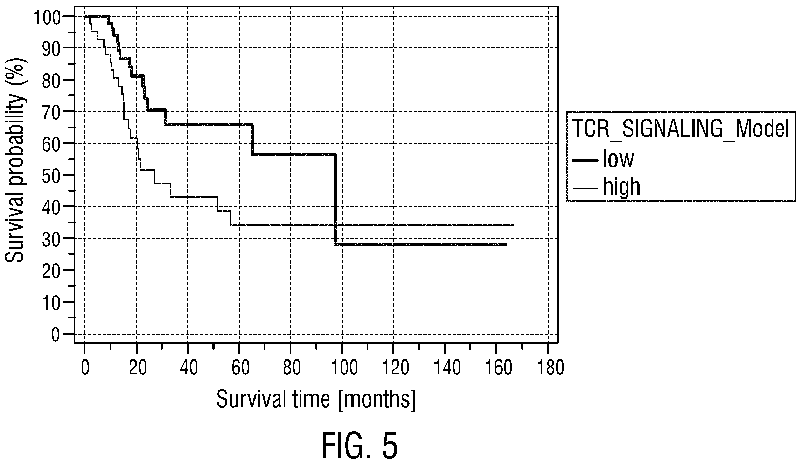

Fig. 5 shows a Kaplan-Meier curve of the TCR_SIGNALING_model in a 94 patient TCGA bladder cancer cohort (testing set used to validate the TCR_SIGNALING_model as developed on the 95 patient training set). The clinical endpoint that was tested was the overall death (logrank $p=0.03$; HR=2.0; 95% CI=1.0-4.0). The following supplementary lists indicate the number of patients at risk for the TCR_SIGNALING_model classes analyzed (threshold=0), i.e., the patients at risk at any time interval +20 months are shown: Low risk: 51, 26, 13, 8, 2, 1, 1, 1, 1, 0; High risk: 43, 19, 10, 7, 4, 3, 2, 2, 1, 0.

Fig. 6 shows a Kaplan-Meier curve of the PDE4D7_CORR_model in a 95 patient TCGA bladder cancer cohort (training set used to develop the PDE4D7_CORR_model). The clinical endpoint that was tested was the overall death (logrank $p=0.0007$; HR=4.5; 95% CI=1.9-10.7). The following supplementary lists indicate the number of patients at risk for the PDE4D7_CORR_model classes analyzed (threshold=0.3), i.e., the patients at risk at any time interval +20 months are shown: Low risk: 71, 38, 19, 8, 4, 4, 2, 1, 1, 0; High risk: 24, 5, 3, 1, 1, 0, 0, 0, 0, 0.

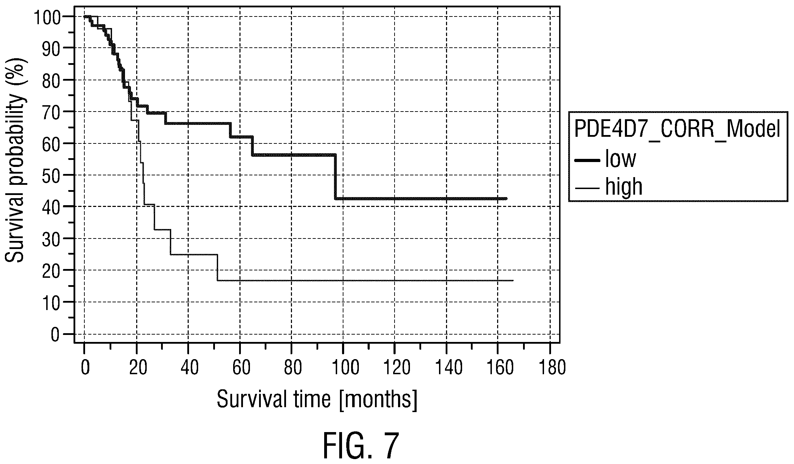

Fig. 7 shows a Kaplan-Meier curve of the PDE4D7_CORR_model in a 94 patient TCGA bladder cancer cohort (testing set used to validate the PDE4D7_CORR_model as developed on the 95 patient training set). The clinical endpoint that was tested was the overall death (logrank p=0.026; HR=2.4; 95% CI=1.1-5.2). The following supplementary lists indicate the number of patients at risk for the PDE4D7_CORR_model classes analyzed (threshold=0.3), i.e., the patients at risk at any time interval +20 months are shown: Low risk: 68, 35, 20, 13, 5, 3, 2, 2, 1, 0; High risk: 26, 10, 3, 2, 1, 1, 1, 1, 1, 0.

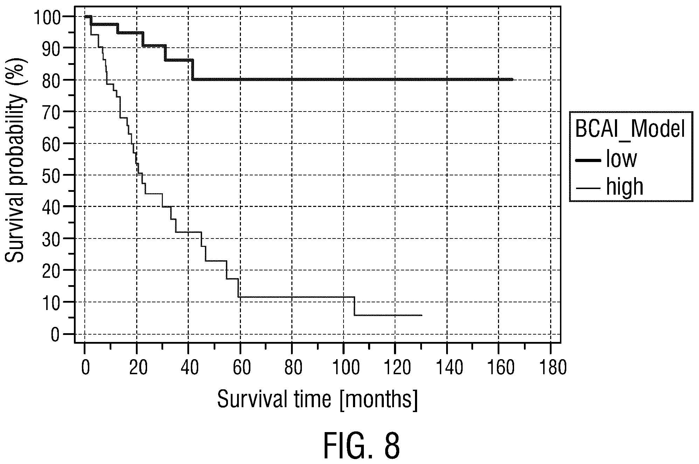

Fig. 8 shows a Kaplan-Meier curve of the BCAI_model in a 95 patient TCGA bladder cancer cohort (training set used to develop the BCAI_model). The clinical endpoint that was tested was the overall death (logrank p<0.0001; HR=5.5; 95% CI=2.9-10.7). The following supplementary lists indicate the number of patients at risk for the BCAI_model classes analyzed (threshold=0), i.e., the patients at risk at any time interval +20 months are shown: Low risk: 42, 26, 14, 7, 3, 2, 1, 1, 1, 0; High risk: 53, 17, 8, 2, 2, 2, 1, 0, 0, 0.

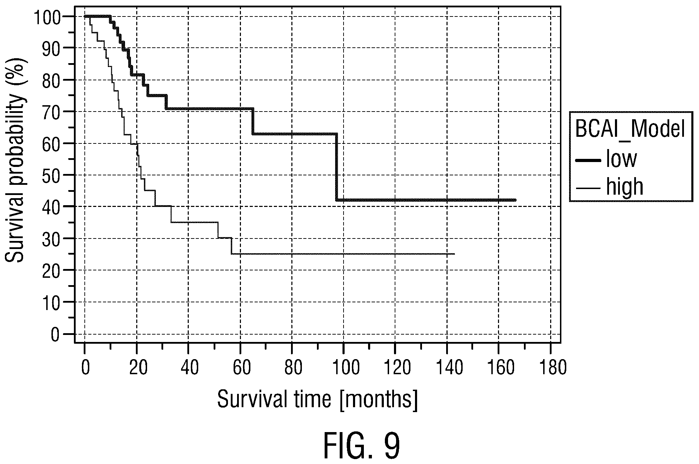

Fig. 9 shows a Kaplan-Meier curve of the BCAI_model in a 94 patient TCGA bladder cancer cohort (testing set used to validate the BCAI_model as developed on the 95 patient training set). The clinical endpoint that was tested was the overall death (logrank p=0.0007; HR=3.2; 95% CI=1.6-6.4). The following supplementary lists indicate the number of patients at risk for the BCAI_model classes analyzed (threshold=0), i.e., the patients at risk at any time interval +20 months are shown: Low risk: 55, 28, 16, 11, 3, 2, 2, 2, 2, 0; High risk: 39, 17, 7, 4, 3, 2, 1, 1, 0, 0.

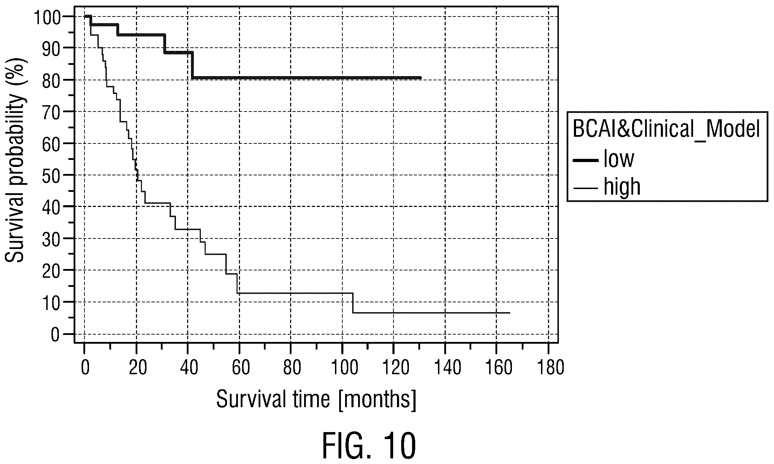

Fig. 10 shows a Kaplan-Meier curve of the BCAI&Clinical_model in a 95 patient TCGA bladder cancer cohort (training set used to develop the BCAI&Clinical_model). The clinical endpoint that was tested was the overall death (logrank p<0.0001; HR=5.1; 95% CI=2.6-10.0). The following supplementary lists indicate the number of patients at risk for the BCAI&Clinical_model classes analyzed (threshold=0), i.e., the patients at risk at any time interval +20 months are shown: Low risk: 37, 22, 11, 6, 3, 2, 1, 0, 0, 0; High risk: 51, 15, 8, 2, 2, 2, 1, 1, 1, 0.

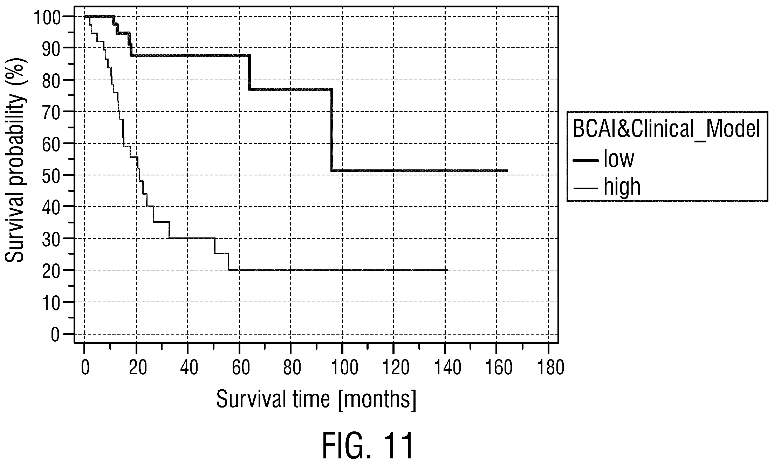

Fig. 11 shows a Kaplan-Meier curve of the BCAI&Clinical_model in a 94 patient TCGA bladder cancer cohort (testing set used to validate the BCAI&Clinical_model as developed on the 95 patient training set). The clinical endpoint that was tested was the overall death (logrank p<0.0001; HR=5.4; 95% CI=2.6-11.2). The following supplementary lists indicate the number of patients at risk for the BCAI&Clinical_model classes analyzed (threshold=0), i.e., the patients at risk at any time interval +20 months are shown: Low risk: 44, 22, 15, 10, 3, 2, 2, 2, 2, 0; High risk: 38, 15, 6, 4, 3, 2, 1, 1, 0, 0.

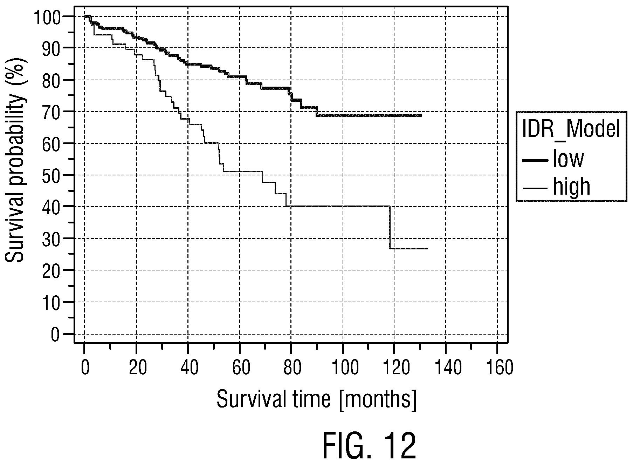

Fig. 12 shows a Kaplan-Meier curve of the IDR_model in a 311 patient TCGA ccRCC kidney cancer cohort (training set used to develop the IDR_model). The clinical endpoint that was tested was the overall death (logrank p<0.0001; HR=3.6; 95% CI=2.1-6.3). The following supplementary lists indicate the number of patients at risk for the IDR_model classes analyzed (threshold=0.3), i.e., the patients at risk at any time interval +20 months are shown: Low risk: 242, 199, 141, 81, 39, 18, 6, 0; High risk: 69, 54, 39, 18, 9, 6, 2, 0.

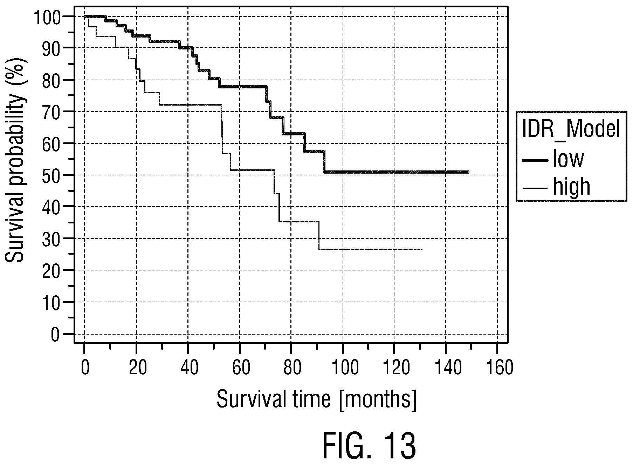

Fig. 13 shows a Kaplan-Meier curve of the IDR_model in a 100 patient TCGA ccRCC kidney cancer cohort (testing set used to validate the IDR_model as developed on the 311 patient training set). The clinical endpoint that was tested was the overall death (logrank p=0.02; HR=2.5; 95% CI=1.1-5.3). The following supplementary lists indicate the number of patients at risk for the IDR_model classes analyzed (threshold=0.3), i.e., the patients at risk at any time interval +20 months are shown: Low risk: 68, 57, 38, 24, 12, 6, 3, 1, 0; High risk: 32, 23, 16, 10, 4, 3, 1, 0, 0.

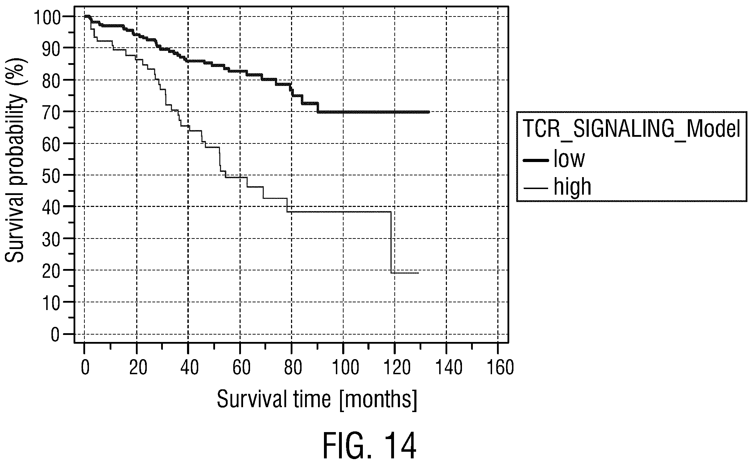

Fig. 14 shows a Kaplan-Meier curve of the TCR_SIGNALING_model in a 311 patient TCGA ccRCC kidney cancer cohort (training set used to develop the TCR_SIGNALING_model). The clinical endpoint that was tested was the overall death logrank p<0.0001; HR=4.5; 95% CI=2.6-7.8). The following supplementary lists indicate the number of patients at risk for the TCR_SIGNALING_model classes analyzed (threshold=0.4), i.e., the patients at risk at any time interval +20 months are shown: Low risk: 235, 196, 140, 81, 41, 20, 7, 0; High risk: 76, 57, 40, 18, 7, 4, 1, 0.

Fig. 15 shows a Kaplan-Meier curve of the TCR_SIGNALING_model in a 100 patient TCGA ccRCC kidney cancer cohort (testing set used to validate the TCR_SIGNALING_model as developed on the 311 patient training set). The clinical endpoint that was tested was the overall death (logrank p=0.06; HR=2.5; 95% CI=0.96-6.4). The following supplementary lists indicate the number of patients at risk for the TCR_SIGNALING_model classes analyzed (threshold=0.4), i.e., the patients at risk at any time interval +20 months are shown: Low risk: 79, 63, 45, 29, 14, 8, 3, 1, 0; High risk: 21, 17, 9,5,2, 1, 1,0,0.

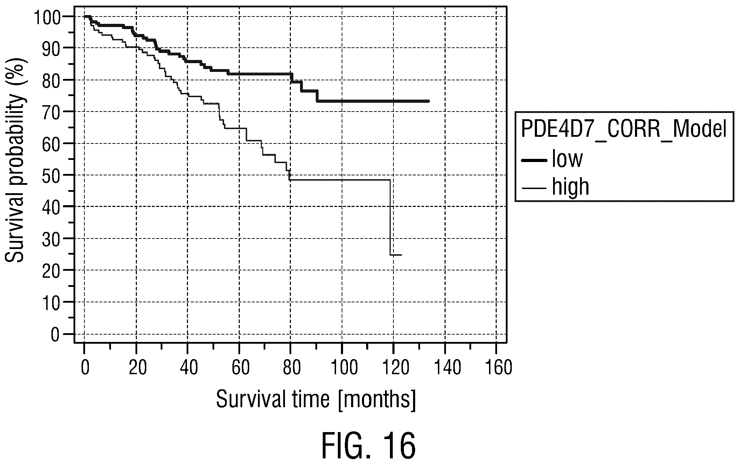

Fig. 16 shows a Kaplan-Meier curve of the PDE4D7_CORR_model in a 311 patient TCGA ccRCC kidney cancer cohort (training set used to develop the PDE4D7_CORR_model). The clinical endpoint that was tested was the overall death (logrank p=0.0003; HR=2.3; 95% CI=1.5-3.7). The following supplementary lists indicate the number of patients at risk for the PDE4D7_CORR_model classes analyzed (threshold=0), i.e., the patients at risk at any time interval +20 months are shown: Low risk: 174, 142, 102, 62, 33, 17, 7, 0; High risk: 137, 111, 78, 37, 15, 7, 1, 0.

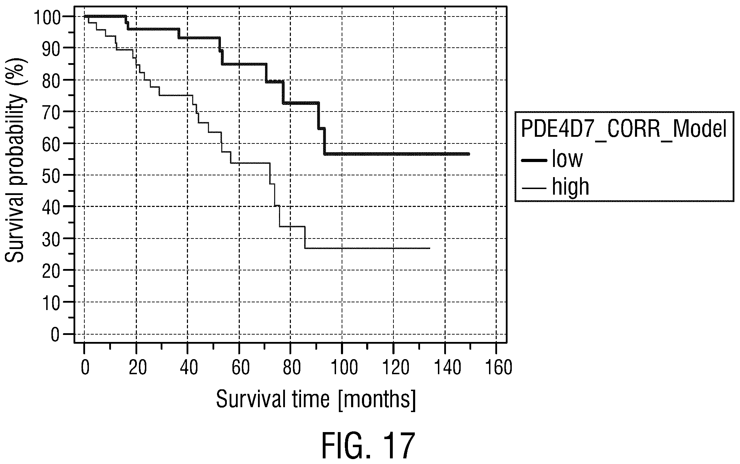

Fig. 17 shows a Kaplan-Meier curve of the PDE4D7_CORR_model in a 100 patient TCGA ccRCC kidney cancer cohort (testing set used to validate the PDE4D7_CORR_model as developed on the 311 patient training set). The clinical endpoint that was tested was the overall death (logrank p=0.001; HR=3.2; 95% CI=1.6-6.5). The following supplementary lists indicate the number of patients at risk for the PDE4D7_CORR_model classes analyzed (threshold=0), i.e., the patients at risk at any time interval +20 months are shown: Low risk: 51, 44, 28, 19, 11, 5, 3, 1, 0; High risk: 49, 36, 26, 15, 5, 4, 1, 0, 0.

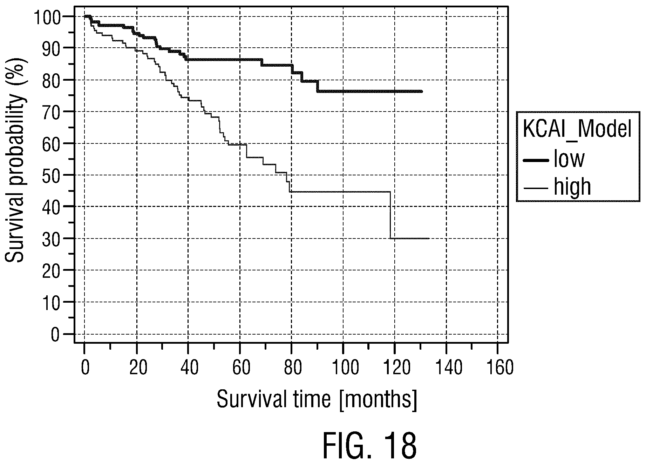

Fig. 18 shows a Kaplan-Meier curve of the KCAI_model in a 311 patient TCGA ccRCC kidney cancer cohort (training set used to develop the KCAI_model). The clinical endpoint that was tested was the overall death (logrank p<0.0001; HR=3.0; 95% CI=1.9-4.8). The following supplementary lists indicate the number of patients at risk for the KCAI_model classes analyzed (threshold=0), i.e., the patients at risk at any time interval +20 months are shown: Low risk: 179, 145, 98, 64, 35, 17, 6, 0; High risk: 132, 108, 82, 35, 13, 7, 2, 0.

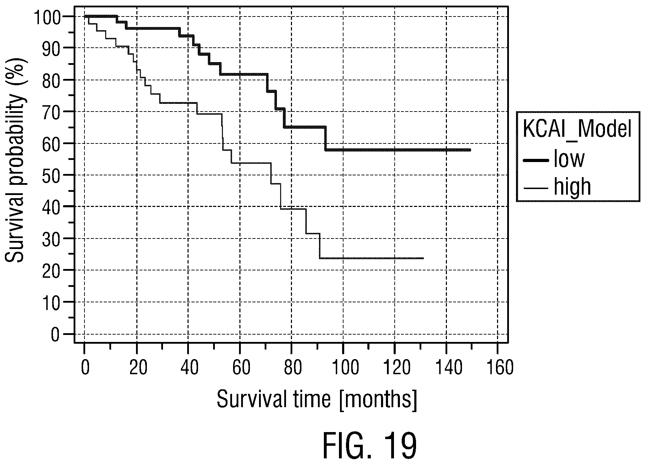

Fig. 19 shows a Kaplan-Meier curve of the KCAI_model in a 100 patient TCGA ccRCC kidney cancer cohort (testing set used to validate the KCAI_model as developed on the 311 patient training set). The clinical endpoint that was tested was the overall death (logrank p=0.003; HR=3.0; 95% CI=1.4-6.1). The following supplementary lists indicate the number of patients at risk for the KCAI_model classes analyzed (threshold=0), i.e., the patients at risk at any time interval +20 months are shown: Low risk: 56, 47, 33, 20, 11, 6, 3, 1, 0; High risk: 44, 33, 21, 14, 5, 3, 1, 0, 0.

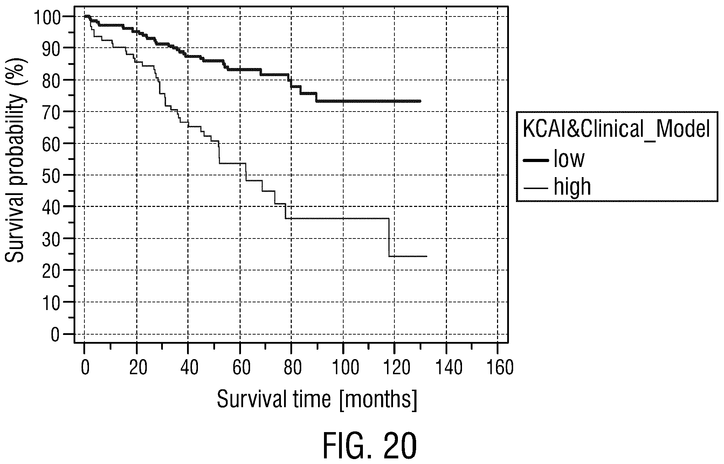

Fig. 20 shows a Kaplan-Meier curve of the KCAI&Clinical_model in a 311 patient TCGA ccRCC kidney cancer cohort (training set used to develop the KCAI&Clinical_model). The clinical endpoint that was tested was the overall death (logrank p<0.0001; HR=4.5; 95% CI=2.7-7.6). The following supplementary lists indicate the number of patients at risk for the KCAI&Clinical_model classes analyzed (threshold=0.5), i.e., the patients at risk at any time interval +20 months are shown: Low risk: 218, 180, 131, 77, 42, 21, 6, 0; High risk: 93, 73, 49, 22, 6, 3, 2, 0.

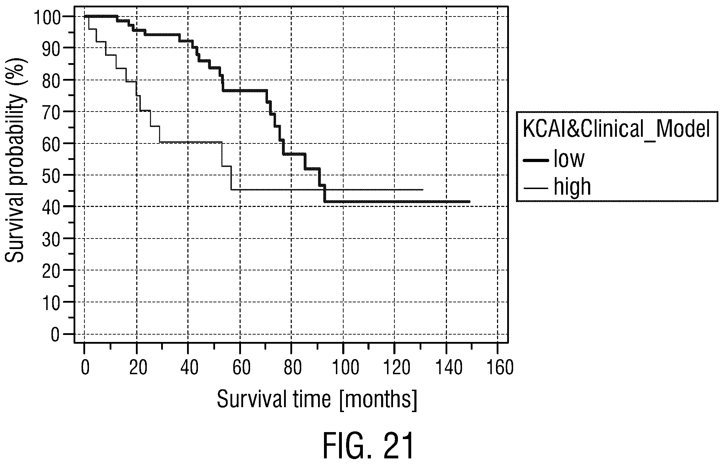

Fig. 21 shows a Kaplan-Meier curve of the KCAI&Clinical_model in a 100 patient TCGA ccRCC kidney cancer cohort (testing set used to validate the KCAI&Clinical_model as developed on the 311 patient training set). The clinical endpoint that was tested was the overall death (logrank p=0.03; HR=2.7; 95% CI=1.1-6.5). The following supplementary lists indicate the number of patients at risk for the KCAI&Clinical_model classes analyzed (threshold=0.5), i.e., the patients at risk at any time interval +20 months are shown: Low risk: 75, 64, 44, 28, 13, 6, 2, 1, 0; High risk: 25, 16, 10, 6, 3, 3, 2, 0, 0.

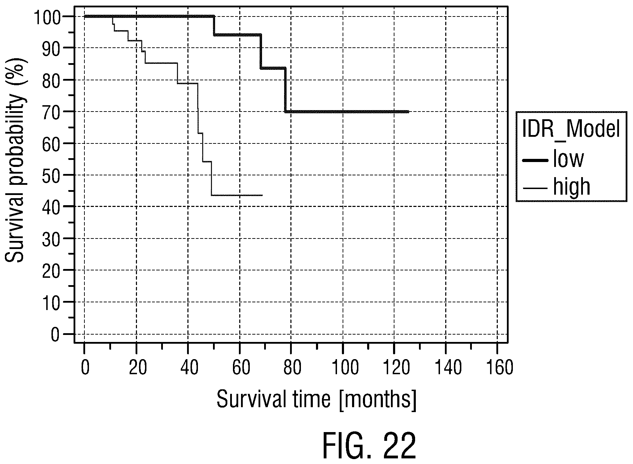

Fig. 22 shows a Kaplan-Meier curve of the IDR_model in a 91 patient TCGA pRCC kidney cancer cohort (training set used to develop the IDR_model). The clinical endpoint that was tested was the overall death (logrank p=0.05; HR=2.9; 95% CI=1.0-8.8). The following supplementary lists indicate the number of patients at risk for the IDR_model classes analyzed (threshold=0), i.e., the patients at risk at any time interval +20 months are shown: Low risk: 41, 26, 14, 6, 2, 1, 1, 0; High risk: 50, 36, 20, 8, 3, 0, 0, 0.

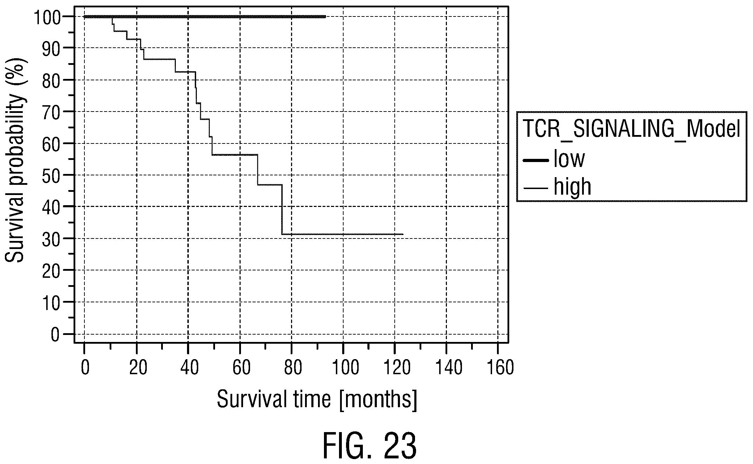

Fig. 23 shows a Kaplan-Meier curve of the TCR_SIGNALING_model in a 91 patient TCGA pRCC kidney cancer cohort (training set used to develop the TCR_SIGNALING_model). The clinical endpoint that was tested was the overall death (logrank p=0.0008; HR=NA; 95% CI=NA). The following supplementary lists indicate the number of patients at risk for the TCR_SIGNALING_model classes analyzed (threshold=0), i.e., the patients at risk at any time interval +20 months are shown: Low risk: 44, 31, 16, 6, 3, 0, 0, 0; High risk: 47, 31, 18, 8, 2, 1, 1, 0.

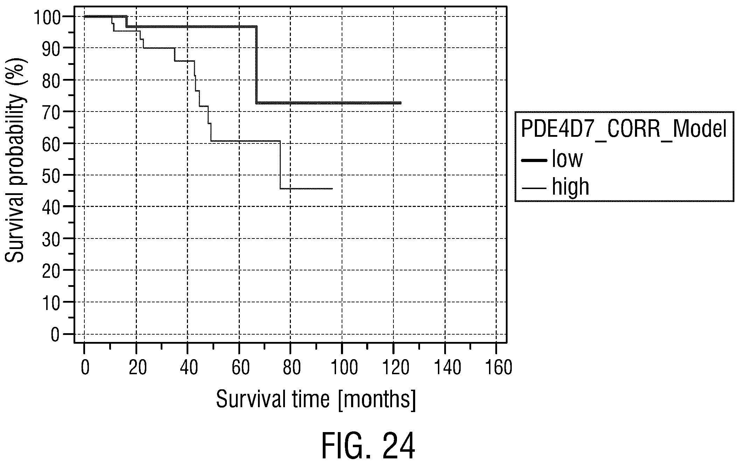

Fig. 24 shows a Kaplan-Meier curve of the PDE4D7_CORR_model in a 91 patient TCGA pRCC kidney cancer cohort (training set used to develop the PDE4D7_CORR_model). The clinical endpoint that was tested was the overall death (logrank p=0.05; HR=2.9; 95% CI=1.0 -8.8). The following supplementary lists indicate the number of patients at risk for the PDE4D7_CORR_model classes analyzed (threshold=0), i.e., the patients at risk at any time interval +20 months are shown: Low risk: 41, 26, 14, 6, 2, 1, 1, 0; High risk: 50, 36, 20, 8, 3, 0, 0, 0.

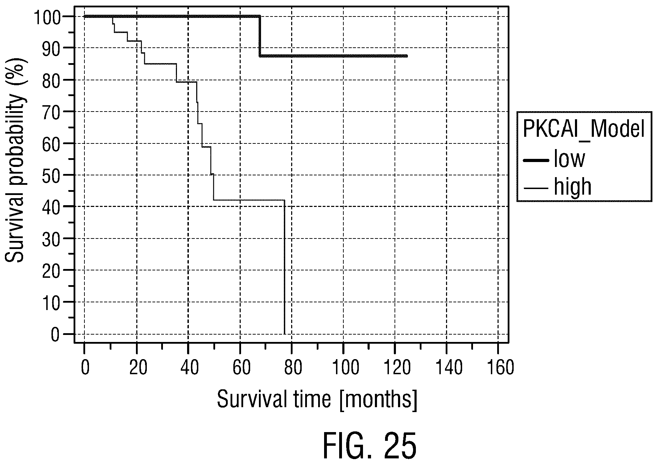

Fig. 25 shows a Kaplan-Meier curve of the PKCAI_model in a 91 patient TCGA pRCC kidney cancer cohort (training set used to develop the PKCAI_model). The clinical endpoint that was tested was the overall death (logrank p<0.0001; HR=11.8; 95% CI=3.7-37.4). The following supplementary lists indicate the number of patients at risk for the PKCAI_model classes analyzed (threshold=0), i.e., the patients at risk at any time interval +20 months are shown: Low risk: 45, 35, 22, 11, 5, 1, 1, 0; High risk: 46, 27, 12, 3, 0, 0, 0, 0.

The Kaplan-Meier survival curve analysis as shown in Figs. 2 to 25 demonstrates the presence of different patient risk groups. The risk group of a patient is determined by the probability to suffer from the respective clinical endpoint (overall death) as calculated by the respective risk model as shown in the figures. Depending on the predicted risk of a patient (i.e., depending on in which risk group the patient may belong) to die from bladder or kidney cancer different types of interventions might be indicated. In the lower risk groups standard of care (SOC) delivers acceptable long-term oncological control. For the patient group demonstrating a higher risk to eventually die from bladder or kidney cancer treatment escalation (e.g., multi-modality treatments of radiation and chemotherapy) might provide improved longitudinal survival outcomes. Alternative options for treatment escalation adjuvant therapies with radiation of cytotoxic drugs or alternative therapies like immunotherapies (e.g., atezolizumab; pembrolizumab; nivolumab; avelumab; durvalumab) or other experimental therapies.

**Further results**

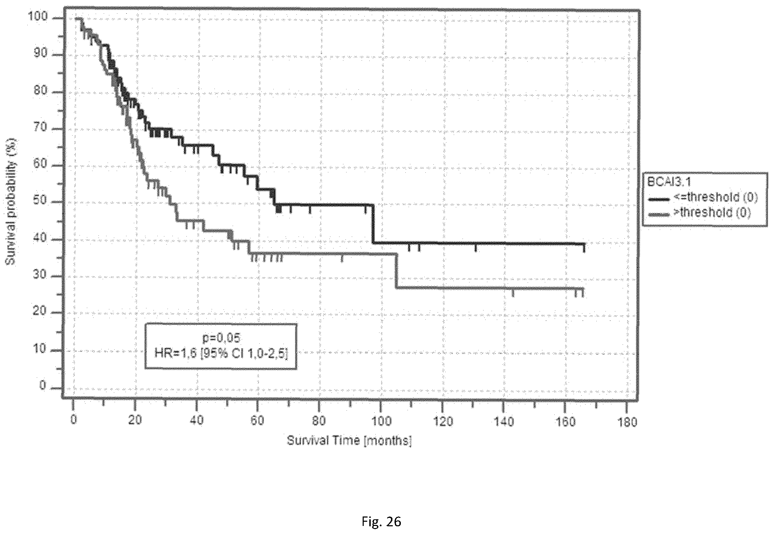

This section shows additional results for Cox regression models based on a multitude of gene models for both bladder cancer and kidney cancer, comprising randomly selected combinations of three (Table 7 & 9) or four genes (Table 8 & 10), wherein said three or four genes comprise per random combination of genes at least one immune defense response gene (IDR), at least one T-cell receptor (TCR) signaling gene and at least one PDE4D7 correlated gene. Variables and corresponding weights are provided in the Tables 7 - 10. The Cox regression models are plotted in the Kaplan-Meier curve analyses of Figures 26 - 49.

TABLE 7: Variables and weights for the six gene Cox regression models (BCAI 3.1 - 3.6) comprising random selected combinations of three genes, having per random combination one immune defense response gene, one TCR signaling gene and one PDE4D7 correlated gene, for bladder cancer.

| | Variable | BCAI 3 combinations regression models | | | | | |
| --- | --- | --- | --- | --- | --- | --- | --- |
| | | 3.1 | 3.2 | 3.3 | 3.4 | 3.5 | 3.6 |
| **Immune defense** | **AIM2** | **-** | **-** | **-** | **-** | **-** | **-** |
| | **APOBEC3A** | **-** | **-** | **-** | **-** | **-** | **-** |
| **response genes** | **CIAO1** | - | - | - | - | - | - |
| | **DDX58** | - | - | - | - | - | -0,104 |
| | **DHX9** | - | - | 0,292 | - | - | - |
| | **IFI16** | 0,147 | - | - | - | - | - |
| | **IFIH1** | - | - | - | - | - | - |
| | **IFIT1** | - | - | - | - | - | - |
| | **IFIT3** | - | - | - | 0,04357 | - | - |
| | **LRRFIP1** | - | 0,09355 | - | - | - | - |
| | **MYD88** | - | - | - | - | - | - |
| | **OAS1** | - | - | - | - | -0,2872 | - |
| | **TLR8** | - | - | - | - | - | - |
| | **ZBP1** | - | - | - | - | - | - |
| **TCR signaling genes** | **CD2** | - | - | - | - | - | - |
| | **CD247** | - | - | - | - | - | - |
| | **CD28** | - | - | - | - | - | - |
| | **CD3E** | - | - | - | - | - | - |
| | **CD3G** | - | - | - | - | - | - |
| | **CD4** | - | - | - | - | - | - |
| | **CSK** | - | - | - | - | - | - |
| | **EZR** | - | - | - | - | - | - |
| | **FYN** | - | 0,1015 | - | - | - | - |
| | **LAT** | - | - | - | - | - | - |
| | **LCK** | - | - | - | - | - | - |
| | **PAG1** | - | - | - | -0,2824 | - | - |
| | **PDE4D** | - | - | 0,2263 | - | - | - |
| | **PRKACA** | 0,1749 | - | - | - | - | 0,1735 |
| | **PRKACB** | - | - | - | - | - | - |
| | **PTPRC** | - | - | - | - | 0,01938 | - |
| | **ZAP70** | - | - | - | - | - | - |

(continued)

| | | BCAI 3 combinations regression models | | | | | |
|---|---|---|---|---|---|---|---|
| | Variable | 3.1 | 3.2 | 3.3 | 3.4 | 3.5 | 3.6 |
| **PDE4D7 correlated genes** | ABCC5 | - | - | - | 0,01201 | - | - |
| | CUX2 | - | -0,3666 | - | - | - | - |
| | HIAA1549 | - | - | - | - | - | - |
| | PDE4D | - | - | - | - | - | - |
| | RAP1GAP2 | 0,07386 | - | - | - | - | - |
| | SLC39A11 | - | - | - | - | - | -0,1823 |
| | TDRD1 | - | - | - | - | 0,2729 | - |
| | VWA2 | - | - | -0,0004 | - | - | - |

TABLE 8: Variables and weights for the six gene Cox regression models (BCAI 4.1 - 4.6) comprising random selected combinations of four genes, having per random combination at least one immune defense response gene, one TCR signaling gene and one PDE4D7 correlated gene and one randomly selected additional gene selected from either immune defense response genes, TCR signaling genes or PDE4D7 correlated genes, for bladder cancer.

| | | BCAI 4 combinations regression models | | | | | |
|---|---|---|---|---|---|---|---|
| | Variable | 4.1 | 4.2 | 4.3 | 4.4 | 4.5 | 4.6 |
| **Immune defense response genes** | AIM2 | - | - | - | - | - | - |
| | APOBEC3A | - | - | - | - | - | 0,1843 |
| | CIAO1 | - | - | - | - | - | - |
| | DDX58 | -0,221 | - | - | - | - | - |
| | DHX9 | - | 0,2533 | - | - | - | - |
| | IFI16 | - | - | 0,07751 | - | - | - |
| | IFIH1 | 0,1764 | - | - | - | - | - |
| | IFIT1 | - | - | - | - | - | - |
| | IFIT3 | - | - | - | - | - | - |
| | LRRFIP1 | - | - | - | 0,08143 | - | - |
| | MYD88 | - | -0,2409 | - | - | - | - |
| | OAS1 | - | - | - | - | - | - |
| | TLR8 | - | - | - | - | 0,5757 | - |
| | ZBP1 | - | - | - | - | - | - |

(continued)

|  | Variable | 4.1 | 4.2 | 4.3 | 4.4 | 4.5 | 4.6 |
|---|---|---|---|---|---|---|---|
|  |  | **BCAI 4 combinations regression models** | | | | | |
| **TCR signaling genes** | **CD2** | - | - | - | - | - | - |
|  | **CD247** | - | - | - | - | - | -0,1394 |
|  | **CD28** | - | - | - | - | - | - |
|  | **CD3E** | - | - | - | - | - | - |
|  | **CD3G** | - | - | - | - | - | - |
|  | **CD4** | - | - | - | - | -0,05087 | - |
|  | **CSK** | - | - | - | - | - | - |
|  | **EZR** | - | - | - | - | - | 0,04952 |
|  | **FYN** | - | - | - | - | - | - |
|  | **LAT** | - | -0,06302 | - | - | - | - |
|  | **LCK** | - | - | - | - | - | - |
|  | **PAG1** | - | - | -0,2481 | - | - | - |
|  | **PDE4D** | - | - | - | 0,1432 | - | - |
|  | **PRKACA** | - | - | 0,1475 | - | - | - |
|  | **PRKACB** | 0,06364 | - | - | - | - | - |
|  | **PTPRC** | - | - | - | - | - | - |
|  | **ZAP70** | - | - | - | - | -0,5158 | - |
| **PDE4D7 correlated genes** | **ABCC5** | - | - | - | - | - | - |
|  | **CUX2** | - | - | - | -0,4093 | - | - |
|  | **HIAA1549** | - | - | 0,02636 | - | - | - |
|  | **PDE4D** | - | 0,2377 | - | - | - | - |
|  | **RAP1GAP2** | - | - | - | - | - | - |
|  | **SLC39A11** | -0,2068 | - | - | - | - | - |
|  | **TDRD1** | - | - | - | 0,3614 | - | 0,3706 |
|  | **VWA2** | - | - | - | - | 0,03537 | - |

EP 4 222 286 B1

TABLE 9: Variables and weights for the six gene Cox regression models (KCAI 3.1 - 3.6) comprising random selected combinations of three genes, having per random combination one immune defense response gene, one TCR signaling gene and one PDE4D7 correlated gene, for kidney cancer.

| | | KCAI 3 combinations regression models | | | | | |
|---|---|---|---|---|---|---|---|
| | Variable | 3.1 | 3.2 | 3.3 | 3.4 | 3.5 | 3.6 |
| Immune defense response genes | AIM2 | - | - | - | - | - | - |
| | APOBEC3A | - | - | - | - | - | - |
| | CIAO1 | - | - | - | - | - | - |
| | DDX58 | - | - | - | - | - | -0,2949 |
| | DHX9 | - | - | -0,1177 | - | - | - |
| | IFI16 | 0,301 | - | - | - | - | - |
| | IFIH1 | - | - | - | - | - | - |
| | IFIT1 | - | - | - | - | -0,07245 | - |
| | IFIT3 | - | - | - | -0,1179 | - | - |
| | LRRFIP1 | - | 0,05819 | - | - | - | - |
| | MYD88 | - | - | - | - | - | - |
| | OAS1 | - | - | - | - | - | - |
| | TLR8 | - | - | - | - | - | - |
| | ZBP1 | - | - | - | - | - | - |
| TCR signaling genes | CD2 | - | - | - | - | - | - |
| | CD247 | - | - | - | - | - | - |
| | CD28 | - | - | - | - | - | - |
| | CD3E | - | - | - | - | - | - |
| | CD3G | - | - | - | - | -0,05339 | - |
| | CD4 | - | - | - | - | - | - |
| | CSK | - | - | - | - | - | - |
| | EZR | - | - | - | - | - | - |
| | FYN | - | -0,04316 | - | - | - | - |
| | LAT | - | - | - | - | - | - |
| | LCK | - | - | - | - | - | 0,1625 |
| | PAG1 | - | - | - | 0,1077 | - | - |
| | PDE4D | - | - | -0,3274 | - | - | - |
| | PRKACA | 0,2932 | - | - | - | - | - |
| | PRKACB | - | - | - | - | - | - |
| | PTPRC | - | - | - | - | - | - |
| | ZAP70 | - | - | - | - | - | - |

45

(continued)

| | | KCAI 3 combinations regression models | | | | | |
|---|---|---|---|---|---|---|---|
| | Variable | 3.1 | 3.2 | 3.3 | 3.4 | 3.5 | 3.6 |
| PDE4D7 correlated genes | ABCC5 | - | - | - | 0,2526 | - | - |
| | CUX2 | - | -0,1284 | - | - | - | - |
| | HIAA1549 | - | - | - | - | -0,3318 | - |
| | PDE4D | - | - | - | - | - | - |
| | RAP1GAP2 | -0,0557 | - | - | - | - | - |
| | SLC39A11 | - | - | - | - | - | 0,1654 |
| | TDRD1 | - | - | - | - | - | - |
| | VWA2 | - | - | 0,04484 | - | - | - |

TABLE 10: Variables and weights for the six gene Cox regression models (KCAI 4.1 - 4.6) comprising random selected combinations of four genes, having per random combination at least one immune defense response gene, one TCR signaling gene and one PDE4D7 correlated gene and one randomly selected additional gene selected from either immune defense response genes, TCR signaling genes or PDE4D7 correlated genes, for kidney cancer.

| | | KCAI 4 combinations regression models | | | | | |
|---|---|---|---|---|---|---|---|
| | Variable | 4.1 | 4.2 | 4.3 | 4.4 | 4.5 | 4.6 |
| Immune defense | AIM2 | - | - | - | - | - | - |
| | APOBEC3A | - | - | - | - | - | 0,1466 |
| response genes | CIAO1 | - | - | - | - | - | - |
| | DDX58 | -0,1104 | - | - | - | - | - |
| | DHX9 | - | -0,1613 | - | - | - | - |
| | IFI16 | - | - | 0,2762 | - | - | - |
| | IFIH1 | -0,06913 | - | - | - | - | - |
| | IFIT1 | - | - | - | - | - | - |
| | IFIT3 | - | - | - | - | - | - |
| | LRRFIP1 | - | - | - | 0,06676 | - | - |
| | MYD88 | - | 0,1535 | - | - | - | - |
| | OAS1 | - | - | - | - | - | - |
| | TLR8 | - | - | - | - | -0,156 | - |
| | ZBP1 | - | - | - | - | - | - |

(continued)

| | | KCAI 4 combinations regression models | | | | | |
|---|---|---|---|---|---|---|---|
| | Variable | 4.1 | 4.2 | 4.3 | 4.4 | 4.5 | 4.6 |
| TCR signaling genes | CD2 | - | - | - | - | - | - |
| | CD247 | - | - | - | - | - | -0,02117 |
| | CD28 | - | - | - | - | - | - |
| | CD3E | - | - | - | - | - | - |
| | CD3G | - | - | - | - | - | - |
| | CD4 | - | - | - | - | -0,09589 | - |
| | CSK | - | - | - | - | - | - |
| | EZR | - | - | - | - | - | -0,3415 |
| | FYN | - | - | - | - | - | - |
| | LAT | - | 0,2072 | - | - | - | - |
| | LCK | - | - | - | - | - | - |
| | PAG1 | - | - | -0,05292 | - | - | - |
| | PDE4D | - | - | - | -0,3915 | - | - |
| | PRKACA | - | - | 0,1934 | - | - | - |
| | PRKACB | -0,1469 | - | - | - | - | - |
| | PTPRC | - | - | - | - | - | - |
| | ZAP70 | - | - | - | - | 0,3894 | - |
| PDE4D7 correlated genes | ABCC5 | 0,1768 | - | - | - | - | - |
| | CUX2 | - | - | - | -0,1205 | - | - |
| | HIAA1549 | - | - | -0,3103 | - | - | - |
| | PDE4D | - | -0,3054 | - | - | - | - |
| | RAP1GAP2 | - | - | - | - | - | -0,0714 |
| | SLC39A11 | - | - | - | - | - | - |
| | TDRD1 | - | - | - | -0,0264 | - | - |
| | VWA2 | - | - | - | - | 0,02667 | - |

For Kaplan-Meier curve analysis the Cox regression function of the 24 risk models as shown in Tables 7 - 10 (BCAI-3.1 - 3.6, BCAI-4.1 - 4.6, KCAI 3.1 - 3.6 and KCAI 4.1 - 4.6) were categorized into two sub-cohorts (low risk vs. high risk) based on a cut-off (see description of the figures 26 - 49 below). The threshold for group separation into low and high risk was based on the risk to experience the clinical endpoint (outcome) as predicted by the respective Cox regression model.

In Figures 26 - 37 a 189 patient TCGA bladder cancer cohort (total number of patients with non-metastasized disease (m0)) was tested for the endpoint: overall death.

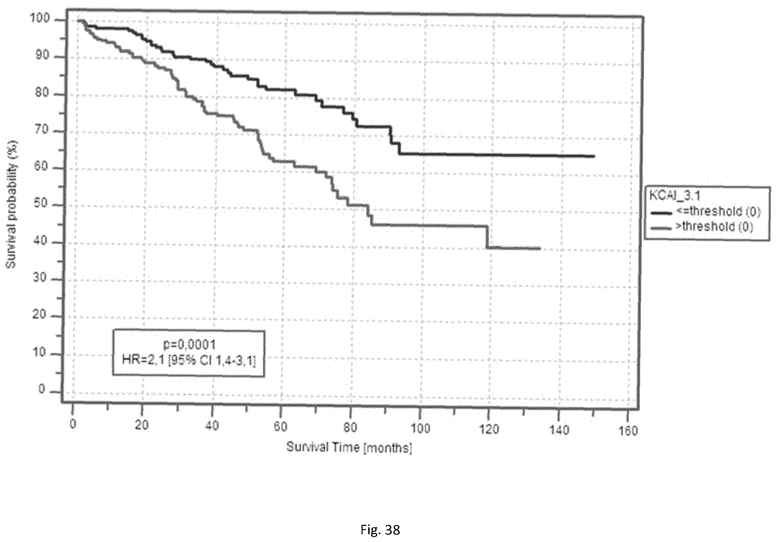

In Figures 38 - 49 a 411 patient TCGA ccRCC kidney cancer cohort (total number of patients with non-metastasized disease (m0)) was tested for the endpoint: overall death.

Figure 26 shows a Kaplan-Meier curve of the BCAI_3.1 model in a 189 patient TCGA bladder cancer cohort (total number of patients with non-metastasized disease (m0), comprising both the training set and validation set). The clinical endpoint that was tested was the overall death (logrank p=0.05; HR=1.6; 95% CI=1.0-2.5). The following supplementary lists indicate the number of patients at risk for the BCAI_model classes analyzed (threshold=0), i.e., the patients at risk at any time interval +20 months are shown: Low risk (<=0): 99, 49, 27, 15, 6, 4, 2, 1, 1, 0; High risk (>0): 90, 39, 18, 9, 5, 4, 3, 3, 2,0.

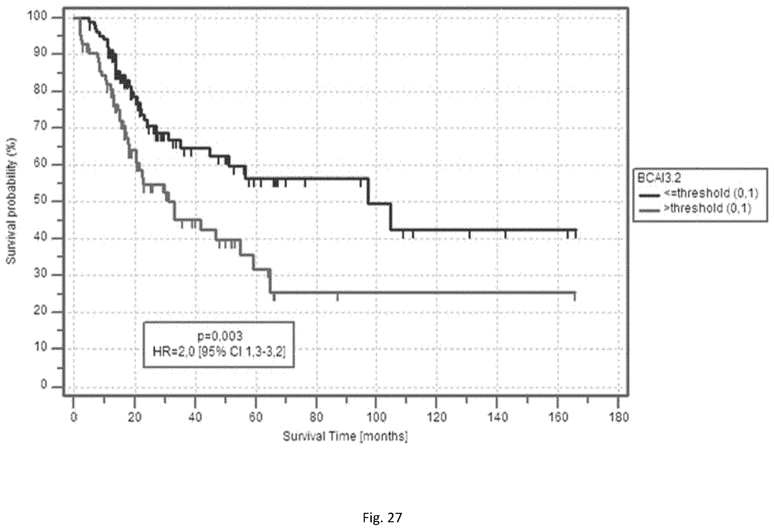

Figure 27 shows a Kaplan-Meier curve of the BCAI_3.2 model in a 189 patient TCGA bladder cancer cohort (total number of patients with non-metastasized disease (m0), comprising both the training set and validation set). The clinical endpoint that was tested was the overall death (logrank p=0.003; HR=2.0; 95% CI=1.3-3.2). The following supplementary lists indicate the number of patients at risk for the BCAI_model classes analyzed (threshold=0.1), i.e., the patients at risk at any time interval +20 months are shown: Low risk (<=0.1): 104, 52, 28, 16, 9, 7, 4, 3, 2, 0; High risk (>0.1): 85, 36, 17, 8, 2, 1, 1, 1, 1,0.

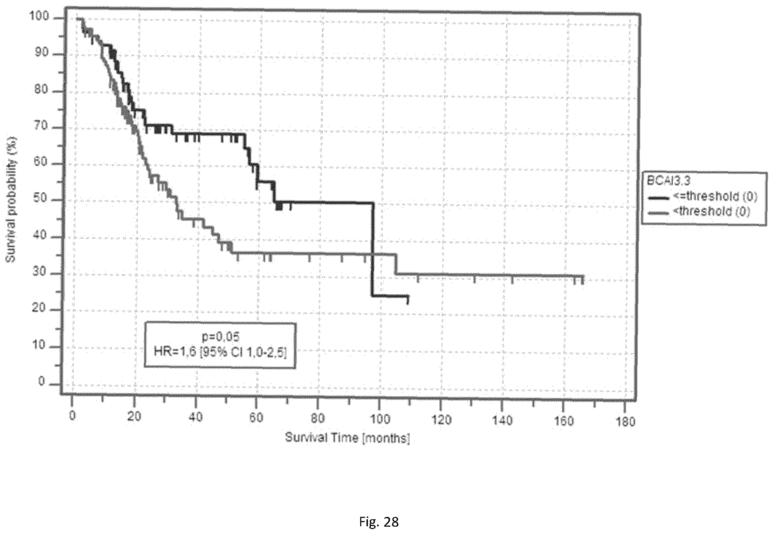

Figure 28 shows a Kaplan-Meier curve of the BCAI_3.3 model in a 189 patient TCGA bladder cancer cohort (total number of patients with non-metastasized disease (m0), comprising both the training set and validation set). The clinical endpoint that was tested was the overall death (logrank p=0.05; HR=1.6; 95% CI=1.0-2.5). The following supplementary lists indicate the number of patients at risk for the BCAI_model classes analyzed (threshold=0), i.e., the patients at risk at any time interval +20 months are shown: Low risk (<=0): 83, 39, 24, 11, 2, 1, 0, 0, 0; High risk (>0): 106, 49, 21, 13, 9, 7, 5, 4,3,0.

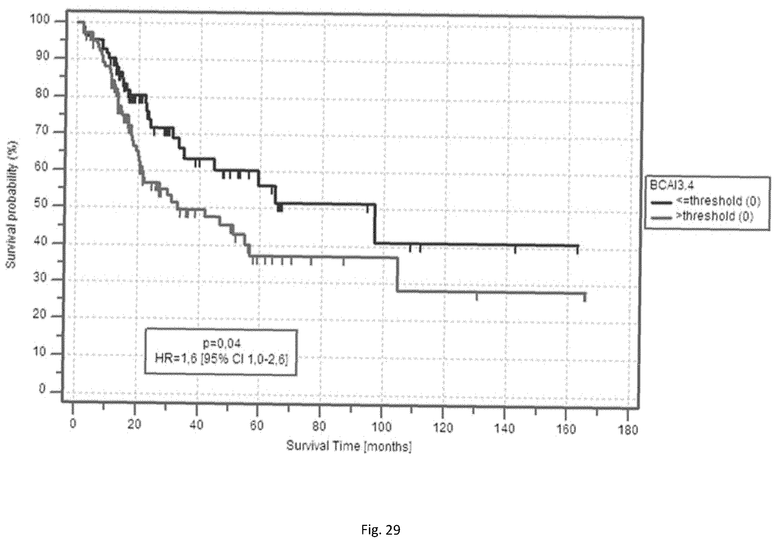

Figure 29 shows a Kaplan-Meier curve of the BCAI_3.4 model in a 189 patient TCGA bladder cancer cohort (total number of patients with non-metastasized disease (m0), comprising both the training set and validation set). The clinical endpoint that was tested was the overall death (logrank p=0.04; HR=1.6; 95% CI=1.0-2.6). The following supplementary lists indicate the number of patients at risk for the BCAI_model classes analyzed (threshold=0), i.e., the patients at risk at any time interval +20 months are shown: Low risk (<=0): 84, 41, 22, 13, 6, 4, 2, 2, 1, 0; High risk (>0): 105, 47, 23, 11, 5, 4, 3, 2, 2, 0.

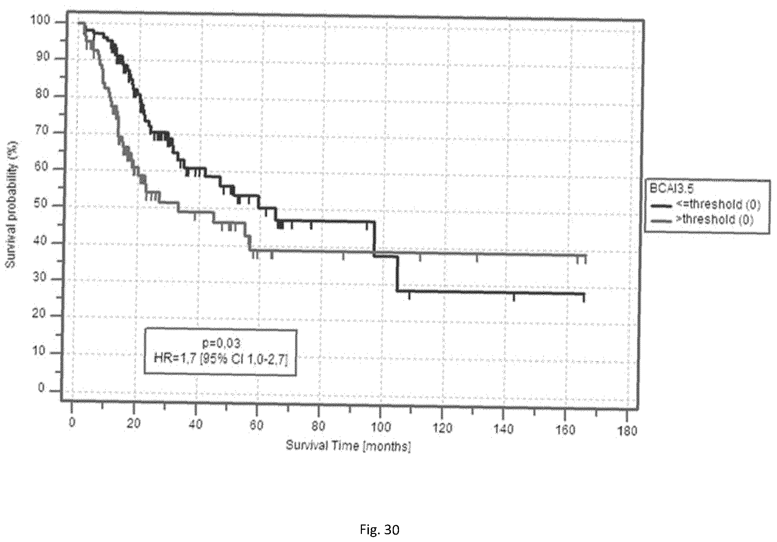

Figure 30 shows a Kaplan-Meier curve of the BCAI_3.5 model in a 189 patient TCGA bladder cancer cohort (total number of patients with non-metastasized disease (m0), comprising both the training set and validation set). The clinical endpoint that was tested was the overall death (logrank p=0.03; HR=1.7; 95% CI=1.0-2.7). The following supplementary lists indicate the number of patients at risk for the BCAI_model classes analyzed (threshold=0), i.e., the patients at risk at any time interval +20 months are shown: Low risk (<=0): 107, 59, 27, 16, 6, 4, 2, 2, 1, 0; High risk (>0): 82, 29, 18, 8, 5, 4, 3, 2, 2, 0.

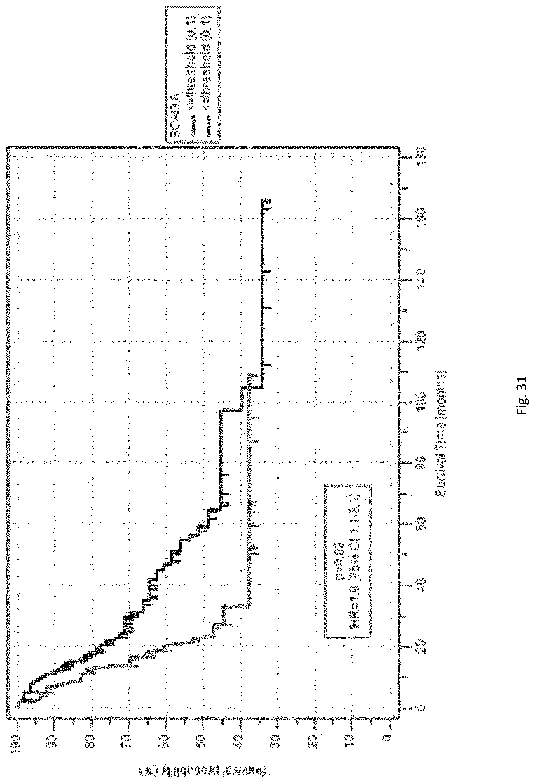

Figure 31 shows a Kaplan-Meier curve of the BCAI_3.6 model in a 189 patient TCGA bladder cancer cohort (total number of patients with non-metastasized disease (m0), comprising both the training set and validation set). The clinical endpoint that was tested was the overall death (logrank p=0.02; HR=1.9; 95% CI=1.1-3.1). The following supplementary lists indicate the number of patients at risk for the BCAI_model classes analyzed (threshold=0.1), i.e., the patients at risk at any time interval +20 months are shown: Low risk (<=0.1): 122, 64, 34, 18, 8, 7, 5, 4, 3, 0; High risk (>0.1): 67, 24, 11, 6, 3, 1, 0, 0, 0, 0.

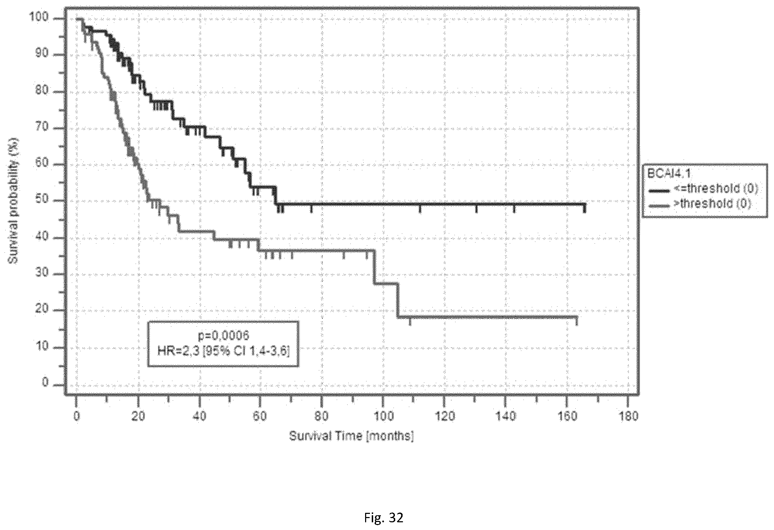

Figure 32 shows a Kaplan-Meier curve of the BCAI_4.1 model in a 189 patient TCGA bladder cancer cohort (total number of patients with non-metastasized disease (m0), comprising both the training set and validation set). The clinical endpoint that was tested was the overall death (logrank p=0.0006; HR=2.3; 95% CI=1.4-3.6). The following supplementary lists indicate the number of patients at risk for the BCAI_model classes analyzed (threshold=0), i.e., the patients at risk at any time interval +20 months are shown: Low risk (<=0): 92, 49, 26, 12, 5, 5, 4, 3, 2, 0; High risk (>0): 97, 39, 19, 12, 6, 3, 1, 1, 1,0.

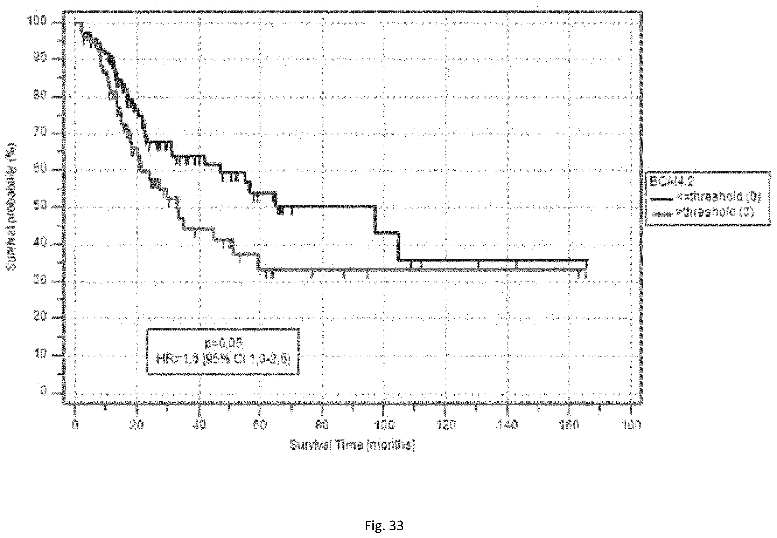

Figure 33 shows a Kaplan-Meier curve of the BCAI_4.2 model in a 189 patient TCGA bladder cancer cohort (total number of patients with non-metastasized disease (m0), comprising both the training set and validation set). The clinical endpoint that was tested was the overall death (logrank p=0.05; HR=1.6; 95% CI=1.0-2.6). The following supplementary lists indicate the number of patients at risk for the BCAI_model classes analyzed (threshold=0), i.e., the patients at risk at any time interval +20 months are shown: Low risk (<=0): 111, 54, 30, 16, 7, 6, 3, 2, 1, 0; High risk (>0): 78, 34, 15, 8, 4, 2, 2, 2, 2, 0.

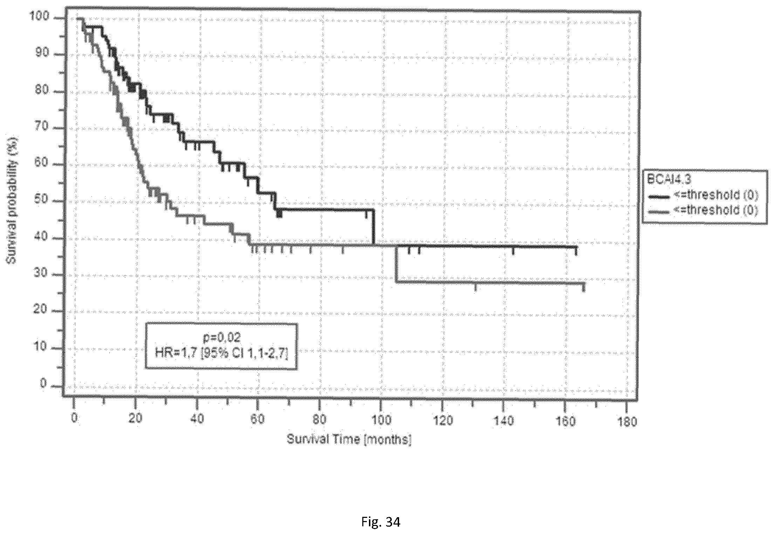

Figure 34 shows a Kaplan-Meier curve of the BCAI_4.3 model in a 189 patient TCGA bladder cancer cohort (total number of patients with non-metastasized disease (m0), comprising both the training set and validation set). The clinical endpoint that was tested was the overall death (logrank p=0.02; HR=1.7; 95% CI=1.1-2.7). The following supplementary lists indicate the number of patients at risk for the BCAI_model classes analyzed (threshold=0), i.e., the patients at risk at any time interval +20 months are shown: Low risk (<=0): 89, 44, 24, 13, 6, 4, 2, 2, 1, 0; High risk (>0): 100, 44, 21, 11, 5, 4, 3, 2, 2, 0.

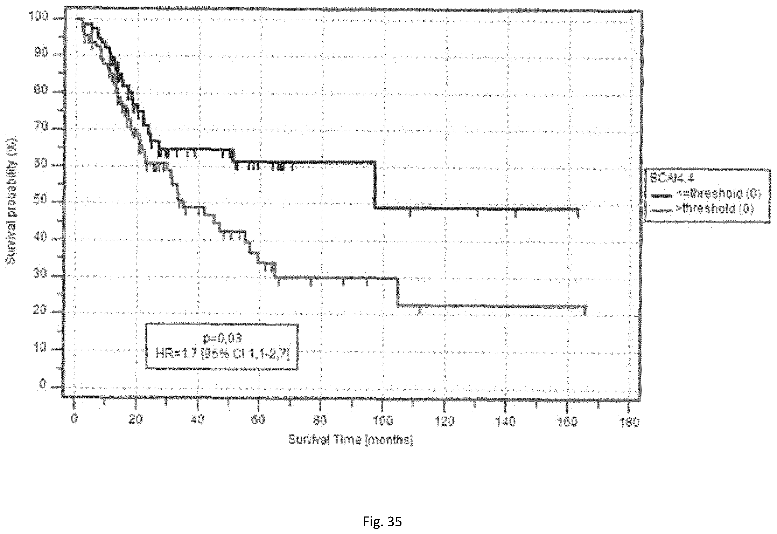

Figure 35 shows a Kaplan-Meier curve of the BCAI_4.4 model in a 189 patient TCGA bladder cancer cohort (total number of patients with non-metastasized disease (m0), comprising both the training set and validation set). The clinical endpoint that was tested was the overall death (logrank p=0.03; HR=1.7; 95% CI=1.1-2.7). The following supplementary lists indicate the number of patients at risk for the BCAI_model classes analyzed (threshold=0), i.e., the patients at risk at any time interval +20 months are shown: Low risk (<=0): 78, 41, 22, 12, 5, 4, 3, 2, 1, 0; High risk (>0): 111, 47, 23, 12, 6, 4, 2, 2, 2, 0.

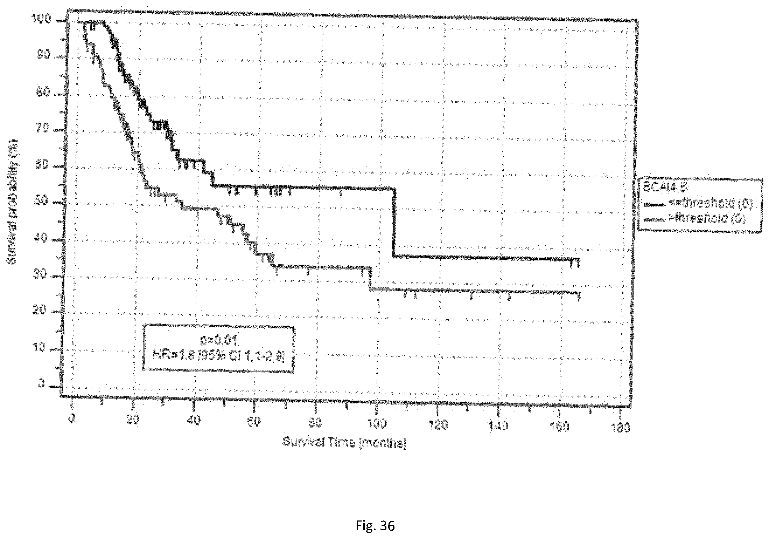

Figure 36 shows a Kaplan-Meier curve of the BCAI_4.5 model in a 189 patient TCGA bladder cancer cohort (total number of patients with non-metastasized disease (m0), comprising both the training set and validation set). The clinical endpoint that was tested was the overall death (logrank p=0.01; HR=1.8; 95% CI=1.1-2.9). The following supplementary lists indicate the number of patients at risk for the BCAI_model classes analyzed (threshold=0), i.e., the patients at risk at any time interval +20 months are shown: Low risk (<=0): 90, 46, 18, 11, 4, 3, 2, 2, 2, 0; High risk (>0):

99, 42, 27, 13, 7, 5, 3, 2, 1,0.

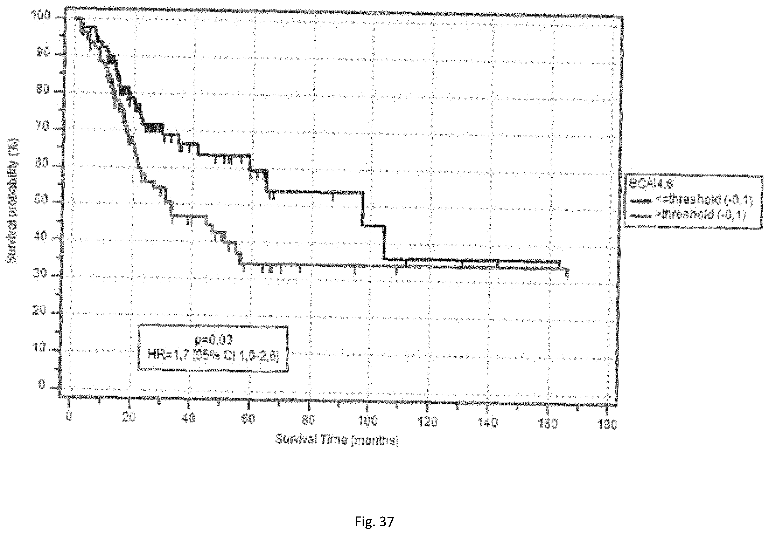

Figure 37 shows a Kaplan-Meier curve of the BCAI_4.6 model in a 189 patient TCGA bladder cancer cohort (total number of patients with non-metastasized disease (m0), comprising both the training set and validation set). The clinical endpoint that was tested was the overall death (logrank p=0.03; HR=1.7; 95% CI=1.0-2.6). The following supplementary lists indicate the number of patients at risk for the BCAI_model classes analyzed (threshold=-0.1), i.e., the patients at risk at any time interval +20 months are shown: Low risk (<=-0.1): 82, 48, 22, 14, 7, 5, 3, 2, 1, 0; High risk (>-0.1): 107, 40, 23, 10, 4, 3, 2, 2, 2, 0.

Fig. 38 shows a Kaplan-Meier curve of the KCAI_3.1 model in a 411 patient TCGA ccRCC kidney cancer cohort (total number of patients with non-metastasized disease (m0), comprising both the training set and validation set). The clinical endpoint that was tested was the overall death (logrank p=0.0001; HR=2.1; 95% CI=1.4-3.1). The following supplementary lists indicate the number of patients at risk for the KCAI_model classes analyzed (threshold=0), i.e., the patients at risk at any time interval +20 months are shown: Low risk (<=0): 202, 172, 122, 74, 42, 20, 5, 1, 0; High risk (>0): 209, 161, 112, 59, 22, 13, 7, 0, 0.

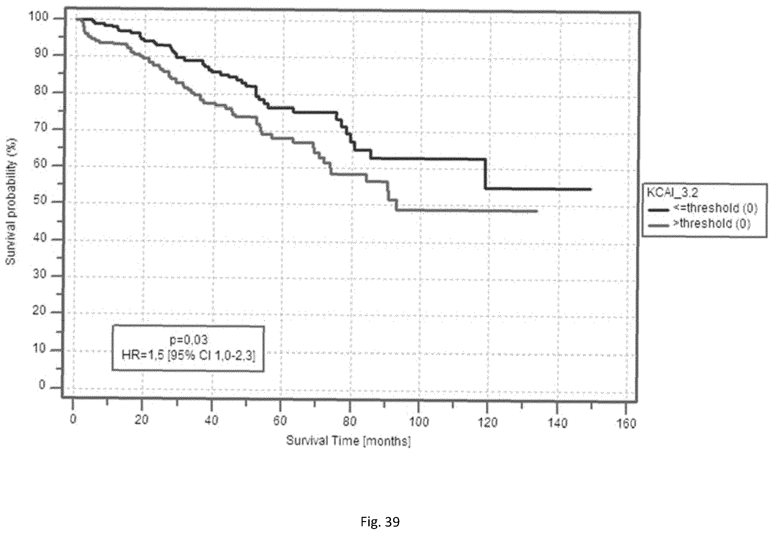

Fig. 39 shows a Kaplan-Meier curve of the KCAI_3.2 model in a 411 patient TCGA ccRCC kidney cancer cohort (total number of patients with non-metastasized disease (m0), comprising both the training set and validation set). The clinical endpoint that was tested was the overall death (logrank p=0.03; HR=1.5; 95% CI=1.0-2.3). The following supplementary lists indicate the number of patients at risk for the KCAI_model classes analyzed (threshold=0), i.e., the patients at risk at any time interval +20 months are shown: Low risk (<=0): 199, 175, 128, 70, 32, 18, 7, 1, 0; High risk (>0): 212, 158, 106, 63, 32, 15, 5, 0, 0.

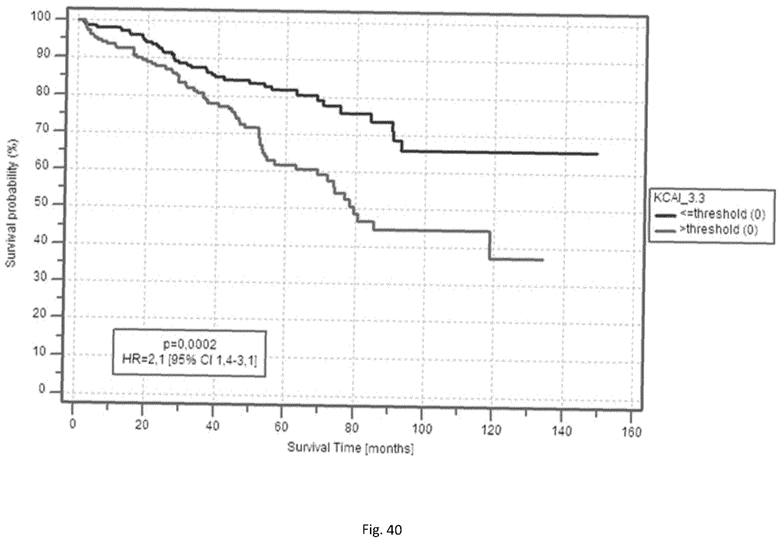

Fig. 40 shows a Kaplan-Meier curve of the KCAI_3.3 model in a 411 patient TCGA ccRCC kidney cancer cohort (total number of patients with non-metastasized disease (m0), comprising both the training set and validation set). The clinical endpoint that was tested was the overall death (logrank p=0.0002; HR=2.1; 95% CI=1.4-3.1). The following supplementary lists indicate the number of patients at risk for the KCAI_model classes analyzed (threshold=0), i.e., the patients at risk at any time interval +20 months are shown: Low risk (<=0): 221, 184, 128, 79, 39, 20, 8, 1, 0; High risk (>0): 190, 149, 106, 54, 25, 13, 4, 0, 0.

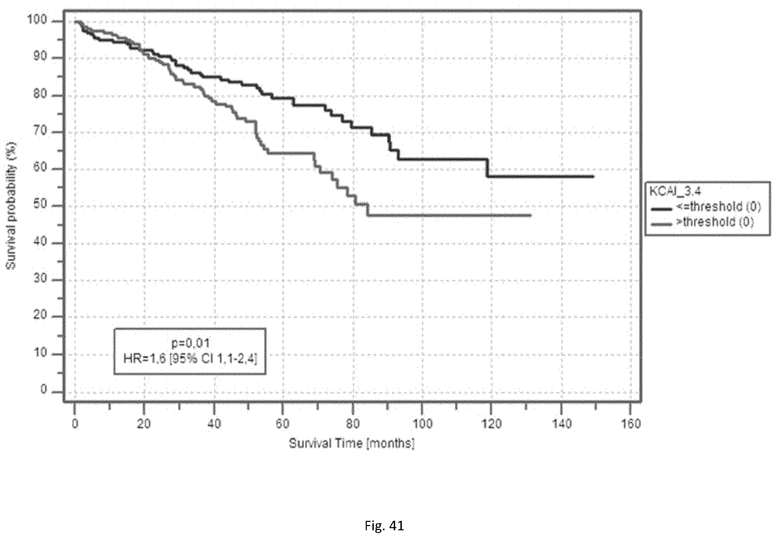

Fig. 41 shows a Kaplan-Meier curve of the KCAI_3.4 model in a 411 patient TCGA ccRCC kidney cancer cohort (total number of patients with non-metastasized disease (m0), comprising both the training set and validation set). The clinical endpoint that was tested was the overall death (logrank p=0.01; HR=1.6; 95% CI=1.1-2.4). The following supplementary lists indicate the number of patients at risk for the KCAI_model classes analyzed (threshold=0), i.e., the patients at risk at any time interval +20 months are shown: Low risk (<=0): 205, 167, 132, 82, 41, 24, 10, 1, 0; High risk (>0): 206, 166, 111, 51, 23, 9, 2, 0, 0.

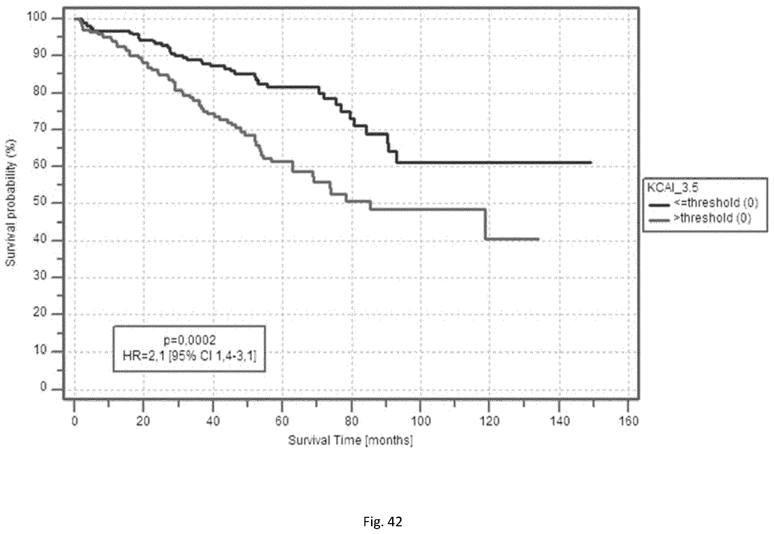

Fig. 42 shows a Kaplan-Meier curve of the KCAI_3.5 model in a 411 patient TCGA ccRCC kidney cancer cohort (total number of patients with non-metastasized disease (m0), comprising both the training set and validation set). The clinical endpoint that was tested was the overall death (logrank p=0.0002; HR=2.1; 95% CI=1.4-3.1). The following supplementary lists indicate the number of patients at risk for the KCAI_model classes analyzed (threshold=0), i.e., the patients at risk at any time interval +20 months are shown: Low risk (<=0): 245, 197, 135, 79, 38, 21, 7, 1, 0; High risk (>0): 166, 136, 99, 54, 26, 12, 5, 0, 0.

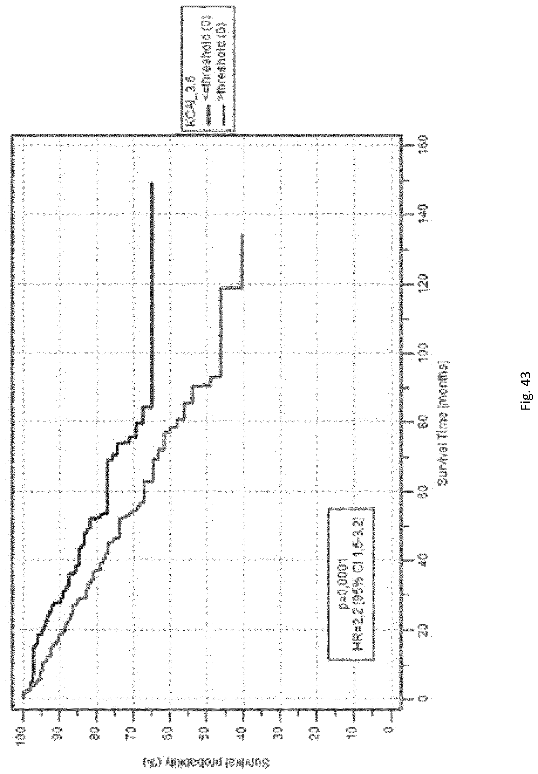

Fig. 43 shows a Kaplan-Meier curve of the KCAI_3.6 model in a 411 patient TCGA ccRCC kidney cancer cohort (total number of patients with non-metastasized disease (m0), comprising both the training set and validation set). The clinical endpoint that was tested was the overall death (logrank p=0.0001; HR=2.2; 95% CI=1.5-3.2). The following supplementary lists indicate the number of patients at risk for the KCAI_model classes analyzed (threshold=0), i.e., the patients at risk at any time interval +20 months are shown: Low risk (<=0): 215, 174, 121, 72, 34, 18, 6, 1, 0; High risk (>0): 196, 159, 113, 61, 30, 15, 6, 0, 0.

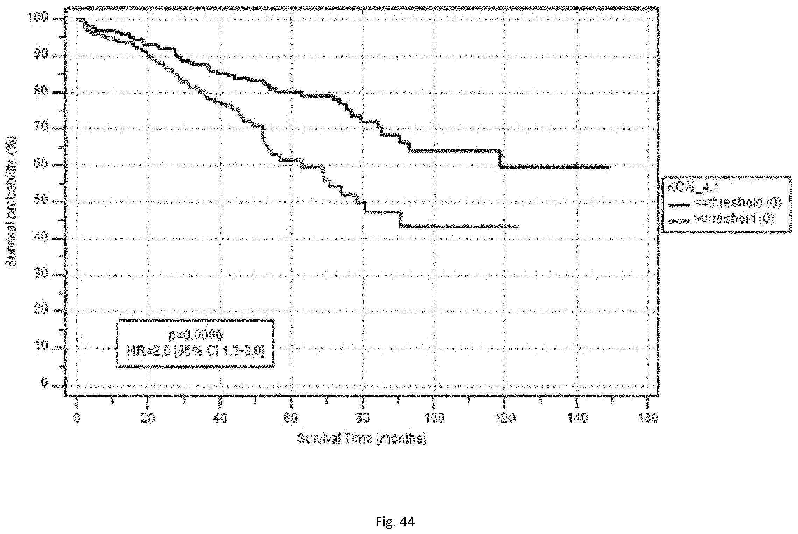

Fig. 44 shows a Kaplan-Meier curve of the KCAI_4.1 model in a 411 patient TCGA ccRCC kidney cancer cohort (total number of patients with non-metastasized disease (m0), comprising both the training set and validation set). The clinical endpoint that was tested was the overall death (logrank p=0.0006; HR=2.0; 95% CI=1.3-3.0). The following supplementary lists indicate the number of patients at risk for the KCAI_model classes analyzed (threshold=0), i.e., the patients at risk at any time interval +20 months are shown: Low risk (<=0): 232, 189, 135, 89, 45, 26, 11, 1, 0; High risk (>0): 179, 144, 99, 44, 19, 7, 1, 0, 0.

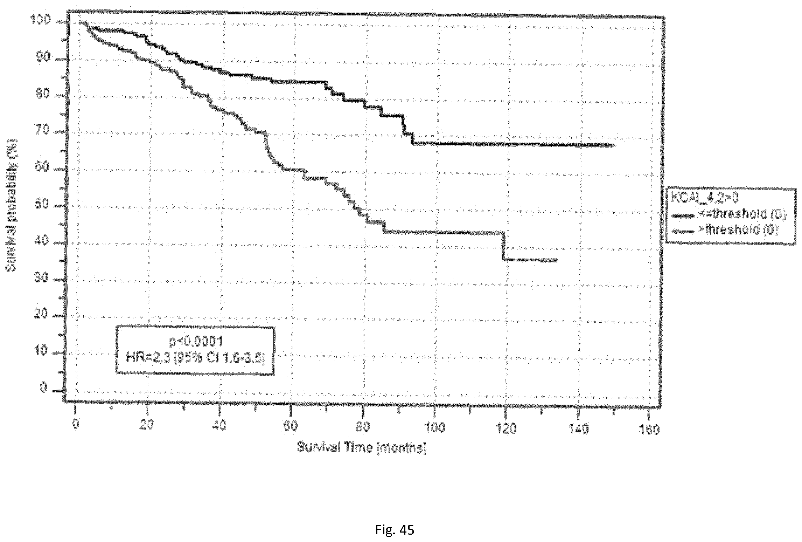

Fig. 45 shows a Kaplan-Meier curve of the KCAI_4.2 model in a 411 patient TCGA ccRCC kidney cancer cohort (total number of patients with non-metastasized disease (m0), comprising both the training set and validation set). The clinical endpoint that was tested was the overall death (logrank p<0.0001; HR=2.3; 95% CI=1.6-3.5). The following supplementary lists indicate the number of patients at risk for the KCAI_model classes analyzed (threshold=0), i.e., the patients at risk at any time interval +20 months are shown: Low risk (<=0): 206, 171, 122, 76, 40, 22, 7, 1, 0; High risk (>0): 205, 162, 112, 57, 24, 11, 5, 0, 0.

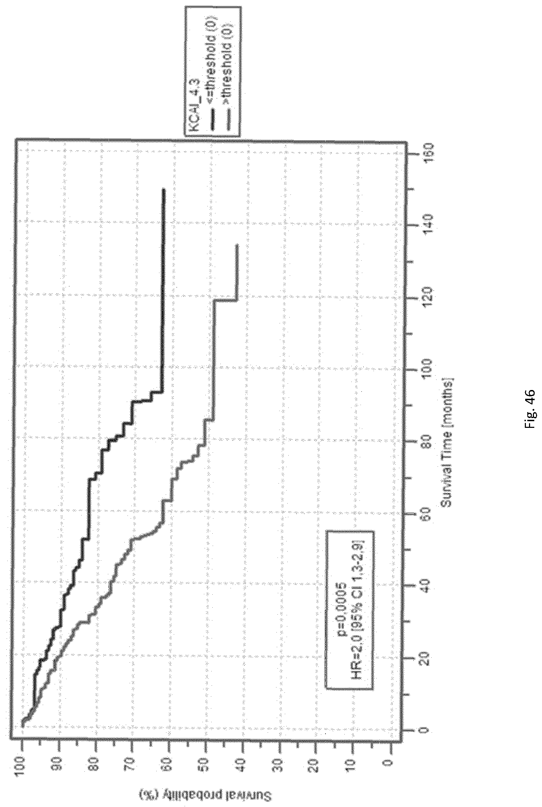

Fig. 46 shows a Kaplan-Meier curve of the KCAI_4.3 model in a 411 patient TCGA ccRCC kidney cancer cohort (total number of patients with non-metastasized disease (m0), comprising both the training set and validation set). The clinical endpoint that was tested was the overall death (logrank p=0.0005; HR=2.0; 95% CI=1.3-2.9). The following supplementary lists indicate the number of patients at risk for the KCAI_model classes analyzed (threshold=0), i.e., the patients at risk at any time interval +20 months are shown: Low risk (<=0): 221, 178, 124, 73, 39, 19, 5, 1, 0; High risk (>0): 190, 155, 110, 60, 25, 14, 7, 0, 0.

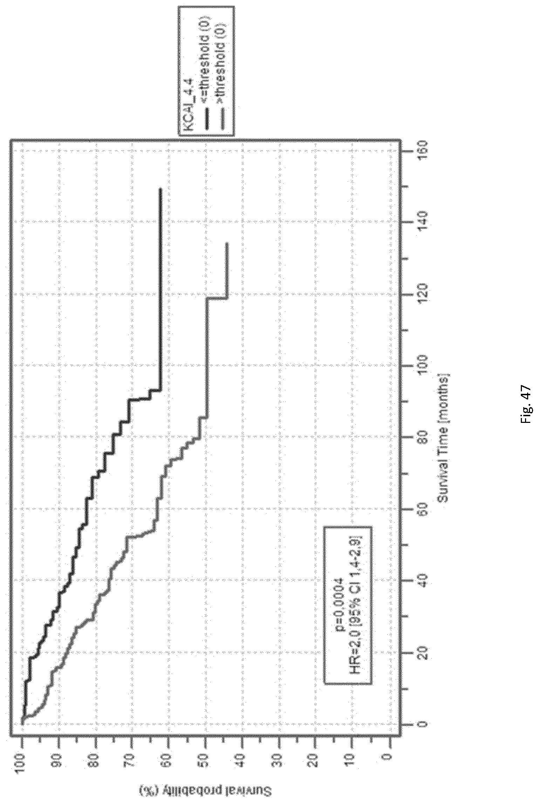

Fig. 47 shows a Kaplan-Meier curve of the KCAI_4.4 model in a 411 patient TCGA ccRCC kidney cancer cohort (total number of patients with non-metastasized disease (m0), comprising both the training set and validation set). ). The clinical endpoint that was tested was the overall death (logrank p=0.0004; HR=2.0; 95% CI=1.4-2.9). The following supplementary lists indicate the number of patients at risk for the KCAI_model classes analyzed (threshold=0), i.e., the patients at risk at any time interval +20 months are shown: Low risk (<=0): 205, 175, 120, 70, 35, 16, 6, 1, 0; High risk (>0): 206, 158, 114, 63, 29, 17, 6, 0, 0.

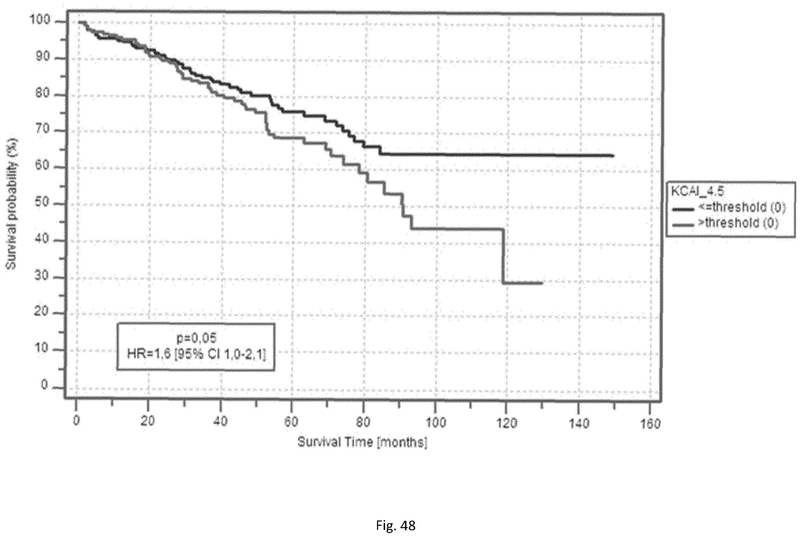

Fig. 48 shows a Kaplan-Meier curve of the KCAI_4.5 model in a 411 patient TCGA ccRCC kidney cancer cohort (total number of patients with non-metastasized disease (m0), comprising both the training set and validation set). The clinical endpoint that was tested was the overall death (logrank p=0.05; HR=1.6; 95% CI=1.0-2.1). The following supplementary lists indicate the number of patients at risk for the KCAI_model classes analyzed (threshold=0), i.e., the patients at risk at any time interval +20 months are shown: Low risk (<=0): 214, 173, 124, 76, 41, 22, 10, 1, 0; High risk (>0): 197, 160, 110, 57, 23, 11, 2, 0, 0.

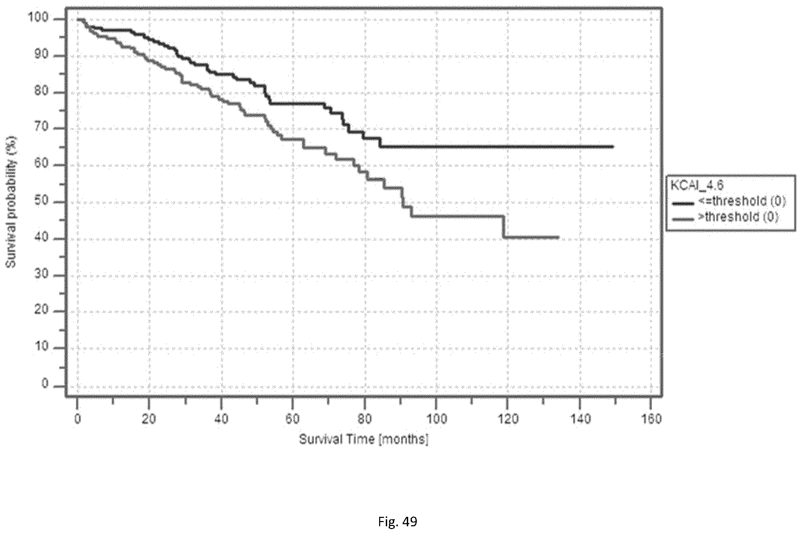

Fig. 49 shows a Kaplan-Meier curve of the KCAI_4.6 model in a 411 patient TCGA ccRCC kidney cancer cohort (total number of patients with non-metastasized disease (m0), comprising both the training set and validation set). The clinical endpoint that was tested was the overall death (logrank p=....; HR=......; 95% CI=........). The following supplementary lists indicate the number of patients at risk for the KCAI_model classes analyzed (threshold=0), i.e., the patients at risk at any time interval +20 months are shown: Low risk (<=0): 215, 174, 121, 72, 34, 18, 6, 1, 0; High risk (>0): 196, 159, 113, 61, 30, 15, 6, 0, 0.

The Kaplan-Meier survival curve analyses as shown in Figs. 26 to 49 demonstrate the presence of different patient risk groups. The risk group of a patient is determined by the probability to suffer from the respective clinical endpoint (outcome), i.e. overall death, as predicted by the respective risk model (see Tables 7 - 10) as shown in the figures. Depending on the predicted risk of a patient (i.e. depending on in which risk group the patient may belong) to suffer the clinical endpoint from bladder or kidney cancer different types of interventions may be indicated.

In the low/lower risk groups a standard of care (SOC) protocol delivers acceptable long-term oncological control. For the patient group demonstrating a high/higher risk to eventually die from bladder or kidney cancer treatment escalation (e.g., multi-modality treatments of radiation and chemotherapy) may provide improved longitudinal survival outcomes. Alternative options for treatment escalation adjuvant therapies with radiation of cytotoxic drugs or alternative therapies like immunotherapies (e.g., atezolizumab; pembrolizumab; nivolumab; avelumab; durvalumab) or other experimental therapies. In other words, the segregation into separate low risk and high risk patient risk groups in bladder cancer or kidney cancer based on the BCAI or KCAI risk models as provided herein (Tables 7 - 10) allows for a method to predict the outcome of a bladder cancer subject or kidney cancer subject and allows for prediction of a therapy response.

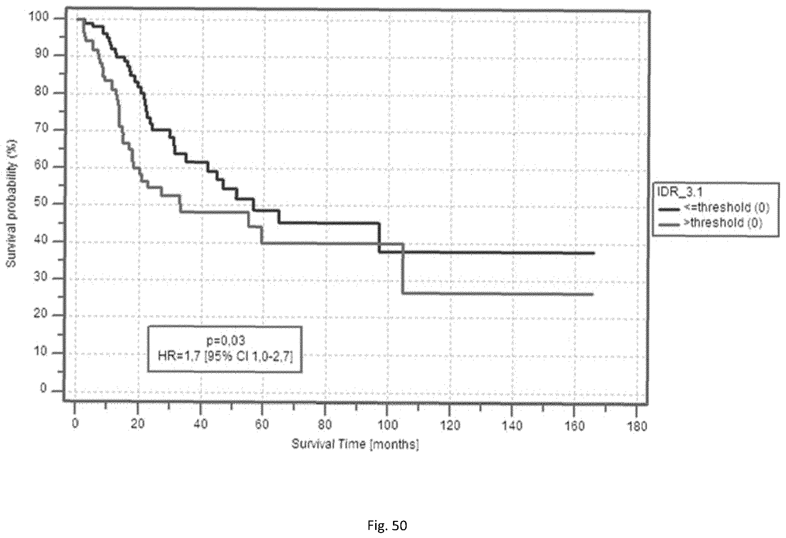

This next section shows additional results for Cox regression models based on a multitude of gene models for both bladder cancer and kidney cancer, comprising randomly selected combinations of at least three genes selected from either immune defense genes (Table 13 & Table 16), TCR signaling genes (Table 14 & Table 17) or PDE4D7 correlated genes (Table 15 & Table 18). Variables and corresponding weights are provided in the Tables 13 - 18. The Cox regression models are plotted in the Kaplan-Meier curve analyses of Figures 50 - 67.

TABLE 13: Variables and weights for three individual Cox regression models of randomized combinations of the immune defense response model (IDR_model) for bladder cancer (BC).

| | | BC IDR_model | | |
|---|---|---|---|---|
| | Variable | 3.1 | 3.2 | 3.3 |
| Immune defense response genes | AIM2 | -0,3067 | - | - |
| | APOBEC3A | - | - | - |
| | CIAO1 | - | -0,2632 | - |
| | DDX58 | - | - | -0,3804 |
| | DHX9 | - | 0,2553 | - |
| | IFI16 | 0,3439 | - | - |

(continued)

| | | BC IDR_model | | |
|---|---|---|---|---|
| | **Variable** | **3.1** | **3.2** | **3.3** |
| | **IFIH1** | - | - | - |
| | **IFIT1** | - | - | 0,3007 |
| | **IFIT3** | - | - | - |
| | **LRRFIP1** | - | 0,1006 | - |
| | **MYD88** | -0,2091 | - | - |
| | **OAS1** | - | - | - |
| | **TLR8** | - | - | 0,1926 |
| | **ZBP1** | - | - | - |

TABLE 14: Variables and weights for three individual Cox regression models of randomized combinations of the T-cell receptor signaling model (TCR_model) for bladder cancer (BC).

| | | BC TCR_model | | |
|---|---|---|---|---|
| | **Variable** | **3.1** | **3.2** | **3.3** |
| **TCR signaling genes** | **CD2** | - | - | - |
| | **CD247** | - | - | 0,2204 |
| | **CD28** | - | 0,09268 | - |
| | **CD3E** | - | - | - |
| | **CD3G** | 0,06752 | - | - |
| | **CD4** | - | - | - |
| | **CSK** | - | - | - |
| | **EZR** | - | 0,02982 | - |
| | **FYN** | - | - | - |
| | **LAT** | -0,1566 | - | - |
| | **LCK** | - | - | 0,223 |
| | **PAG1** | - | - | - |
| | **PDE4D** | 0,2019 | - | - |
| | **PRKACA** | - | 0,1754 | - |
| | **PRKACB** | - | - | - |
| | **PTPRC** | - | - | - |
| | **ZAP70** | - | - | -0,5361 |

TABLE 15: Variables and weights for three individual Cox regression models of randomized combinations of the PDE4D7 model (PDE4D7_model) for bladder cancer (BC).

|  | | BC PDE4D7_model | | |
|---|---|---|---|---|
|  | Variable | 3.1 | 3.2 | 3.3 |
| PDE4D7 correlated genes | ABCC5 | - | -0,062 | - |
|  | CUX2 | -0,3497 | - | - |
|  | KIAA1549 | - | - | 0,0167 |
|  | PDE4D | 0,1715 | 0,1738 | - |
|  | RAP1GAP2 | - | - | -0,024 |
|  | SLC39A11 | -0,1709 | - | -0,1763 |
|  | TDRD1 | - | 0,3135 | - |
|  | VWA2 | - | - | - |

TABLE 16: Variables and weights for three individual Cox regression models of randomized combinations of the immune defense response model (IDR_model) for kidney cancer (KC).

|  | | KC IDR_models | | |
|---|---|---|---|---|
|  | Variable | 3.1 | 3.2 | 3.3 |
| Immune defense response genes | AIM2 | 0,2343 | - | - |
|  | APOBEC3A | - | - | - |
|  | CIAO1 | - | 0,04187 | - |
|  | DDX58 | - | - | -0,1435 |
|  | DHX9 | - | -0,2345 | - |
|  | IFI16 | 0,2761 | - | - |
|  | IFIH1 | - | - | - |
|  | IFIT1 | - | - | -0,09556 |
|  | IFIT3 | - | - | - |
|  | LRRFIP1 | - | 0,1099 | - |
|  | MYD88 | -0,1758 | - | - |
|  | OAS1 | - | - | - |
|  | TLR8 | - | - | 0,001001 |
|  | ZBP1 | - | - | - |

TABLE 17: Variables and weights for three individual Cox regression models of randomized combinations of the T-cell receptor signaling model (TCR_model) for kidney cancer (KC).

|  | | KC TCR_model | | |
|---|---|---|---|---|
|  | Variable | 3.1 | 3.2 | 3.3 |
| TCR signaling genes | CD2 | - | - | - |
|  | CD247 | - | - | -0,09983 |
|  | CD28 | - | 0,1968 | - |

(continued)

| | | KC TCR_model | | |
|---|---|---|---|---|
| | Variable | 3.1 | 3.2 | 3.3 |
| | CD3E | - | - | - |
| | CD3G | -0,3344 | - | - |
| | CD4 | - | - | - |
| | CSK | - | - | - |
| | EZR | - | -0,3355 | - |
| | FYN | - | - | - |
| | LAT | 0,4783 | - | - |
| | LCK | - | - | -0,4485 |
| | PAG1 | - | - | - |
| | PDE4D | -0,1829 | - | - |
| | PRKACA | - | - | - |
| | PRKACB | - | - | - |
| | PTPRC | - | -0,2909 | - |
| | ZAP70 | - | - | 0,746 |

TABLE 18: Variables and weights for three individual Cox regression models of randomized combinations of the PDE4D7 model (PDE4D7_model) for kidney cancer (KC).

| | | KC PDE4D7_model models | | |
|---|---|---|---|---|
| | Variable | 3.1 | 3.2 | 3.3 |
| PDE4D7 correlated genes | ABCC5 | - | 0,1764 | - |
| | CUX2 | -0,1341 | - | - |
| | KIAA1549 | - | -0,3353 | -0,3549 |
| | PDE4D | -0,3597 | - | - |
| | RAP1GAP2 | - | - | -0,0452 |
| | SLC39A11 | 0,1972 | - | - |
| | TDRD1 | - | -0,0038 | -0,0034 |
| | VWA2 | - | - | - |

For Kaplan-Meier curve analysis the Cox regression function of the 18 risk models as shown in Tables 13 - 18 (bladder cancer: IDR 3.1 - 3.3, TCR 3.1 - 3.3 and PDE4D7 3.1 - 3.3; kidney cancer: IDR 3.1 - 3.3, TCR 3.1 - 3.3 and PDE4D7 3.1 - 3.3) were categorized into two sub-cohorts (low risk vs. high risk) based on a cut-off (see description of the figures 50 - 67 below). The threshold for group separation into low and high risk was based on the risk to experience the clinical endpoint (outcome) as predicted by the respective Cox regression model

In Figures 50 - 58 a 189 patient TCGA bladder cancer cohort (total number of patients with non-metastasized disease (m0)) was tested for the endpoint: overall death.

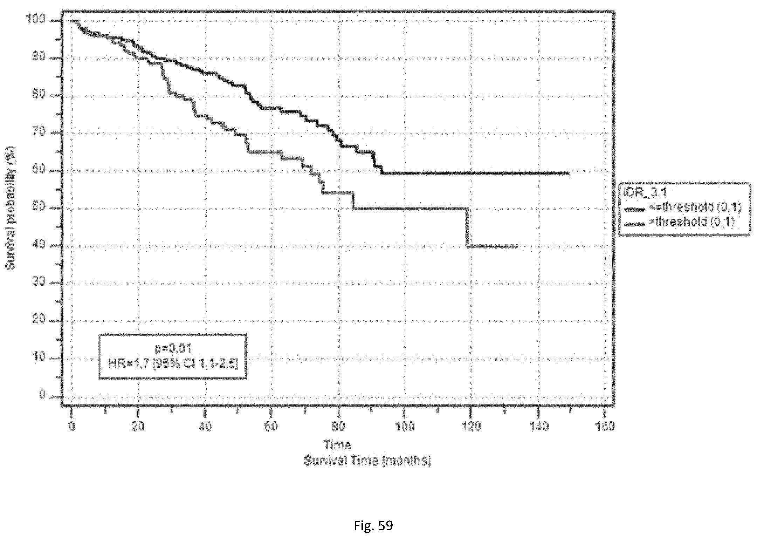

In Figures 59 - 67 a 411 patient TCGA ccRCC kidney cancer cohort (total number of patients with non-metastasized disease (m0)) was tested for the endpoint: overall death.

Figure 50 shows a Kaplan-Meier curve of the IDR_3.1 model in a 189 patient TCGA bladder cancer cohort (total number of patients with non-metastasized disease (m0), comprising both the training set and validation set). The clinical endpoint that was tested was the overall death (logrank p=0.03; HR=1.7; 95% CI=1.0-2.7). The following supplementary lists indicate the number of patients at risk for the IDR_model classes analyzed (threshold=0), i.e., the patients at risk at any time interval +20 months are shown: Low risk (<=0): 103, 54, 26, 16, 7, 5, 3, 2, 1, 0; High risk (>0): 86, 34, 19, 8, 4, 3, 2, 2, 2, 0.

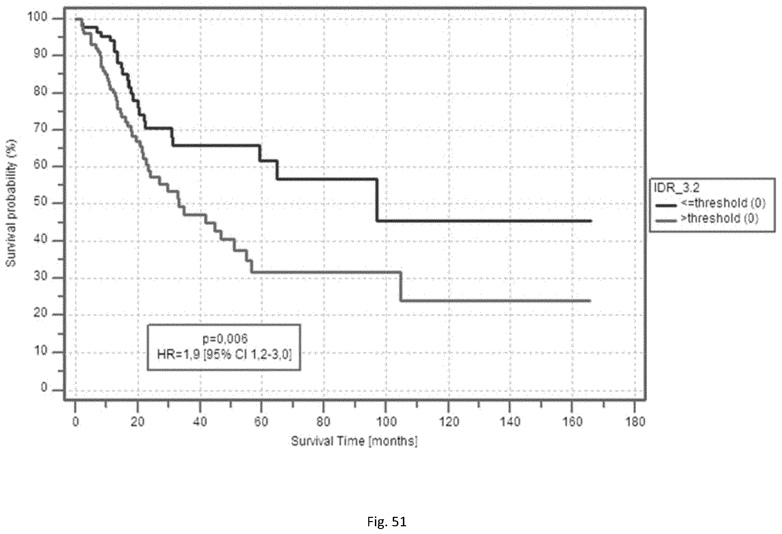

Figure 51 shows a Kaplan-Meier curve of the IDR_3.2 model in a 189 patient TCGA bladder cancer cohort (total number of patients with non-metastasized disease (m0), comprising both the training set and validation set). The clinical endpoint that was tested was the overall death (logrank p=0.006; HR=1.9; 95% CI=1.2-3.0). The following supplementary lists indicate the number of patients at risk for the IDR_model classes analyzed (threshold=0), i.e., the patients at risk at any time interval +20 months are shown: Low risk (<=0): 86, 42, 23, 15, 6, 4, 2, 1, 1, 0; High risk (>0): 103, 46, 22, 9, 5, 4, 3, 3, 2, 0.

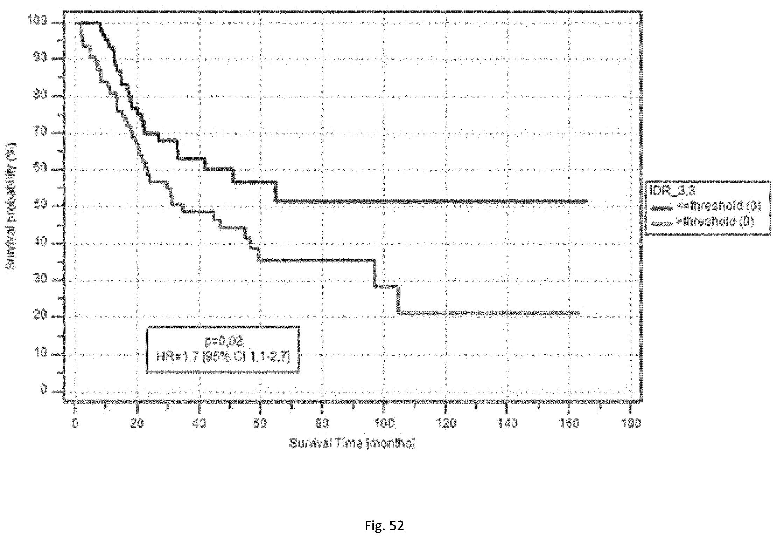

Figure 52 shows a Kaplan-Meier curve of the IDR_3.3 model in a 189 patient TCGA bladder cancer cohort (total number of patients with non-metastasized disease (m0), comprising both the training set and validation set). The clinical endpoint that was tested was the overall death (logrank p=0.02; HR=1.7; 95% CI=1.1-2.7). The following supplementary lists indicate the number of patients at risk for the IDR_model classes analyzed (threshold=0), i.e., the patients at risk at any time interval +20 months are shown: Low risk (<=0): 92, 46, 22, 13, 6, 4, 3, 2, 2, 0; High risk (>0): 97, 42, 23, 11, 5, 4, 2, 2, 1,0.

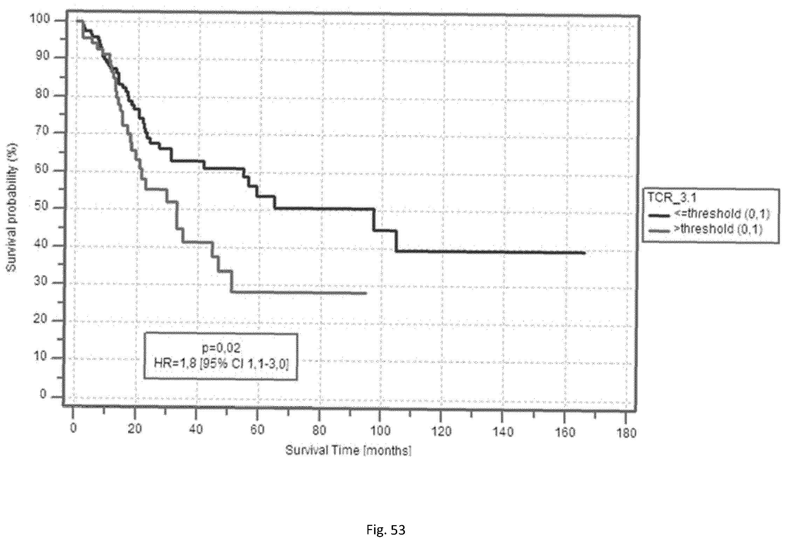

Figure 53 shows a Kaplan-Meier curve of the TCR_3.1 model in a 189 patient TCGA bladder cancer cohort (total number of patients with non-metastasized disease (m0), comprising both the training set and validation set). The clinical endpoint that was tested was the overall death (logrank p=0.02; HR=1.8; 95% CI=1.1-3.0). The following supplementary lists indicate the number of patients at risk for the TCR_model classes analyzed (threshold=0.1), i.e., the patients at risk at any time interval +20 months are shown: Low risk (<=0.1): 120, 62, 34, 20, 9, 8, 5, 4, 3, 0; High risk (>0.1): 69, 26, 11, 4, 2, 0, 0, 0, 0, 0.

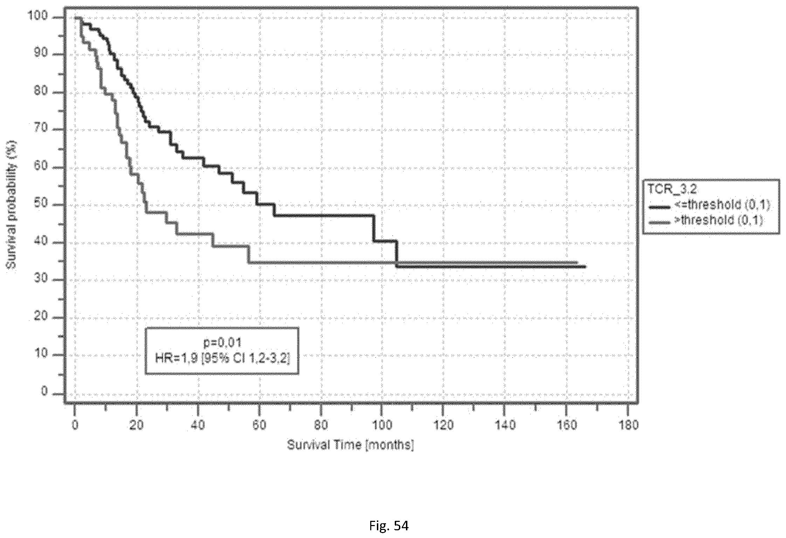

Figure 54 shows a Kaplan-Meier curve of the TCR_3.2 model in a 189 patient TCGA bladder cancer cohort (total number of patients with non-metastasized disease (m0), comprising both the training set and validation set). The clinical endpoint that was tested was the overall death (logrank p=0.01; HR=1.9; 95% CI=1.2-3.2). The following supplementary lists indicate the number of patients at risk for the TCR_model classes analyzed (threshold=0.1), i.e., the patients at risk at any time interval +20 months are shown: Low risk (<=0.1): 129, 64, 32, 17, 9, 6, 3, 2, 2, 0; High risk (>0.1): 60, 24, 13, 7, 2, 2, 2, 2, 1,0.

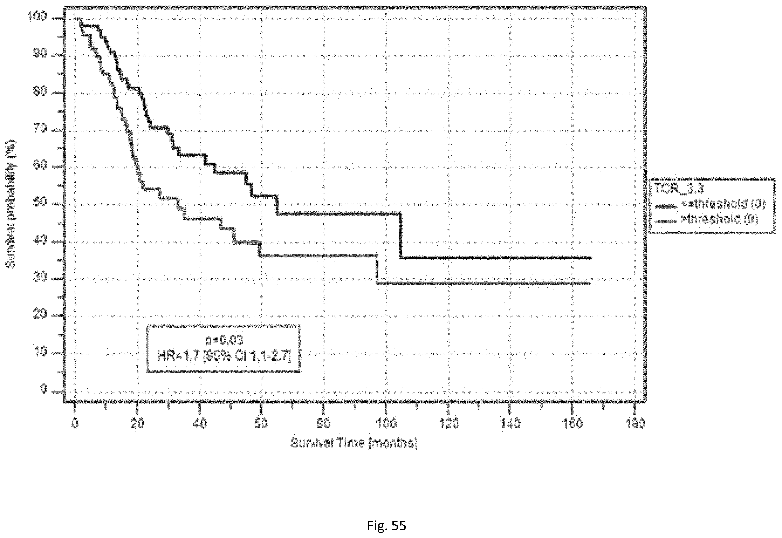

Figure 55 shows a Kaplan-Meier curve of the TCR_3.3 model in a 189 patient TCGA bladder cancer cohort (total number of patients with non-metastasized disease (m0), comprising both the training set and validation set). The clinical endpoint that was tested was the overall death (logrank p=0.03; HR=1.7; 95% CI=1.1-2.7). The following supplementary lists indicate the number of patients at risk for the TCR_model classes analyzed (threshold=0), i.e., the patients at risk at any time interval +20 months are shown: Low risk (<=0): 100, 59, 28, 14, 6, 4, 3, 3, 2, 0; High risk (>0): 89, 29, 17, 10, 5, 4, 2, 1, 1,0.

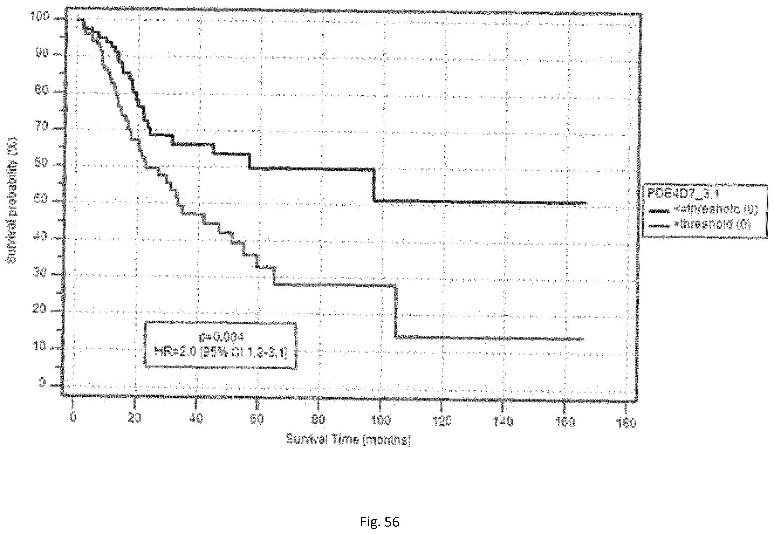

Figure 56 shows a Kaplan-Meier curve of the PDE4D7_3.1 model in a 189 patient TCGA bladder cancer cohort (total number of patients with non-metastasized disease (m0), comprising both the training set and validation set). The clinical endpoint that was tested was the overall death (logrank p=0.004; HR=2.0; 95% CI=1.2-3.1). The following supplementary lists indicate the number of patients at risk for the PDE4D7_model classes analyzed (threshold=0), i.e., the patients at risk at any time interval +20 months are shown: Low risk (<=0): 82, 42, 26, 14, 7, 6, 4, 3, 2, 0; High risk (>0): 107, 46, 19, 10, 4, 2, 1, 1, 1,0.

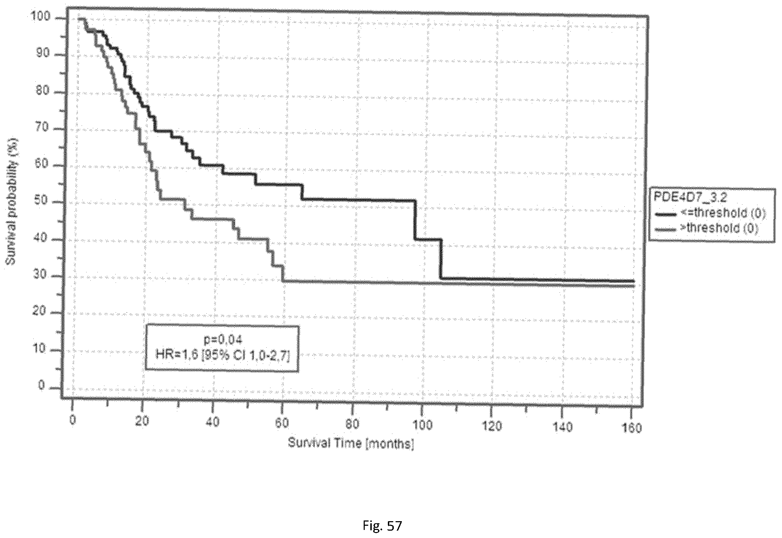

Figure 57 shows a Kaplan-Meier curve of the PDE4D7_3.2 model in a 189 patient TCGA bladder cancer cohort (total number of patients with non-metastasized disease (m0), comprising both the training set and validation set). The clinical endpoint that was tested was the overall death (logrank p=0.04; HR=1.6; 95% CI=1.0-2.7). The following supplementary lists indicate the number of patients at risk for the PDE4D7_model classes analyzed (threshold=0), i.e., the patients at risk at any time interval +20 months are shown: Low risk (<=0): 118, 60, 27, 17, 6, 4, 1, 1, 1; High risk (>0): 71, 28, 18, 7, 5, 4, 4, 3, 2.

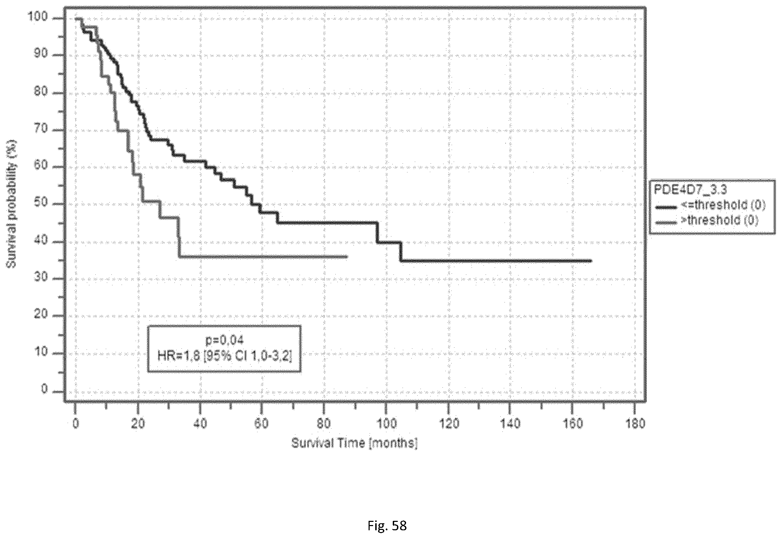

Figure 58 shows a Kaplan-Meier curve of the PDE4D7_3.3 model in a 189 patient TCGA bladder cancer cohort (total number of patients with non-metastasized disease (m0), comprising both the training set and validation set). The clinical endpoint that was tested was the overall death (logrank p=0.04; HR=1.8; 95% CI=1.0-3.2). The following supplementary lists indicate the number of patients at risk for the PDE4D7_model classes analyzed (threshold=0), i.e., the patients at risk at any time interval +20 months are shown: Low risk (<=0): 141, 71, 38, 19, 10, 8, 5, 4, 3, 0; High risk (>0): 48, 17, 7, 5, 1, 0, 0, 0, 0. 0.

Figure 59 shows a Kaplan-Meier curve of the IDR_3.1 model in a 411 patient TCGA ccRCC kidney cancer cohort (total number of patients with non-metastasized disease (m0), comprising both the training set and validation set). The clinical endpoint that was tested was the overall death (logrank p=0.01; HR=1.7; 95% CI=1.1-2.5). The following supplementary lists indicate the number of patients at risk for the IDR_model classes analyzed (threshold=0.1), i.e., the patients at risk at any time interval +20 months are shown: Low risk (<=0.1): 252, 203, 154, 86, 47, 23, 8, 1, 0; High risk (>0.1): 159, 130, 80, 47, 17, 10, 4, 0, 0.

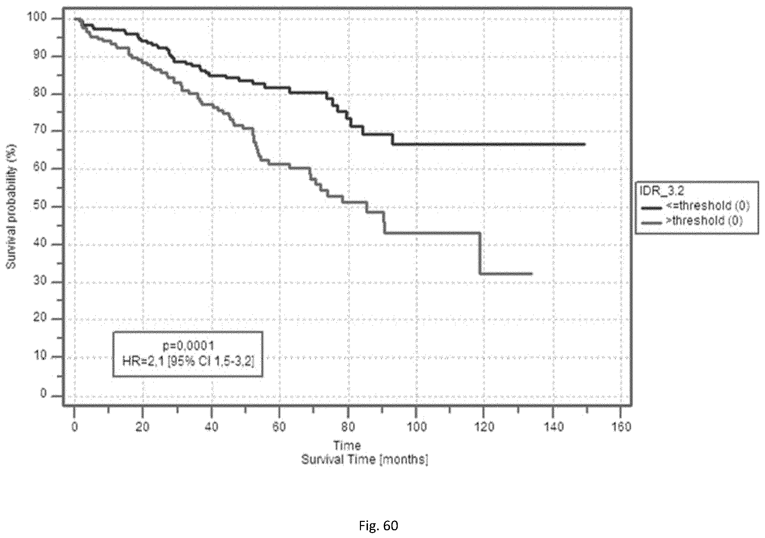

Figure 60 shows a Kaplan-Meier curve of the IDR_3.2 in a 411 patient TCGA ccRCC kidney cancer cohort (total number of patients with non-metastasized disease (m0), comprising both the training set and validation set). The clinical endpoint that was tested was the overall death (logrank p=0.0001; HR=2.1; 95% CI=1.5-3.2). The following supplementary lists indicate the number of patients at risk for the IDR_model classes analyzed (threshold=0), i.e., the patients at risk at any time interval +20 months are shown: Low risk (<=0): 240, 199, 133, 76, 38, 22, 9, 1, 0; High risk (>0): 171, 134, 101, 57, 26, 11, 3, 0, 0, 0.

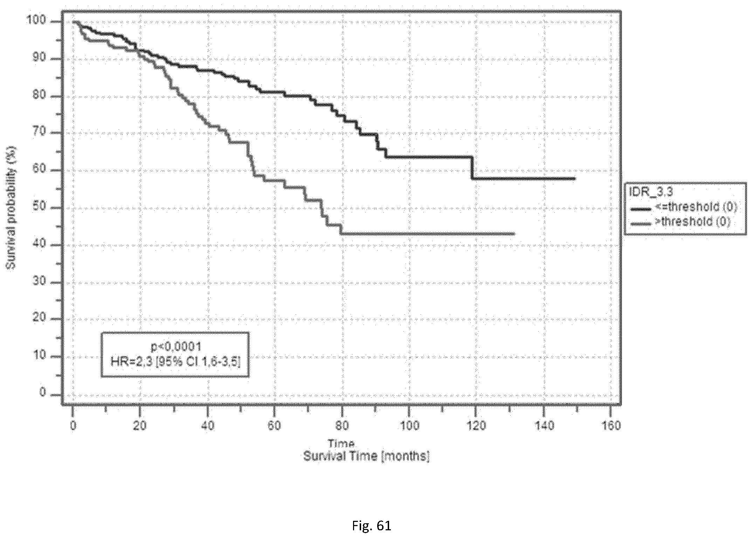

Figure 61 shows a Kaplan-Meier curve of the IDR_3.3 model in a 411 patient TCGA ccRCC kidney cancer cohort (total number of patients with non-metastasized disease (m0), comprising both the training set and validation set). The clinical endpoint that was tested was the overall death (logrank p<0.0001; HR=2.36; 95% CI=1.6-3.5). The following supplementary lists indicate the number of patients at risk for the IDR_model classes analyzed (threshold=0), i.e., the patients at risk at any time interval +20 months are shown: Low risk (<=0): 251, 210, 154, 95, 48, 24, 9, 1, 0; High risk (>0): 160, 123, 80, 38, 16, 9, 3, 0, 0.

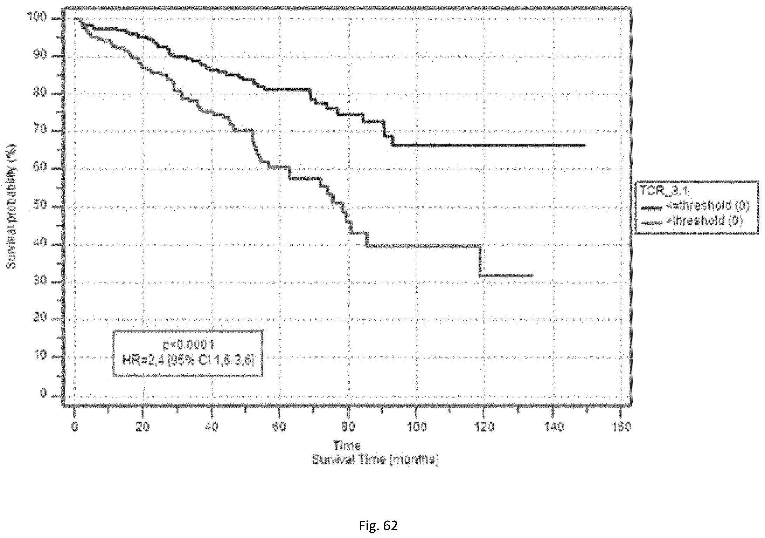

Figure 62 shows a Kaplan-Meier curve of the TCR_3.1 model in a 411 patient TCGA ccRCC kidney cancer cohort (total number of patients with non-metastasized disease (m0), comprising both the training set and validation set). The clinical endpoint that was tested was the overall death (logrank p<0.0001; HR=2.4; 95% CI=1.6-3.6). The following supplementary lists indicate the number of patients at risk for the TCR_model classes analyzed (threshold=0), i.e., the patients at risk at any time interval +20 months are shown: Low risk (<=0): 239, 201, 137, 88, 48, 24, 8, 1, 0; High risk (>0): 172, 132, 97, 45, 16, 9, 4, 0, 0.

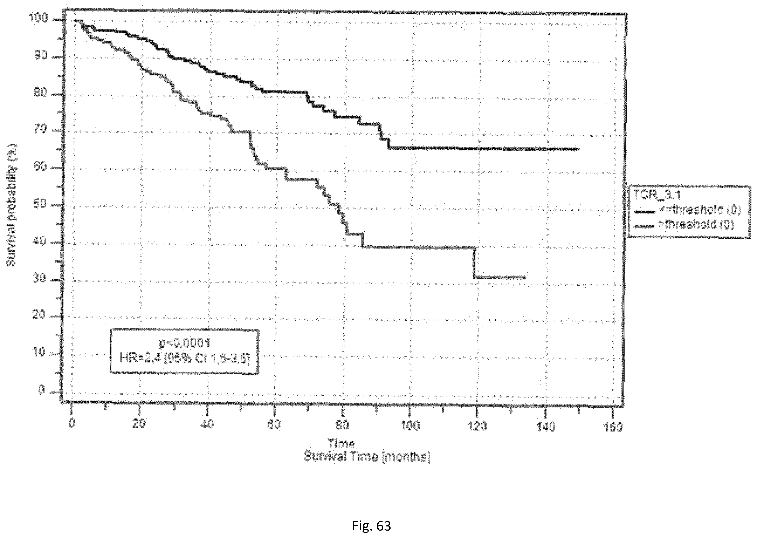

Figure 63 shows a Kaplan-Meier curve of the TCR_3.2 model in a 411 patient TCGA ccRCC kidney cancer cohort (total number of patients with non-metastasized disease (m0), comprising both the training set and validation set). The clinical endpoint that was tested was the overall death (logrank p<0.0001; HR=2.4; 95% CI=1.6-3.6). The following supplementary lists indicate the number of patients at risk for the TCR_model classes analyzed (threshold=0), i.e., the patients at risk at any time interval +20 months are shown: Low risk (<=0): 239, 201, 137, 88, 48, 24, 8, 1, 0; High risk (>0): 172, 132, 97, 45, 16, 9, 4, 0, 0.

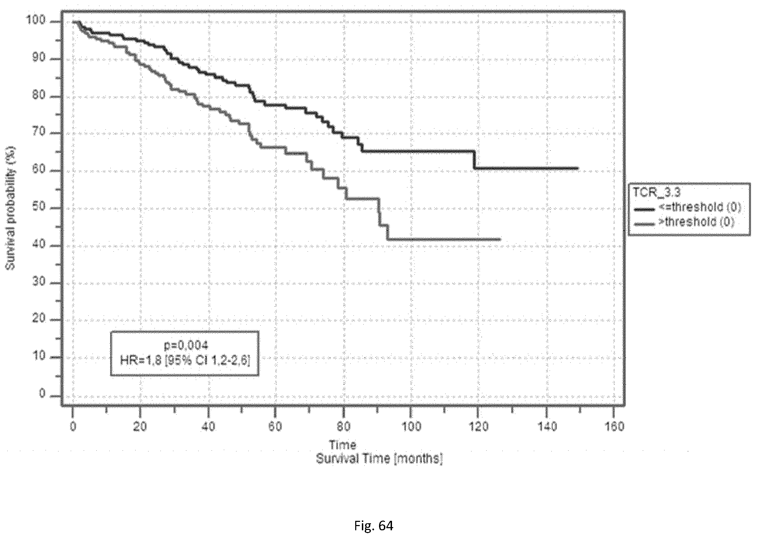

Figure 64 shows a Kaplan-Meier curve of the TCR_3.3 model in a 411 patient TCGA ccRCC kidney cancer cohort (total number of patients with non-metastasized disease (m0), comprising both the training set and validation set). The clinical endpoint that was tested was the overall death (logrank p=0.004; HR=1.8; 95% CI=1.2-2.6). The following supplementary lists indicate the number of patients at risk for the TCR_model classes analyzed (threshold=0), i.e., the patients at risk at any time interval +20 months are shown: Low risk (<=0): 209, 174, 126, 83, 45, 24, 11, 1, 0; High risk (>0): 202, 159, 108, 50, 19, 9, 1 ,0,0.

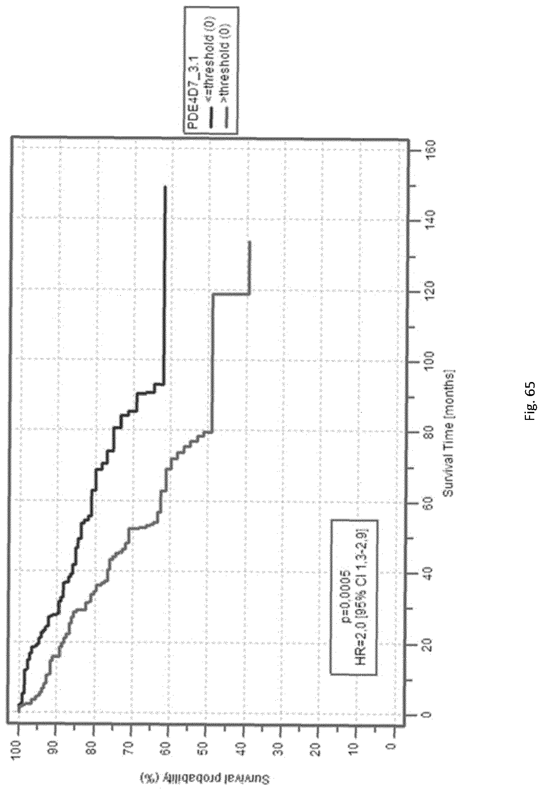

Figure 65 shows a Kaplan-Meier curve of the PDE4D7_3.1 model in a 411 patient TCGA ccRCC kidney cancer cohort (total number of patients with non-metastasized disease (m0), comprising both the training set and validation set). The clinical endpoint that was tested was the overall death (logrank p=0.0005; HR=2.0; 95% CI=1.3-2.9). The following supplementary lists indicate the number of patients at risk for the PDE4D7_model classes analyzed (threshold=0), i.e., the patients at risk at any time interval +20 months are shown: Low risk (<=0): 224, 190, 134, 80, 41, 20, 10, 1, 0; High risk (>0): 187, 143, 100, 53, 23, 13, 2, 0, 0.

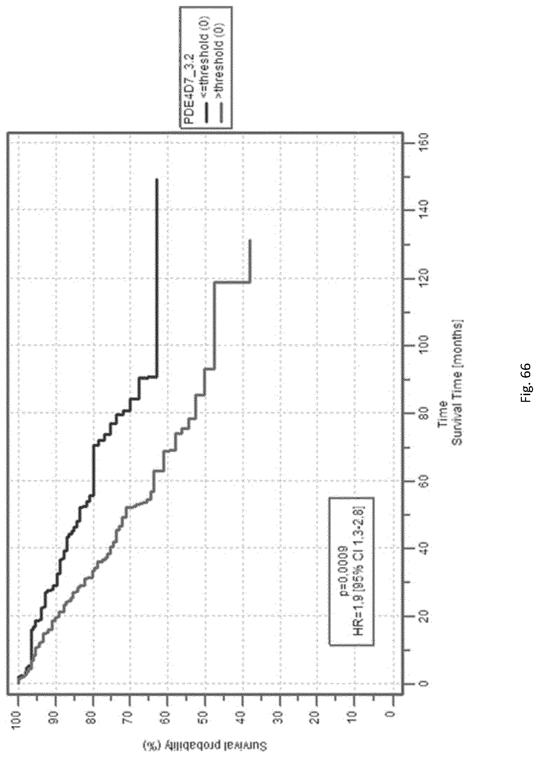

Figure 66 shows a Kaplan-Meier curve of the PDE4D7_3.2 model in a 411 patient TCGA ccRCC kidney cancer cohort (total number of patients with non-metastasized disease (m0), comprising both the training set and validation set). The clinical endpoint that was tested was the overall death (logrank p=0.0009; HR=1.6; 95% CI=1.9-2.8). The following supplementary lists indicate the number of patients at risk for the PDE4D7_model classes analyzed (threshold=0), i.e., the patients at risk at any time interval +20 months are shown: Low risk (<=0): 229, 186, 134, 77, 38, 20, 8, 1, 0; High risk (>0): 182, 147, 100, 56, 26, 13, 4, 0, 0.

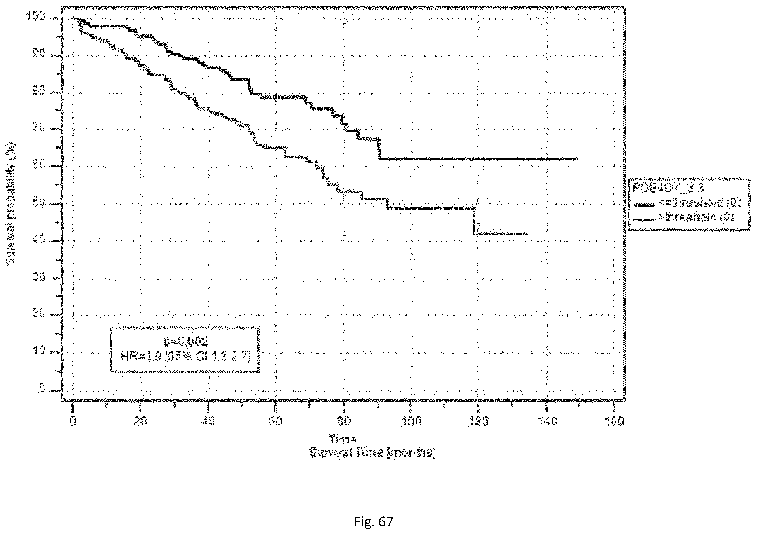

Figure 67 shows a Kaplan-Meier curve of the PDE4D7_3.3 model in a 411 patient TCGA ccRCC kidney cancer cohort (total number of patients with non-metastasized disease (m0), comprising both the training set and validation set). The clinical endpoint that was tested was the overall death (logrank p=0.002; HR=1.9; 95% CI=1.3-2.7). The following supplementary lists indicate the number of patients at risk for the PDE4D7_model classes analyzed (threshold=0), i.e., the patients at risk at any time interval +20 months are shown: Low risk (<=0): 231, 189, 128, 72, 36, 19, 6, 1, 0; High risk (>0): 180, 144, 106, 61, 28, 14, 6, 0, 0.

The Kaplan-Meier survival curve analyses as shown in Figs. 50 to 67 demonstrate the presence of different patient risk groups. The risk group of a patient is determined by the probability to suffer from the respective clinical endpoint (outcome), i.e. overall death as predicted by the respective risk model (see Tables 13 - 18) as shown in the figures. Depending on the predicted risk of a patient (i.e. depending on in which risk group (low or high) the patient may belong) to die from bladder or kidney cancer different types of interventions might be indicated.

In the lower risk groups standard of care (SOC) delivers acceptable long-term oncological control. For the patient group demonstrating a higher risk to eventually die from bladder or kidney cancer treatment escalation (e.g., multi-modality treatments of radiation and chemotherapy) might provide improved longitudinal survival outcomes. Alternative options for treatment escalation adjuvant therapies with radiation of cytotoxic drugs or alternative therapies like immunotherapies (e.g., atezolizumab; pembrolizumab; nivolumab; avelumab; durvalumab) or other experimental therapies.

In other words, the segregation into separate low risk and high risk patient risk groups in bladder cancer or kidney cancer based on any one of the IDR models, any one of the TCR models or any one of the PDE4D7 models as provided herein (Tables 13 - 18) allows for a method to predict the outcome of a bladder cancer subject or kidney cancer subject and allows for prediction of a therapy response.

Discussion

The effectiveness of therapies for primary bladder and kidney cancers is limited, resulting in disease progression and ultimately death of patients, especially for those at high risk of recurrence of disease after primary intervention. The prediction of the therapy outcome is very challenging as many factors play a role in therapy effectiveness and disease recurrence. It is likely that important factors have not yet been identified, while the effect of others cannot be determined precisely. Multiple clinico- pathological measures are currently investigated and applied in a clinical setting to improve response prediction and therapy selection, providing some degree of improvement. Nevertheless, a strong need remains for better prediction of the treatment response in order to increase the success rate of bladder and kidney cancer therapies.

We have identified molecules of which expression shows a significant relation to mortality after primer therapy of bladder and kidney cancers and therefore are expected to improve the prediction of the effectiveness of secondary treatments. This can be achieved by 1) standard of case for those patients with low risk of progressive disease correlated with death from cancer and/or 2) guiding patients with high risk of progressive disease and subsequent death from cancer to an alternative, potentially more effective form of treatment as compared to currently applied standard of care. This would reduce suffering for those patients who would be spared ineffective therapy and would reduce cost spent on ineffective therapies.

Other variations to the disclosed realizations can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

One or more steps of the method illustrated in Fig. 1 may be implemented in a computer program product that may be executed on a computer. The computer program product may comprise a non-transitory computer-readable recording medium on which a control program is recorded (stored), such as a disk, hard drive, or the like. Common forms of non-transitory computer-readable media include, for example, floppy disks, flexible disks, hard disks, magnetic tape, or any other magnetic storage medium, CD-ROM, DVD, or any other optical medium, a RAM, a PROM, an EPROM, a FLASH-EPROM, or other memory chip or cartridge, or any other non-transitory medium from which a computer can read and use.

Alternatively, the one or more steps of the method may be implemented in transitory media, such as a transmittable carrier wave in which the control program is embodied as a data signal using transmission media, such as acoustic or light waves, such as those generated during radio wave and infrared data communications, and the like.

The exemplary method may be implemented on one or more general purpose computers, special purpose computer(s), a programmed microprocessor or microcontroller and peripheral integrated circuit elements, an ASIC or other integrated circuit, a digital signal processor, a hardwired electronic or logic circuit such as a discrete element circuit, a programmable logic device such as a PLD, PLA, FPGA, Graphical card CPU (GPU), or PAL, or the like. In general, any device, capable of implementing a finite state machine that is in turn capable of implementing the flowchart shown in Fig. 1, can be used to implement one or more steps of the method of risk stratification for therapy selection in a patient with prostate cancer is illustrated. As will be appreciated, while the steps of the method may all be computer implemented, in some embodiments one or more of the steps may be at least partially performed manually.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified herein.

Any reference signs in the claims should not be construed as limiting the scope.

The invention relates to a method of predicting an outcome of a bladder or kidney cancer subject, comprising determining or receiving the result of a determination of a first gene expression profile for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, said first gene expression profile(s) being determined in a biological sample obtained from the subject, and/or determining or receiving the result of a determination of a second gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, said second gene expression profile(s) being determined in a biological sample obtained from the subject, and/or determining or receiving the result of a determination of a third gene expression profile for each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, said third gene expression profile(s) being determined in a biological sample obtained from the subject, determining the prediction of outcome based on the first gene expression profile(s), or on the second gene expression profile(s), or on the third gene expression profile(s), or on the first, second, and third gene expression profile(s), and, optionally, providing the prediction to a medical caregiver or the subject.

In some embodiments, the prediction of the outcome can also be determined based on the first gene expression profile(s) and the second genes expression profile(s). In some embodiments, the prediction of the outcome can also be determined based on the first gene expression profile(s) and the third genes expression profile(s). In some embodiments, the prediction of the outcome can also be determined based on the second gene expression profile(s) and the third genes expression profile(s). **The attached Sequence Listing, entitled 2020PF00580_Sequence Listing_ST25 is incorporated herein by reference, in its entirety.**

## Claims

1. A computer implemented method of predicting an outcome of a bladder cancer subject or a kidney cancer subject, comprising:

    - receiving the result of a determination of the gene expression profiles of three or more genes, wherein the three or more genes are selected from:

        - immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, or
        - T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, or
        - PDE4D7 correlated genes selected from the group consisting of: ABCC5, CUX2, KIAA1549, PDE4D, RAP1GAP2, SLC39A11, TDRD1, and VWA2, or
        - wherein said three or more genes comprise at least one or more gene(s) selected from each of the immune defense response genes, T-Cell receptor signaling genes and PDE4D7 correlated genes,

    - wherein the gene expression profiles of the three or more genes are determined in a biological sample obtained from the subject, and
    - determining the prediction of the outcome based on the gene expression profiles of the three or more genes, and
    - optionally, providing the prediction to a medical caregiver or the subject.

2. A method of predicting an outcome of a bladder cancer subject or a kidney cancer subject, comprising:

    - determining the result of a determination of the gene expression profiles of three or more genes, wherein the three or more genes are selected from:

        - immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, or
        - T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, or
        - PDE4D7 correlated genes selected from the group consisting of: ABCC5, CUX2, KIAA1549, PDE4D, RAP1GAP2, SLC39A11, TDRD1, and VWA2, or
        - wherein said three or more genes comprise at least one or more gene(s) selected from each of the immune defense response genes, T-Cell receptor signaling genes and PDE4D7 correlated genes,

    - wherein the gene expression profiles of the three or more genes are determined in a biological sample obtained from the subject, and
    - determining the prediction of the outcome based on the gene expression profiles of the three or more genes, and
    - optionally, providing the prediction to a medical caregiver or the subject.

3. The method as defined in claim 1 or 2, wherein:

    - the three or more immune defense response genes comprise six or more, preferably, nine or more, more preferably all of the immune defense genes, and/or
    - the three or more T-Cell receptor signaling genes comprise six or more, preferably, nine or more, more preferably, all of the T-Cell receptor signaling genes, and/or
- the three or more PDE4D7 correlated genes comprise six or more, preferably, all of the PDE4D7 correlated genes, or
- the three or more genes comprise at least two or more genes selected from each of the immune defense response genes, T-Cell receptor signaling genes and PDE4D7 correlated genes, preferably three or more genes selected from each of the immune defense response genes, T-Cell receptor signaling genes and PDE4D7 correlated genes, more preferably all of the genes selected from each of the immune defense response genes, T-Cell receptor signaling genes and PDE4D7 correlated genes, preferablywherein the determining of the prediction of the outcome comprises:
- combining the gene expression profiles for two or more, for example, 2, 3, 4, 5, 6, 7, 8, 9, 10, 11, 12, 13 or all, of the immune defense response genes with a regression function that had been derived from a population of bladder or kidney cancer subjects, and/or
- combining the gene expression profiles for two or more, for example, 2, 3, 4, 5, 6, 7, 8, 9, 10, 11, 12, 13, 14, 15, 16 or all, of the T-Cell receptor signaling genes with a regression function that had been derived from a population of bladder or kidney cancer subjects, and/or
- combining the gene expression profiles for two or more, for example, 2, 3, 4, 5, 6, 7 or all, of the PDE4D7 correlated genes with a regression function that had been derived from a population of bladder or kidney cancer subjects.

4. The method as defined in claim 3, wherein the determining of the prediction of the outcome further comprises combining the combination of the gene expression profiles with a regression function that had been derived from a population of bladder or kidney cancer subjects.

5. The method as defined in any of claims 1 to 4, wherein the determining of the outcome is further based on one or more clinical parameters obtained from the subject,

   preferably wherein the clinical parameters comprise one or more of: (i) T stage attribute (T1, T2, T3, or T4); (ii) N stage attribute (N0, N1, N2, or N3), and; (iii) M stage attribute (M0, M1),
   more preferably wherein the determining of the prediction of outcome comprises combining one or more of the gene expression profile(s) of: (i) the three immune defense response genes; (ii) the three T-Cell receptor signaling genes; (iii) the three PDE4D7 correlated genes, and; (iv) the combination of one or more immune defense response genes, one or more T-Cell receptor signaling genes and one or more PDE4D7 correlated genes, and the one or more clinical parameters obtained from the subject with a regression function that had been derived from a population of bladder or kidney cancer subjects.

6. The method as defined in any of claims 1 to 5, wherein the biological sample(s) is/are obtained from the subject before the start of the therapy.

7. The method as defined in any of claims 1 to 6, wherein the therapy is surgery, radiotherapy, cytotoxic chemotherapy (CTX), or immunotherapy.

8. The method as defined in any of claims 1 to 7, wherein the prediction of the therapy response is negative or positive for the effectiveness of the therapy, wherein a therapy is recommended based on the prediction and, if the prediction is negative, the recommended therapy comprises one or more of: (i) therapy provided earlier than is the standard; (ii) radiotherapy with an increased effective dose; (iii) an adjuvant therapy, such a chemotherapy; and (iv) an alternative therapy, such as immunotherapy.

9. A computer program product comprising instructions which, when the program is executed by a computer, cause the computer to carry out a method comprising:

   - receiving data indicative of the gene expression profiles of three or more genes, wherein the three or more genes are selected from:

      - immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, or
      - T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, or
      - PDE4D7 correlated genes selected from the group consisting of: ABCC5, CUX2, KIAA1549, PDE4D, RAP1GAP2, SLC39A11, TDRD1, and VWA2, or

- wherein said three or more genes comprise at least one or more gene(s) selected from each of the immune defense response genes, T-Cell receptor signaling genes and PDE4D7 correlated genes, and

- wherein the gene expression profile(s) are being determined in a biological sample obtained from a bladder or kidney cancer subject
- determining the prediction of the outcome based on the gene expression profiles of the three or more genes, and
- optionally, providing the prediction to a medical caregiver or the subject.

10. An apparatus for predicting an outcome of a bladder or kidney cancer subject, comprising:

- an input adapted to receive data indicative of the gene expression profiles of three or more genes, wherein the three or more genes are selected from:

- immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, or
- T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, or
- PDE4D7 correlated genes selected from the group consisting of: ABCC5, CUX2, KIAA1549, PDE4D, RAP1GAP2, SLC39A11, TDRD1, and VWA2, or
- wherein said three or more genes comprise at least one or more gene(s) selected from each of the immune defense response genes, T-Cell receptor signaling genes and PDE4D7 correlated genes;

- wherein the gene expression profile(s) are being determined in a biological sample obtained from a bladder or kidney cancer subject
- a processor adapted to determine the prediction of outcome based on the gene expression profiles of the three or more genes,
- a computer program product according to claim 9, and
- optionally, a providing unit adapted to provide the prediction to a medical caregiver or the subject.

11. A diagnostic kit, comprising:

- at least three primers and probes for determining the gene expression profiles of three or more genes, wherein the three or more genes are selected from:

- immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, or
- T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, or
- PDE4D7 correlated genes selected from the group consisting of: ABCC5, CUX2, KIAA1549, PDE4D, RAP1GAP2, SLC39A11, TDRD1, and VWA2, or
- wherein said three or more genes comprise at least one or more gene(s) selected from each of the immune defense response genes, T-Cell receptor signaling genes and PDE4D7 correlated genes, and

- wherein the gene expression profile(s) are being determined in a biological sample obtained from a bladder or kidney cancer subject
- optionally, an apparatus as defined in claim 10 or a computer program product as defined in claim 9.

12. Use of the kit as defined in claim 11.

13. The use as defined in claim 12 in a method of predicting an outcome of a bladder cancer subject or a kidney cancer subject.

14. A method, comprising:

- receiving one or more biological sample(s) obtained from a bladder cancer subject or a kidney cancer subject,
- using the kit as defined in claim 13 to determine the gene expression profiles of three or more genes, wherein the three or more genes are selected from:

- immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, or
- T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, or
- PDE4D7 correlated genes selected from the group consisting of: ABCC5, CUX2, KIAA1549, PDE4D, RAP1GAP2, SLC39A11, TDRD1, and VWA2 or
- wherein said three or more genes comprise at least one or more gene(s) selected from each of the immune defense response genes, T-Cell receptor signaling genes and PDE4D7 correlated genes.

15. Use of a computer program product according to claim 11 or an apparatus according to claim 12 for using the gene expression profiles of three or more genes, wherein the three or more genes are selected from:

- immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, or
- T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, or
- PDE4D7 correlated genes selected from the group consisting of: ABCC5, CUX2, KIAA1549, PDE4D, RAP1GAP2, SLC39A11, TDRD1, and VWA2, or
- wherein said three or more genes comprise at least one or more gene(s) selected from each of the immune defense response genes, T-Cell receptor signaling genes and PDE4D7 correlated genes,

in a method of predicting an outcome of a bladder or kidney cancer subject, comprising:

- determining the prediction of the outcome based on the gene expression profiles of the three or more genes, and
- optionally, providing the prediction to a medical caregiver or the subject.

**Patentansprüche**

1. Ein durch Computer verwirklichtes Verfahren zur Vorhersage der Ausgangs eines Blasenkrebses oder Nierenkrebses, umfassend:

- empfangen des Ergebnisses einer Bestimmung der Genexpressionsprofile von drei oder mehr Genen, wobei die drei oder mehr Gene ausgewählt werden aus:
- Immunabwehrreaktionsgenen, ausgewählt aus der Gruppe bestehend aus: AIM2, APOBEC3A, CIAO1, DDX58, DHX9, IFI16, IFIH1, IFIT1, IFIT3, LRRFIP1, MYD88, OAS1, TLR8 und ZBP1, oder
- T-Zell-Rezeptor-Signalgenen, ausgewählt aus der Gruppe bestehend aus: CD2, CD247, CD28, CD3E, CD3G, CD4, CSK, EZR, FYN, LAT, LCK, PAG1, PDE4D, PRKACA, PRKACB, PTPRC und ZAP70 oder
- PDE4D7-korrelierten Genen, ausgewählt aus der Gruppe bestehend aus: ABCC5, CUX2, KIAA1549, PDE4D, RAP1GAP2, SLC39A11, TDRD1 und VWA2 oder
- wobei die drei oder mehr Gene mindestens eines oder mehrere Gene umfassen, die aus jedem von Immunabwehrreaktionsgenen, T-Zell-Rezeptor-Signalgenen und PDE4D7-korrelierten Genen ausgewählt wurden,
- wobei die Genexpressionsprofile der drei oder mehr Gene in einer biologischen Probe des Patienten bestimmt werden, und
- Bestimmung der Vorhersage des Ausgangs auf der Grundlage der Genexpressionsprofile der drei oder mehr Gene, und
- auf Wunsch die Vorhersage an einen medizinischen Betreuer oder den Patienten.

2. Verfahren zur Vorhersage der Weiterentwicklung eines Patienten mit Blasenkrebs oder eines Patienten mit Nierenkrebs, umfassend:

- Bestimmung des Ergebnisses einer Bestimmung der Genexpressionsprofile von drei oder mehr Genen, wobei die drei oder mehr Gene ausgewählt werden aus:

- Immunabwehrreaktionsgenen, ausgewählt aus der Gruppe bestehend aus: AIM2, APOBEC3A, CIAO1, DDX58, DHX9, IFI16, IFIH1, IFIT1, IFIT3, LRRFIP1, MYD88, OAS1, TLR8 und ZBP1, oder
- T-Zell-Rezeptor-Signalgenen, ausgewählt aus der Gruppe bestehend aus: CD2, CD247, CD28, CD3E, CD3G, CD4, CSK, EZR, FYN, LAT, LCK, PAG1, PDE4D, PRKACA, PRKACB, PTPRC und ZAP70, oder

- PDE4D7-korrelierten Genen, ausgewählt aus der Gruppe bestehend aus: ABCC5, CUX2, KIAA1549, PDE4D, RAP1GAP2, SLC39A11, TDRD1, und VWA2, oder
- wobei die drei oder mehr Gene mindestens ein oder mehrere Gene umfassen, die jeweils aus Immunabwehrreaktionsgenen, T-Zell-Rezeptor-Signalgenen und PDE4D7-korrelierten Genen ausgewählt wurden,
- wobei die Genexpression der drei oder mehr Gene in einer biologischen Probe des Patienten bestimmt wird, und
- Bestimmung der Vorhersage des Ausgangs auf der Grundlage der Genexpressions-Profile der drei oder mehr Gene, und
- auf Wunsch die Weitergabe der Vorhersage an einen medizinischen Betreuer oder den Patienten.

3. Verfahren nach Anspruch 1 oder 2, wobei:

- die drei oder mehr Immunabwehrreaktionsgene sechs oder mehr, vorzugsweise neun oder mehr, besonders bevorzugt alle Immunabwehrgene umfassen, und/oder
- die drei oder mehr T-Zell-Rezeptor-Signalgene sechs oder mehr, vorzugsweise neun oder mehr, besonders bevorzugt alle T-Zell-Rezeptor-Signalgene umfassen, und/oder
- die drei oder mehr PDE4D7-korrelierten Gene sechs oder mehr, vorzugsweise alle PDE4D7-korrelierten Gene umfassen, oder
- die drei oder mehr Gene mindestens zwei oder mehr Gene, die aus jedem von den Immunabwehrreaktionsgenen, T-Zell-Rezeptor-Signalgenen und PDE4D7-korrelierten Genen ausgewählt wurden, umfassen, vorzugsweise drei oder mehr Gene, die aus jedem der Immunabwehrreaktionsgene ausgewählt wurden; T-Zell-Rezeptor-Signalgene und PDE4D7-korrelierte Gene, besonders bevorzugt alle Gene, die aus jedem der Immunabwehrreaktion-Gene ausgewählt wurden, T-Zell-Rezeptor-Signalgene und PDE4D7-korrelierte Gene, vorzugsweise wobei die Bestimmung der Vorhersage des Ausgangs umfasst:
- Kombination der Genexpressionsprofile für zwei oder mehr, z.B. 2, 3, 4, 5, 6, 7, 8, 9, 10, 11, 12, 13 oder alle Gene der Immunabwehrreaktion mit einer Regressionsfunktion, die aus einer Zielgruppe von Patienten mit Blasen- oder Nierenkrebs abgeleitet worden waren, und/oder
- Kombination der Genexpressionsprofile für zwei oder mehr, z.B. 2, 3, 4, 5, 6, 7, 8, 9, 10, 11, 12, 13, 14, 15, 16 oder alle T-Zell-Rezeptor-Signalgene mit einer Regressionsfunktion, die aus einer Zielgruppe von Patienten mit Blasen- oder Nierenkrebs abgeleitet worden waren, und/oder
- Kombination der Genexpressionsprofile für zwei oder mehr, z.B. 2, 3, 4, 5, 6, 7 oder alle der PDE4D7-korrelierten Gene mit einer Regressionsfunktion, die aus einer Zielgruppe von Patienten mit Blasen- oder Nierenkrebs abgeleitet worden waren.

4. Verfahren nach Anspruch 3, **dadurch gekennzeichnet, dass** die Bestimmung der Vorhersage des Ausgangs weiterhin die Kombination der Genexpressionsprofile mit einer Regressionsfunktion umfasst, die aus einer Zielgruppe von Patienten mit Blase- oder Nierenkrebs abgeleitet worden waren.

5. Verfahren nach einem der Ansprüche 1 bis 4, wobei die Bestimmung des Ausgangs weiterhin auf einem oder mehreren klinischen Parametern basiert, die vom Patienten erhalten wurden, vorzugsweise wobei die klinischen Parameter eines oder mehrere der folgenden Elemente umfassen: (i) T-Stadium-Attribut (T1, T2, T3 oder T4); (ii) N-Stadium-Attribut (N0, N1, N2 oder N3) und (iii) M-Stadium-Attribut (M0, M1), besonders bevorzugt, wobei die Bestimmung der Vorhersage des Ausgangs die Kombination eines oder mehrerer Genexpressionsprofile der Folgenden umfasst: (i) die drei Gene der Immunabwehr; (ii) die drei T-Zell-Rezeptor-Signalgene; (iii) die drei PDE4D7-korrelierten Gene und (iv) die Kombination eines oder mehrerer Gene der Immunabwehr, eines oder mehrerer T-Zell-Rezeptor-Signalgene und eines oder mehrerer PDE4D7-korrelierter Gene, und den einem oder mehreren klinischen Parametern, die von dem Patienten mit einer Regressionsfunktion ermittelt wurden, die aus einer Zielgruppe von Patienten mit Blasen- oder Nierenkrebs abgeleitet wurden.

6. Verfahren nach einem der Ansprüche 1 bis 5, wobei die biologischen Proben vor Beginn der Therapie vom Patienten gewonnen werden.

7. Verfahren nach einem der Ansprüche 1 bis 6, **dadurch gekennzeichnet, dass** die Therapie Chirurgie, Strahlentherapie, zytotoxische Chemotherapie (CTX) oder Immuntherapie ist.

8. Verfahren nach einem der Ansprüche 1 bis 7, wobei die Vorhersage des Ansprechens auf die Therapie negativ oder positiv für die Wirksamkeit der Therapie ist, wobei eine Therapie basierend auf der Vorhersage empfohlen wird und, wenn die Vorhersage negativ ist, die empfohlene Therapie eine oder mehrere der folgenden umfasst: (i) Therapie, die früher durchgeführt wird als die Standardtherapie; (ii) Strahlentherapie mit erhöhter wirksamer Dosis; (iii) eine unterstützende Therapie, wie eine Chemotherapie; und (iv) eine alternative Therapie, wie eine Immuntherapie.

9. Ein Computerprogrammprodukt, das Anweisungen enthält, die, wenn das Programm von einem Computer ausgeführt wird, den Computer veranlassen, ein Verfahren auszuführen, das Folgendes umfasst:

- Empfang von Daten, die auf die Genexpression von drei oder mehr Genen hinweisen, wobei die drei oder mehr Gene ausgewählt werden aus:
- Immunabwehrreaktionsgenen, ausgewählt aus der Gruppe, bestehend aus: AIM2, APOBEC3A, CIAO1, DDX58, DHX9, IFI16, IFIH1, IFIT1, IFIT3, LRRFIP1, MYD88, OAS1, TLR8 und ZBP1, oder
- T-Zell-Rezeptor-Signalgenen, ausgewählt aus der Gruppe, bestehend aus: CD2, CD247, CD28, CD3E, CD3G, CD4, CSK, EZR, FYN, LAT, LCK, PAG1, PDE4D, PRKACA, PRKACB, PTPRC und ZAP70, oder
- PDE4D7-korrelierten Genen, ausgewählt aus der Gruppe bestehend aus: ABCC5, CUX2, KIAA1549, PDE4D, RAP1GAP2, SLC39A11, TDRD1 und VWA2 oder
- wobei die drei oder mehr Gene mindestens eines oder mehrere Gene umfassen, die aus jedem von den Immunabwehrreaktionsgenen, T-Zell-Rezeptor-Signalgenen und PDE4D7-korrelierten Genen ausgewählt wurden, und
- wobei das oder die Genexpressions-Profile in einer biologischen Probe bestimmt werden, die von einem Patienten mit Blasen- oder Nierenkrebs gewonnen wurde
- Bestimmung der Vorhersage des Ausgangs auf der Grundlage der Genexpressions-Profile der drei oder mehr Gene und
- auf Wunsch, die Weitergabe der Vorhersage an einen medizinischen Betreuer oder den Patienten.

10. Ein Gerät zur Vorhersage des Ausgangs eines Blasen- oder Nierenkrebses, umfassend:

- eine Eingabe, die für den Empfang von Daten geeignet ist, die auf die Genexpression von drei oder mehr Genen hinweisen, wobei die drei oder mehr Gene ausgewählt werden aus:
- Immunabwehrreaktionsgenen, ausgewählt aus der Gruppe, bestehend aus: AIM2, APOBEC3A, CIAO1, DDX58, DHX9, IFI16, IFIH1, IFIT1, IFIT3, LRRFIP1, MYD88, OAS1, TLR8 und ZBP1, oder
- T-Zell-Rezeptor-Signalgenen, ausgewählt aus der Gruppe, bestehend aus: CD2, CD247, CD28, CD3E, CD3G, CD4, CSK, EZR, FYN, LAT, LCK, PAG1, PDE4D, PRKACA, PRKACB, PTPRC und ZAP70, oder
- PDE4D7-korrelierten Genen ausgewählt aus der Gruppe bestehend aus: ABCC5, CUX2, KIAA1549, PDE4D, RAP1GAP2, SLC39A11, TDRD1 und VWA2 oder
- wobei die drei oder mehr Gene mindestens eines oder mehrere Gene umfassen, die aus jedem der Immunabwehrreaktionsgene, T-Zell-Rezeptor-Signalgene und PDE4D7-korrelierter Gene ausgewählt wurden;
- wobei das oder die Genexpressions-Profile in einer biologischen Probe bestimmt werden, die von einem Patienten mit Blasen- oder Nierenkrebs gewonnen wurde
- ein Prozessor, der die Vorhersage des Ausgangs anhand der Genexpressions-Profile der drei oder mehr Gene bestimmt,
- ein Computerprogrammprodukt nach Anspruch 9, und
- auf Wunsch eine bereitstellende Einheit, die angepasst ist für die Weitergabe der Vorhersage an einen medizinischen Betreuer oder Patienten.

11. Ein Diagnose-Kit, umfassend:

- mindestens drei Primer und Sonden zur Bestimmung der Genexpressions-Profile von drei oder mehr Genen, wobei die drei oder mehr Gene ausgewählt werden aus:
- Immunabwehrreaktionsgenen, ausgewählt aus der Gruppe, bestehend aus: AIM2, APOBEC3A, CIAO1, DDX58, DHX9, IFI16, IFIH1, IFIT1, IFIT3, LRRFIP1, MYD88, OAS1, TLR8 und ZBP1, oder
- T-Zell-Rezeptor-Signalgenen, ausgewählt aus der Gruppe, bestehend aus: CD2, CD247, CD28, CD3E, CD3G, CD4, CSK, EZR, FYN, LAT, LCK, PAG1, PDE4D, PRKACA, PRKACB, PTPRC und ZAP70, oder
- PDE4D7-korrelierten Genen ausgewählt aus der Gruppe bestehend aus: ABCC5, CUX2, KIAA1549, PDE4D, RAP1GAP2, SLC39A11, TDRD1 und VWA2 oder
- wobei die drei oder mehr Gene mindestens eines oder mehrere Gene umfassen, die aus jedem von den Immunabwehrreaktionsgenen, T-Zell-Rezeptor-Signalgenen und PDE4D7-korrelierten Genen ausgewählt wurden, und
- wobei das oder die Genexpressions-Profile in einer biologischen Probe bestimmt werden, die von einem Patienten mit Blasen- oder Nierenkrebs gewonnen wurde
- auf Wunsch ein Gerät gemäß Anspruch 10 oder ein Computerprogrammprodukt gemäß Anspruch 9.

**12.** Verwendung des Kits gemäß Anspruch 11.

**13.** Verwendung nach Anspruch 12 bei einem Verfahren zur Vorhersage eines Ausgangs eines Blasenkrebses oder eines Nierenkrebses.

**14.** Ein Verfahren, das Folgendes umfasst:

- erhalten einer oder mehrerer biologischer Proben von einem Patienten mit Blasenkrebs oder Nierenkrebs,
- Verwendung des Kits nach Anspruch 13 zur Bestimmung der Genexpressionsprofile von drei oder mehr Genen, wobei die drei oder mehr Gene ausgewählt sind aus:
- Immunabwehrreaktionsgenen, ausgewählt aus der Gruppe, bestehend aus: AIM2, APOBEC3A, CIAO1, DDX58, DHX9, IFI16, IFIH1, IFIT1, IFIT3, LRRFIP1, MYD88, OAS1, TLR8 und ZBP1, oder
- T-Zell-Rezeptor-Signalgenen, ausgewählt aus der Gruppe, bestehend aus: CD2, CD247, CD28, CD3E, CD3G, CD4, CSK, EZR, FYN, LAT, LCK, PAG1, PDE4D, PRKACA, PRKACB, PTPRC und ZAP70, oder
- PDE4D7-korrelierten Genen ausgewählt aus der Gruppe bestehend aus: ABCC5, CUX2, KIAA1549, PDE4D, RAP1GAP2, SLC39A11, TDRD1 und VWA2 oder
- wobei die drei oder mehr Gene mindestens ein oder mehrere Gene umfassen, die aus jedem der Immunabwehrreaktionsgene, T-Zell-Rezeptor-Signalgene und PDE4D7-korrelierter Gene ausgewählt wurden.

**15.** Verwendung eines Computerprogrammprodukts nach Anspruch 11 oder einer Vorrichtung nach Anspruch 12 zur Verwendung der Genexpressionsprofile von drei oder mehr Genen, wobei die drei oder mehr Gene ausgewählt sind aus:

- Immunabwehrreaktionsgenen, ausgewählt aus der Gruppe, bestehend aus: AIM2, APOBEC3A, CIAO1, DDX58, DHX9, IFI16, IFIH1, IFIT1, IFIT3, LRRFIP1, MYD88, OAS1, TLR8 und ZBP1, oder
- T-Zell-Rezeptor-Signalgenen, ausgewählt aus der Gruppe, bestehend aus: CD2, CD247, CD28, CD3E, CD3G, CD4, CSK, EZR, FYN, LAT, LCK, PAG1, PDE4D, PRKACA, PRKACB, PTPRC und ZAP70, oder
- PDE4D7-korrelierten Genen, ausgewählt aus der Gruppe bestehend aus: ABCC5, CUX2, KIAA1549, PDE4D, RAP1GAP2, SLC39A11, TDRD1, und VWA2, oder
- wobei die drei oder mehr Gene mindestens ein oder mehrere Gene umfassen, die aus jedem der Immunabwehrreaktionsgene, T-Zell-Rezeptor-Signalgene und PDE4D7-korrelierter Gene ausgewählt wurden, in einem Verfahren zur Vorhersage eines Ausgangs eines Blasen- oder Nierenkrebses, umfassend:
- Bestimmung der Vorhersage des Ausgangs auf der Grundlage der Genexpressions-Profile der drei oder mehr Gene und
- auf Wunsch die Vorhersage an einen medizinischen Betreuer oder den Patienten.

**Revendications**

**1.** Méthode informatisée de prédiction du résultat pour un sujet atteint d'un cancer de la vessie ou d'un cancer du rein comprenant:

- recevoir le résultat d'une détermination des profils d'expression génique de trois gènes ou plus, dans laquelle les trois gènes ou plus sont sélectionnés parmi:

- des gènes de réponse immunitaire choisis dans le groupe constitué par: AIM2, APOBEC3A, CIAO1, DDX58, DHX9, IFI16, IFIH1, IFIT1, IFIT3, LRRFIP1, MYD88, OAS1, TLR8, et ZBP1, ou
- gènes de signalisation des récepteurs des cellules T choisis dans le groupe constitué par: CD2, CD247, CD28, CD3E, CD3G, CD4, CSK, EZR, FYN, LAT, LCK, PAG1, PDE4D, PRKACA, PRKACB, PTPRC, et ZAP70, ou
- gènes corrélés à PDE4D7 choisis dans le groupe constitué par: ABCC5, CUX2, KIAA1549, PDE4D, RAP1GAP2, SLC39A11, TDRD1, et VWA2, ou
- dans laquelle ces trois gènes ou plus comprennent au moins un ou plusieurs gènes choisis parmi les gènes de réponse immunitaire, les gènes de signalisation des récepteurs des cellules T et les gènes corrélés à PDE4D7,

- dans laquelle les profils d'expression génétique des trois gènes ou plus sont déterminés dans un échantillon biologique obtenu à partir du sujet, et
- déterminer la prédiction du résultat sur la base des profils d'expression génique des trois gènes ou plus, et
- éventuellement, fournir la prédiction à un aidant ou au sujet.

2. Méthode de prédiction du résultat pour un sujet atteint d'un cancer de la vessie ou d'un cancer du rein, comprenant:

- déterminer le résultat d'une détermination des profils d'expression génique de trois gènes ou plus, les trois gènes ou plus étant choisis parmi:

- des gènes de réponse immunitaire choisis dans le groupe constitué par: AIM2, APOBEC3A, CIAO1, DDX58, DHX9, IFI16, IFIH1, IFIT1, IFIT3, LRRFIP1, MYD88, OAS1, TLR8, et ZBP1, ou
- gènes de signalisation des récepteurs des cellules T choisis dans le groupe constitué par: CD2, CD247, CD28, CD3E, CD3G, CD4, CSK, EZR, FYN, LAT, LCK, PAG1, PDE4D, PRKACA, PRKACB, PTPRC, et ZAP70, ou
- gènes corrélés à PDE4D7 choisis dans le groupe constitué par: ABCC5, CUX2, KIAA1549, PDE4D, RAP1GAP2, SLC39A11, TDRD1, et VWA2, ou
- dans laquelle ces trois gènes ou plus comprennent au moins un ou plusieurs gènes choisis parmi les gènes de réponse immunitaire, les gènes de signalisation des récepteurs des cellules T et les gènes corrélés à PDE4D7,
- dans laquelle les profils d'expression génique des trois gènes ou plus sont déterminés dans un échantillon biologique obtenu à partir du sujet, et
- déterminer la prédiction du résultat sur la base des profils d'expression génique des trois gènes ou plus, et
- éventuellement, fournir la prédiction à un aidant ou au sujet.

3. Méthode définie dans la revendication 1 ou 2, dans laquelle :

- les trois gènes de réponse de défense immunitaire ou plus comprennent six gènes de défense immunitaire ou plus, de préférence neuf gènes de défense immunitaire ou plus, de préférence tous les gènes de défense immunitaire, et/ou
- les trois gènes de signalisation du récepteur T ou plus comprennent six gènes de signalisation du récepteur T ou plus, de préférence neuf gènes de signalisation du récepteur T ou plus, de préférence tous les gènes de signalisation du récepteur T, et/ou
- les trois gènes corrélés à PDE4D7 ou plus comprennent six gènes corrélés à PDE4D7 ou plus, de préférence tous les gènes corrélés à PDE4D7, ou
- les trois gènes ou plus comprennent au moins deux gènes ou plus choisis parmi les gènes de réponse immunitaire, les gènes de signalisation des récepteurs des cellules T et les gènes corrélés à PDE4D7, de préférence trois gènes ou plus choisis parmi les gènes de réponse immunitaire, les gènes de signalisation des récepteurs T et les gènes corrélés à PDE4D7, de préférence tous les gènes sélectionnés parmi les gènes de réponse immunitaire, les gènes de signalisation des récepteurs T et les gènes corrélés à la PDE4D7, de préférence où la détermination de la prédiction du résultat comprend:

- combiner les profils d'expression génique pour deux ou plusieurs, par exemple, 2, 3, 4, 5, 6, 7, 8, 9, 10, 11, 12, 13 ou tous les gènes de la réponse immunitaire avec une fonction de régression dérivée d'une population de sujets atteints d'un cancer de la vessie ou du rein, et/ou
- combiner les profils d'expression génique pour deux ou plusieurs, par exemple 2, 3, 4, 5, 6, 7, 8, 9, 10, 11, 12, 13, 14, 15, 16 ou tous les gènes de signalisation du récepteur T avec une fonction de régression dérivée d'une population de sujets atteints d'un cancer de la vessie ou du rein, et/ou
- combiner les profils d'expression génique pour deux ou plusieurs, par exemple 2, 3, 4, 5, 6, 7 ou tous les gènes corrélés à PDE4D7 avec une fonction de régression dérivée d'une population de sujets atteints d'un cancer de la vessie ou du rein.

4. Méthode définie dans la revendication 3, dans laquelle la détermination de la prédiction du résultat consiste en outre à combiner la combinaison des profils d'expression génique avec une fonction de régression dérivée d'une population de sujets atteints d'un cancer de la vessie ou du rein.

5. Méthode définie dans l'une des revendications 1 à 4, dans laquelle la détermination du résultat est en outre basée sur un ou plusieurs paramètres cliniques obtenus du sujet, de préférence dans laquelle les paramètres cliniques comprennent un ou plusieurs des éléments suivants: (i) Attribut du stade T (T1, T2, T3 ou T4); (ii) Attribut du stade N (N0, N1, N2 ou N3), et; (iii) Attribut du stade M (M0, M1), de préférence, la détermination de la prédiction du résultat comprend la combinaison d'un ou de plusieurs profils d'expression génique: (i) des trois gènes de réponse immunitaire; (ii) les trois gènes de signalisation des récepteurs des cellules T; (iii) les trois gènes corrélés à PDE4D7, et; (iv) la combinaison d'un ou plusieurs gènes de réponse immunitaire, d'un ou plusieurs gènes de signalisation des récepteurs des cellules T et d'un ou plusieurs gènes corrélés à PDE4D7, et d'un ou plusieurs paramètres cliniques obtenus chez le sujet avec une fonction de régression dérivée d'une population de sujets atteints d'un cancer de la vessie ou du rein.

6. Méthode définie dans l'une des revendications 1 à 5, dans laquelle le(s) échantillon(s) biologique(s) est/sont obtenu(s) du sujet avant le début de la thérapie.

7. Méthode définie dans l'une des revendications 1 à 6, dans laquelle la thérapie est une chirurgie, une radiothérapie, une chimiothérapie cytotoxique (CTX) ou une immunothérapie.

8. Méthode définie dans l'une des revendications 1 à 7, dans laquelle la prédiction de la réponse thérapeutique est négative ou positive pour l'efficacité de la thérapie, dans laquelle une thérapie est recommandée sur la base de la prédiction et, si la prédiction est négative, la thérapie recommandée comprend un ou plusieurs des éléments suivants: (i) une thérapie administrée plus tôt que la norme; (ii) une radiothérapie avec une dose efficace accrue; (iii) une thérapie adjuvante, telle qu'une chimiothérapie; et (iv) une thérapie alternative, telle qu'une immunothérapie.

9. Produit de programme informatique comprenant des instructions qui, lorsque le programme est exécuté par un ordinateur, amènent l'ordinateur à exécuter une méthode comprenant:

    - recevoir des données indiquant les profils d'expression génique de trois gènes ou plus, les trois gènes ou plus étant choisis parmi:
    - des gènes de réponse immunitaire choisis dans le groupe constitué par: AIM2, APOBEC3A, CIAO1, DDX58, DHX9, IFI16, IFIH1, IFIT1, IFIT3, LRRFIP1, MYD88, OAS1, TLR8, et ZBP1, ou
    - gènes de signalisation des récepteurs des cellules T choisis dans le groupe constitué par: CD2, CD247, CD28, CD3E, CD3G, CD4, CSK, EZR, FYN, LAT, LCK, PAG1, PDE4D, PRKACA, PRKACB, PTPRC, et ZAP70, ou
    - gènes corrélés à PDE4D7 choisis dans le groupe constitué par: ABCC5, CUX2, KIAA1549, PDE4D, RAP1GAP2, SLC39A11, TDRD1, et VWA2, ou
    - dans laquelle lesdits trois gènes ou plus comprennent au moins un ou plusieurs gènes choisis parmi les gènes de réponse immunitaire, les gènes de signalisation des récepteurs des cellules T et les gènes corrélés à PDE4D7, et
    - dans laquelle les profils d'expression génique sont déterminés dans un échantillon biologique obtenu à partir d'un sujet atteint d'un cancer de la vessie ou du rein
    - déterminer la prédiction du résultat sur la base des profils d'expression génique des trois gènes ou plus, et
    - éventuellement, fournir la prédiction à un aidant ou au sujet.

10. Appareil permettant de prédire le résultat d'un sujet atteint d'un cancer de la vessie ou du rein, comprenant:

    - une entrée adaptée pour recevoir des données indiquant les profils d'expression génique de trois gènes ou plus, les trois gènes ou plus étant choisis parmi:
    - des gènes de réponse immunitaire choisis dans le groupe constitué par: AIM2, APOBEC3A, CIAO1, DDX58, DHX9, IFI16, IFIH1, IFIT1, IFIT3, LRRFIP1, MYD88, OAS1, TLR8, et ZBP1, ou
    - gènes de signalisation des récepteurs des cellules T choisis dans le groupe constitué par: CD2, CD247, CD28, CD3E, CD3G, CD4, CSK, EZR, FYN, LAT, LCK, PAG1, PDE4D, PRKACA, PRKACB, PTPRC, et ZAP70, ou
    - gènes corrélés à PDE4D7 choisis dans le groupe constitué par: ABCC5, CUX2, KIAA1549, PDE4D, RAP1GAP2, SLC39A11, TDRD1, et VWA2, ou
    - dans laquelle ces trois gènes ou plus comprennent au moins un ou plusieurs gènes choisis parmi les gènes de réponse immunitaire, les gènes de signalisation des récepteurs des cellules T et les gènes corrélés à PDE4D7;
    - dans laquelle le(s) profil(s) d'expression génique est (sont) déterminé(s) dans un échantillon biologique obtenu à partir d'un sujet atteint d'un cancer de la vessie ou du rein,
    - un processeur adapté pour déterminer la prédiction du résultat sur la base des profils d'expression génique des trois gènes ou plus,
    - un produit de programme informatique selon la revendication 9, et
    - éventuellement, une unité de fourniture adaptée pour fournir la prédiction à un aidant ou au sujet.

**11.** Kit de diagnostic comprenant:

- au moins trois amorces et sondes pour déterminer les profils d'expression génique de trois gènes ou plus, les trois gènes ou plus étant choisis parmi:
- des gènes de réponse immunitaire choisis dans le groupe constitué par: AIM2, APOBEC3A, CIAO1, DDX58, DHX9, IFI16, IFIH1, IFIT1, IFIT3, LRRFIP1, MYD88, OAS1, TLR8, et ZBP1, ou
- gènes de signalisation des récepteurs des cellules T choisis dans le groupe constitué par: CD2, CD247, CD28, CD3E, CD3G, CD4, CSK, EZR, FYN, LAT, LCK, PAG1, PDE4D, PRKACA, PRKACB, PTPRC, et ZAP70, ou
- gènes corrélés à PDE4D7 choisis dans le groupe constitué par: ABCC5, CUX2, KIAA1549, PDE4D, RAP1GAP2, SLC39A11, TDRD1, et VWA2, ou
- dans laquelle lesdits trois gènes ou plus comprennent au moins un ou plusieurs gènes choisis parmi les gènes de réponse immunitaire, les gènes de signalisation des récepteurs des cellules T et les gènes corrélés à PDE4D7, et
- dans laquelle le(s) profil(s) d'expression génique est (sont) déterminé(s) dans un échantillon biologique obtenu à partir d'un sujet atteint d'un cancer de la vessie ou du rein
- éventuellement, un appareil tel que défini dans la revendication 10 ou un produit de programme informatique tel que défini dans la revendication 9.

**12.** Utilisation du kit tel que défini dans la revendication 11.

**13.** Utilisation telle que définie dans la revendication 12 dans une méthode de prédiction du résultat d'un sujet atteint d'un cancer de la vessie ou d'un sujet atteint d'un cancer du rein.

**14.** Méthode comprenant:

- recevoir un ou plusieurs échantillons biologiques provenant d'un sujet atteint d'un cancer de la vessie ou d'un sujet atteint d'un cancer du rein,
- utiliser le kit défini dans la revendication 13 pour déterminer les profils d'expression génique de trois gènes ou plus, dans lesquels les trois gènes ou plus sont choisis parmi:
- des gènes de réponse immunitaire choisis dans le groupe constitué par: AIM2, APOBEC3A, CIAO1, DDX58, DHX9, IFI16, IFIH1, IFIT1, IFIT3, LRRFIP1, MYD88, OAS1, TLR8, et ZBP1, ou
- gènes de signalisation des récepteurs des cellules T choisis dans le groupe constitué par: CD2, CD247, CD28, CD3E, CD3G, CD4, CSK, EZR, FYN, LAT, LCK, PAG1, PDE4D, PRKACA, PRKACB, PTPRC, et ZAP70, ou
- gènes corrélés à PDE4D7 choisis dans le groupe constitué par: ABCC5, CUX2, KIAA1549, PDE4D, RAP1GAP2, SLC39A11, TDRD1, et VWA2 ou
- dans laquelle ces trois gènes ou plus comprennent au moins un ou plusieurs gènes choisis parmi les gènes de réponse immunitaire, les gènes de signalisation des récepteurs des cellules T et les gènes corrélés à PDE4D7.

**15.** Utilisation d'un produit de programme informatique selon la revendication 11 ou d'un appareil selon la revendication 12 pour utiliser les profils d'expression génique de trois gènes ou plus, dans lesquels les trois gènes ou plus sont choisis parmi:

- des gènes de réponse immunitaire choisis dans le groupe constitué par: AIM2, APOBEC3A, CIAO1, DDX58, DHX9, IFI16, IFIH1, IFIT1, IFIT3, LRRFIP1, MYD88, OAS1, TLR8, et ZBP1, ou
- gènes de signalisation des récepteurs des cellules T choisis dans le groupe constitué par: CD2, CD247, CD28, CD3E, CD3G, CD4, CSK, EZR, FYN, LAT, LCK, PAG1, PDE4D, PRKACA, PRKACB, PTPRC, et ZAP70, ou
- gènes corrélés à PDE4D7 choisis dans le groupe constitué par: ABCC5, CUX2, KIAA1549, PDE4D, RAP1GAP2, SLC39A11, TDRD1, et VWA2, ou
- dans laquelle lesdits trois gènes ou plus comprennent au moins un ou plusieurs gènes choisis parmi les gènes de réponse immunitaire, les gènes de signalisation des récepteurs T et les gènes corrélés à PDE4D7, dans une méthode de prédiction du résultat d'un sujet atteint d'un cancer de la vessie ou du rein, comprenant:
- déterminer la prédiction du résultat sur la base des profils d'expression génique des trois gènes ou plus, et
- éventuellement, fournir la prédiction à un aidant ou au sujet.

S100 → ( START )

S102 → OBTAIN BIOLOGICAL SAMPLES FROM FIRST SET OF PATIENTS

S104 → OBTAIN GENE EXPRESSION PROFILES FOR BIOLOGICAL SAMPLES

S106 → GENERATE REGRESSION FUNCTION

S108 → OBTAIN BIOLOGICAL SAMPLE FROM PATIENT

S110 → OBTAIN GENE EXPRESSION PROFILES FOR BIOLOGICAL SAMPLE

S112 → COMPUTE PREDICTION FOR PATIENT WITH REGRESSION FUNCTION

S114 → PROVIDE THERAPY RECOMMENDATION FOR THE PATIENT BASED ON THE PREDICTION

S116 → ( END )

FIG. 1

FIG. 2

EP 4 222 286 B1

FIG. 3

FIG. 4

FIG. 5

FIG. 6

FIG. 7

FIG. 8

FIG. 9

FIG. 10

FIG. 11

FIG. 12

FIG. 13

FIG. 14

FIG. 15

EP 4 222 286 B1

FIG. 16

FIG. 17

FIG. 18

FIG. 19

EP 4 222 286 B1

FIG. 20

FIG. 21

FIG. 22

FIG. 23

FIG. 24

FIG. 25

Fig. 26

Fig. 27

Fig. 28

EP 4 222 286 B1

Fig. 29

Fig. 30

Fig. 31

Fig. 32

Fig. 33

Fig. 34

Fig. 35

EP 4 222 286 B1

Fig. 36

Fig. 37

EP 4 222 286 B1

Fig. 38

Fig. 39

Fig. 40

Fig. 41

Fig. 42

Fig. 43

Fig. 44

Fig. 45

KCAL_4.3
<=threshold (0)
>threshold (0)

Survival probability (%)

Survival Time [months]

p=0.0005
HR=2.0 (95% CI 1.3-2.9)

Fig. 46

Fig. 47

EP 4 222 286 B1

Fig. 48

Fig. 49

Fig. 50

Fig. 51

Fig. 52

Fig. 53

EP 4 222 286 B1

Fig. 54

Fig. 55

Fig. 56

Fig. 57

Fig. 58

Fig. 59

Fig. 60

Fig. 61

Fig. 62

Fig. 63

Fig. 64

Fig. 65

Fig. 66

Fig. 67

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007146668 A2
- WO 2016049276 A1
- WO 2018104147 A1
- WO 2014194078 A1

**Non-patent literature cited in the description**

- Cancer Stat Facts: Bladder Cancer. *National Cancer Institute, Surveillance, Epidemology, and End Results Program*
- Bladder Cancer: Diagnosis and Management. National Collaborating Centre for Cancer. National Institute for Health and Care Excellence, 2015
- **ZHANG S. et al.** Radiotherapy in muscleinvasive bladder: the latest research progress and clinical application. *Am J Cancer Res,* 2015, vol. 5 (2), 854-868
- **ALHUNAIDI O. ; ZLOTTA A.R.** The use of intravesical BCG in urothelial carcinoma of the bladder. *Ecancermedicalscience,* 2019, vol. 13, 905
- **FAKHREJAHANI F. et al.** Immunotherapies for bladder cancer: a new hope. *Curr Opin Urol,* 2015, vol. 25 (6), 586-596
- **SANTONI G. et al.** Urinary Markers in Bladder Cancer: An Update. *Front Oncol,* 2018, vol. 8, 362
- Cancer Stat Facts: Kidney Cancer. *National Cancer Institute, Surveillance, Epidemology, and End Results Program*
- **RICKETTS C.J. et al.** The Cancer Genome Atlas Comprehensive Molecular Characterization of Renal Cell Carcinoma. *Cell Rep,* 2018, vol. 23 (1), 313-326
- **LINEHAN W.M. ; RICKETTS C.J.** The Cancer Genome Atlas of renal cell carcinoma: findings and clinical implications. *Nat Rev Urol,* 2019, vol. 16 (9), 539-552
- **ATKINS M.B. ; TANNIR N.M.** Current and emerging therapies for first-line treatment of metastatic clear cell renal cell carcinoma. *Cancer Treat Rev,* 2018, vol. 70, 127-137
- **RHOADES SMITH K.E. ; BILEN M.A.** A Review of Papillary Renal Cell Carcinoma and MET Inhibitors. *Kidney Cancer,* 2019, vol. 3 (3), 151-161
- **MANTOVANI A. et al.** Cancerrelated inflammation. *Nature,* 2008, vol. 454 (7203), 436-444
- **GIRALDO N.A. et al.** The clinical role of the TME in solid cancer. *Br J Cancer,* 2019, vol. 120 (1), 45-53

- **SHIAO S.L. et al.** Regulation of prostate cancer progression by tumor microenvironment. *Cancer Lett,* 2016, vol. 380 (1), 340-348
- **BARKER H.E. et al.** The tumor microenvironment after radiotherapy: Mechanisms of resistance or recurrence. *Nat Rev Cancer,* 2015, vol. 15 (7), 409-425
- **GASSER S. et al.** Sensing of dangerous DNA. *Mechanisms of Aging and Development,* 2017, vol. 165, 33-46
- **MOSENDEN R. ; TASKEN K.** Cyclic AMP-mediated immune regulation - Overview of mechanisms of action in T-cells. *Cell Signal,* 2011, vol. 23 (6), 1009-1016
- **PKA ; TASKEN K. ; RUPPELT A.** Negative regulation of T-cell receptor activation by the cAMP-PKA-Csk signaling pathway in T-cell lipid rafts. *Front Biosci,* 2006, vol. 11, 2929-2939
- **ABRAHAMSEN H. et al.** TCR- and CD28-mediated recruitment of phosphodiesterase 4 to lipid rafts potentiates TCR signaling. *J Immunol,* 2004, vol. 173, 4847-4848
- Immunity Leashed - Mechanisms of Regulation in the Human Immune System. **TORHEIM E.A.** Thesis for the degree of Philosophiae Doctor (PhD). The Biotechnology Centre of Ola, University of Oslo, 2009
- **ALVES DE INDA M. et al.** Validation of Cyclic Adenosine Monophosphate Phosphodiesterase-4D7 for its Independent Contribution to Risk Stratification in a Prostate Cancer Patient Cohort with Longitudinal Biological Outcomes. *Eur Urol Focus,* 2018, vol. 4 (3), 376-384
- TCGA Bladder Urothelial Carcinoma. *Firehose legacy,* 06 March 2020, http://www.linkedomics.org
- Kidney Renal Clear Cell Carcinoma, TCGA. *Firehose legacy,* 02 February 2020, http://www.cbioportal.org
- Kidney Renal Papillary Cell Carcinoma, TCGA. *Firehose legacy,* 17 February 2020, http://www.linkedomics.org